(19) Europäisches Patentamt
European Patent Office
Office européen des brevets

(11) EP 1 505 073 B1

(12) **EUROPEAN PATENT SPECIFICATION**

(45) Date of publication and mention of the grant of the patent:
**19.03.2008 Bulletin 2008/12**

(51) Int Cl.:
***C07K 14/47*** *(2006.01)* ***C07K 14/435*** *(2006.01)*

(21) Application number: **03016691.2**

(22) Date of filing: **04.08.2003**

(54) **Genetically encoded bioindicators of calcium-ions**

Genetisch-enkodierte Bioindikatoren von Kalziumionen

Bioindicateurs d'ions de calcium codés génétiquement

(84) Designated Contracting States:
**AT BE BG CH CY CZ DE DK EE ES FI FR GB GR HU IE IT LI LU MC NL PT RO SE SI SK TR**

(43) Date of publication of application:
**09.02.2005 Bulletin 2005/06**

(73) Proprietor: **Max-Planck-Gesellschaft zur Förderung der Wissenschaften e.V.**
**80539 München (DE)**

(72) Inventors:
- **Griesbeck, Oliver**
  **81667 München (DE)**
- **Heim, Nicola**
  **81241 München (DE)**

(74) Representative: **Wichmann, Hendrik**
**Patentanwälte**
**Isenbruck Bösl Hörschler Wichmann Huhn**
**Postfach 86 08 80**
**81635 München (DE)**

(56) References cited:
**EP-A- 1 238 982**

- **MIYAWAKI A ET AL: "Dynamic and quantitative Ca2+ measurements using improved cameleons." PROCEEDINGS OF THE NATIONAL ACADEMY OF SCIENCES OF THE UNITED STATES OF AMERICA. 2 MAR 1999, vol. 96, no. 5, 2 March 1999 (1999-03-02), pages 2135-2140, XP002268872 ISSN: 0027-8424**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 505 073 B1

Printed by Jouve, 75001 PARIS (FR)

SEQUENCE LISTING

<110> Max-Planck-Gesellschaft

<120> Novel genetically encoded bioindicators of calcium-ions

<130> G62276EP

<160> 30

<170> PatentIn version 3.1

<210> 1
<211> 1863
<212> DNA
<213> Artificial sequence

<220>

<223> calcium binding moiety: csTnC 15-163

<400> 1

```
atggtgagca agggcgagga gctgttcacc ggggtggtgc ccatcctggt cgagctggac      60
ggcgacgtaa acggccacag gttcagcgtg tccggcgagg gcgagggcga tgccacctac     120
ggcaagctga ccctgaagtt catctgcacc accggcaagc tgcccgtgcc ctggcccacc     180
ctcgtgacca ccctgacctg gggcgtgcag tgcttcagcc gctaccccga ccacatgaag     240
cagcacgact tcttcaagtc cgccatgccc gaaggctacg tccaggagcg taccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacat cagccacaac gtctatatca ccgccgacaa gcagaagaac     480
ggcatcaagg cccacttcaa gatccgccac aacatcgagg acggcagcgt gcagctcgcc     540
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgcccgcatg ctcagcgagg agatgattgc tgagttcaaa     720
gctgcctttg acatgtttga tgcggacggt ggtggggaca tcagcaccaa ggagttgggc     780
acggtgatga ggatgctggg ccagaacccc accaaagagg agctggatgc catcatcgag     840
gaggtggacg aggatggcag cggcaccatc gacttcgagg agttcctggt gatgatggtg     900
```

```
cgccagatga aagaggacgc caagggcaag tctgaggagg agctggccaa ctgcttccgc     960
atcttcgaca agaacgctga tgggttcatc gacatcgagg agctgggtga gattctcagg    1020
gccactgggg agcacgtcat cgaggaggac atagaagacc tcatgaagga ttcagacaag    1080
aacaatgacg gccgcattga cttcgatgag ttcctgaaga tgatggaggg tgtgcaggag    1140
ctcatggtga gcaagggcga ggagctgttc accggggtgg tgcccatcct ggtcgagctg    1200
gacggcgacg taaacggcca caagttcagc gtgtccggcg agggcgaggg cgatgccacc    1260
tacggcaagc tgaccctgaa gttcatctgc accaccggca agctgcccgt gccctggccc    1320
accctcgtga ccaccttcgg ctacggcctg atgtgcttcg cccgctaccc cgaccacatg    1380
cgccagcacg acttcttcaa gtccgccatg cccgaaggct acgtccagga gcgcaccatc    1440
ttcttcaagg acgacggcaa ctacaagacc cgcgccgagg tgaagttcga gggcgacacc    1500
ctggtgaacc gcatcgagct gaagggcatc gacttcaagg aggacggcaa catcctgggg    1560
cacaagctgg agtacaacta caacagccac aacgtctata tcatggccga caagcagaag    1620
aacggcatca aggccaactt caagatccgc cacaacatcg aggacggcag cgtgcagctc    1680
gccgaccact accagcagaa cacccccatc ggcgacggcc ccgtgctgct gcccgacaac    1740
cactacctga gctaccagtc cgccctgagc aaagacccca acgagaagcg cgatcacatg    1800
gtcctgctgg agttcgtgac cgccgccggg atcactctcg gcatggacga gctgtacaag    1860
taa                                                                  1863
```

<210> 2
<211> 620
<212> PRT
<213> Artificial Sequence

<220>
<223> Calcium binding moiety: csTnC 15-163

<400> 2

```
Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60
```

```
Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Ser Glu Glu Met Ile Ala Glu Phe Lys
225                 230                 235                 240

Ala Ala Phe Asp Met Phe Asp Ala Asp Gly Gly Gly Asp Ile Ser Thr
                245                 250                 255

Lys Glu Leu Gly Thr Val Met Arg Met Leu Gly Gln Asn Pro Thr Lys
            260                 265                 270

Glu Glu Leu Asp Ala Ile Ile Glu Glu Val Asp Glu Asp Gly Ser Gly
        275                 280                 285

Thr Ile Asp Phe Glu Glu Phe Leu Val Met Met Val Arg Gln Met Lys
    290                 295                 300

Glu Asp Ala Lys Gly Lys Ser Glu Glu Glu Leu Ala Asn Cys Phe Arg
305                 310                 315                 320

Ile Phe Asp Lys Asn Ala Asp Gly Phe Ile Asp Ile Glu Glu Leu Gly
                325                 330                 335
```

Glu Ile Leu Arg Ala Thr Gly Glu His Val Ile Glu Glu Asp Ile Glu
            340                 345                 350

Asp Leu Met Lys Asp Ser Asp Lys Asn Asn Asp Gly Arg Ile Asp Phe
        355                 360                 365

Asp Glu Phe Leu Lys Met Met Glu Gly Val Gln Glu Leu Met Val Ser
    370                 375                 380

Lys Gly Glu Glu Leu Phe Thr Gly Val Val Pro Ile Leu Val Glu Leu
385                 390                 395                 400

Asp Gly Asp Val Asn Gly His Lys Phe Ser Val Ser Gly Glu Gly Glu
                405                 410                 415

Gly Asp Ala Thr Tyr Gly Lys Leu Thr Leu Lys Phe Ile Cys Thr Thr
            420                 425                 430

Gly Lys Leu Pro Val Pro Trp Pro Thr Leu Val Thr Thr Phe Gly Tyr
        435                 440                 445

Gly Leu Met Cys Phe Ala Arg Tyr Pro Asp His Met Arg Gln His Asp
    450                 455                 460

Phe Phe Lys Ser Ala Met Pro Glu Gly Tyr Val Gln Glu Arg Thr Ile
465                 470                 475                 480

Phe Phe Lys Asp Asp Gly Asn Tyr Lys Thr Arg Ala Glu Val Lys Phe
                485                 490                 495

Glu Gly Asp Thr Leu Val Asn Arg Ile Glu Leu Lys Gly Ile Asp Phe
            500                 505                 510

Lys Glu Asp Gly Asn Ile Leu Gly His Lys Leu Glu Tyr Asn Tyr Asn
        515                 520                 525

Ser His Asn Val Tyr Ile Met Ala Asp Lys Gln Lys Asn Gly Ile Lys
    530                 535                 540

Ala Asn Phe Lys Ile Arg His Asn Ile Glu Asp Gly Ser Val Gln Leu
545                 550                 555                 560

Ala Asp His Tyr Gln Gln Asn Thr Pro Ile Gly Asp Gly Pro Val Leu
                565                 570                 575

Leu Pro Asp Asn His Tyr Leu Ser Tyr Gln Ser Ala Leu Ser Lys Asp
            580                 585                 590

Pro Asn Glu Lys Arg Asp His Met Val Leu Leu Glu Phe Val Thr Ala
        595                 600                 605

Ala Gly Ile Thr Leu Gly Met Asp Glu Leu Tyr Lys
610 615 620

<210> 3
<211> 1902
<212> DNA
<213> Artificial Sequence

<220>
<223> Calcium binding moiety: humcTnC

<400> 3

```
atggtgagca agggcgagga gctgttcacc ggggtggtgc ccatcctggt cgagctggac     60
ggcgacgtaa acggccacag gttcagcgtg tccggcgagg gcgagggcga tgccacctac    120
ggcaagctga ccctgaagtt catctgcacc accggcaagc tgcccgtgcc ctggcccacc    180
ctcgtgacca ccctgacctg gggcgtgcag tgcttcagcc gctaccccga ccacatgaag    240
cagcacgact tcttcaagtc cgccatgccc gaaggctacg tccaggagcg taccatcttc    300
ttcaaggacg acggcaacta caagacccgc gccgaggtga agttcgaggg cgacaccctg    360
gtgaaccgca tcgagctgaa gggcatcgac ttcaaggagg acggcaacat cctggggcac    420
aagctggagt acaactacat cagccacaac gtctatatca ccgccgacaa gcagaagaac    480
ggcatcaagg cccacttcaa gatccgccac aacatcgagg acggcagcgt gcagctcgcc    540
gaccactacc agcagaacac ccccatcggc gacggccccg tgctgctgcc cgacaaccac    600
tacctgagca cccagtccgc cctgagcaaa gaccccaacg agaagcgcga tcacatggtc    660
ctgctggagt tcgtgaccgc cgcccgcatg ctaatggatg acatctacaa ggctgcggta    720
gagcagctga cagaagagca gaaaaatgag ttcaaggcag ccttcgacat cttcgtgctg    780
ggcgctgagg atggctgcat cagcaccaag gagctgggca aggtgatgag gatgctgggc    840
cagaacccca cccctgagga gctgcaggag atgatcgatg aggtggacga ggacggcagc    900
ggcacggtgg actttgatga gttcctggtc atgatggttc ggtgcatgaa ggacgacagc    960
aaagggaaat ctgaggagga gctgtctgac ctcttccgca tgtttgacaa aaatgctgat   1020
ggctacatcg acctggatga gctgaagata atgctgcagg ctacaggcga gaccatcacg   1080
gaggacgaca tcgaggaact catgaaggac ggagacaaga acaacgacgg ccgcatcgac   1140
tatgatgagt tcctggagtt catgaagggt gtggaggagc tcatggtgag caagggcgag   1200
gagctgttca ccggggtggt gcccatcctg gtcgagctgg acggcgacgt aaacggccac   1260
aagttcagcg tgtccggcga gggcgagggc gatgccacct acggcaagct gaccctgaag   1320
ttcatctgca ccaccggcaa gctgcccgtg ccctggccca ccctcgtgac caccttcggc   1380
tacggcctga tgtgcttcgc ccgctacccc gaccacatgc gccagcacga cttcttcaag   1440
tccgccatgc ccgaaggcta cgtccaggag cgcaccatct tcttcaagga cgacggcaac   1500
```

tacaagaccc gcgccgaggt gaagttcgag ggcgacaccc tggtgaaccg catcgagctg 1560
aagggcatcg acttcaagga ggacggcaac atcctggggc acaagctgga gtacaactac 1620
aacagccaca acgtctatat catggccgac aagcagaaga acggcatcaa ggccaacttc 1680
aagatccgcc acaacatcga ggacggcagc gtgcagctcg ccgaccacta ccagcagaac 1740
acccccatcg gcgacggccc cgtgctgctg cccgacaacc actacctgag ctaccagtcc 1800
gccctgagca aagaccccaa cgagaagcgc gatcacatgg tcctgctgga gttcgtgacc 1860
gccgccggga tcactctcgg catggacgag ctgtacaagt aa 1902

<210> 4
<211> 633
<212> PRT
<213> Artificial Sequence

<220>
<223> Calcium binding moiety: humcTnC

<400> 4

Met Val Ser Lys Gly Glu Glu Leu Phe Thr Gly Val Val Pro Ile Leu
1 5 10 15

Val Glu Leu Asp Gly Asp Val Asn Gly His Arg Phe Ser Val Ser Gly
20 25 30

Glu Gly Glu Gly Asp Ala Thr Tyr Gly Lys Leu Thr Leu Lys Phe Ile
35 40 45

Cys Thr Thr Gly Lys Leu Pro Val Pro Trp Pro Thr Leu Val Thr Thr
50 55 60

Leu Thr Trp Gly Val Gln Cys Phe Ser Arg Tyr Pro Asp His Met Lys
65 70 75 80

Gln His Asp Phe Phe Lys Ser Ala Met Pro Glu Gly Tyr Val Gln Glu
85 90 95

Arg Thr Ile Phe Phe Lys Asp Asp Gly Asn Tyr Lys Thr Arg Ala Glu
100 105 110

Val Lys Phe Glu Gly Asp Thr Leu Val Asn Arg Ile Glu Leu Lys Gly
115 120 125

Ile Asp Phe Lys Glu Asp Gly Asn Ile Leu Gly His Lys Leu Glu Tyr
130 135 140

Asn Tyr Ile Ser His Asn Val Tyr Ile Thr Ala Asp Lys Gln Lys Asn
145 150 155 160

Gly Ile Lys Ala His Phe Lys Ile Arg His Asn Ile Glu Asp Gly Ser
165 170 175

Val Gln Leu Ala Asp His Tyr Gln Gln Asn Thr Pro Ile Gly Asp Gly
180 185 190

Pro Val Leu Leu Pro Asp Asn His Tyr Leu Ser Thr Gln Ser Ala Leu
195 200 205

Ser Lys Asp Pro Asn Glu Lys Arg Asp His Met Val Leu Leu Glu Phe
210 215 220

Val Thr Ala Ala Arg Met Leu Met Asp Asp Ile Tyr Lys Ala Ala Val
225 230 235 240

Glu Gln Leu Thr Glu Glu Gln Lys Asn Glu Phe Lys Ala Ala Phe Asp
245 250 255

Ile Phe Val Leu Gly Ala Glu Asp Gly Cys Ile Ser Thr Lys Glu Leu
260 265 270

Gly Lys Val Met Arg Met Leu Gly Gln Asn Pro Thr Pro Glu Glu Leu
275 280 285

Gln Glu Met Ile Asp Glu Val Asp Glu Asp Gly Ser Gly Thr Val Asp
290 295 300

Phe Asp Glu Phe Leu Val Met Met Val Arg Cys Met Lys Asp Asp Ser
305 310 315 320

Lys Gly Lys Ser Glu Glu Glu Leu Ser Asp Leu Phe Arg Met Phe Asp
325 330 335

Lys Asn Ala Asp Gly Tyr Ile Asp Leu Asp Glu Leu Lys Ile Met Leu
340 345 350

Gln Ala Thr Gly Glu Thr Ile Thr Glu Asp Asp Ile Glu Glu Leu Met
355 360 365

Lys Asp Gly Asp Lys Asn Asn Asp Gly Arg Ile Asp Tyr Asp Glu Phe
370 375 380

Leu Glu Phe Met Lys Gly Val Glu Glu Leu Met Val Ser Lys Gly Glu
385 390 395 400

Glu Leu Phe Thr Gly Val Val Pro Ile Leu Val Glu Leu Asp Gly Asp
405 410 415

Val Asn Gly His Lys Phe Ser Val Ser Gly Glu Gly Glu Gly Asp Ala
420 425 430

Thr Tyr Gly Lys Leu Thr Leu Lys Phe Ile Cys Thr Thr Gly Lys Leu
435 440 445

Pro Val Pro Trp Pro Thr Leu Val Thr Thr Phe Gly Tyr Gly Leu Met
450 455 460

Cys Phe Ala Arg Tyr Pro Asp His Met Arg Gln His Asp Phe Phe Lys
465 470 475 480

Ser Ala Met Pro Glu Gly Tyr Val Gln Glu Arg Thr Ile Phe Phe Lys
485 490 495

Asp Asp Gly Asn Tyr Lys Thr Arg Ala Glu Val Lys Phe Glu Gly Asp
500 505 510

Thr Leu Val Asn Arg Ile Glu Leu Lys Gly Ile Asp Phe Lys Glu Asp
515 520 525

Gly Asn Ile Leu Gly His Lys Leu Glu Tyr Asn Tyr Asn Ser His Asn
530 535 540

Val Tyr Ile Met Ala Asp Lys Gln Lys Asn Gly Ile Lys Ala Asn Phe
545 550 555 560

Lys Ile Arg His Asn Ile Glu Asp Gly Ser Val Gln Leu Ala Asp His
565 570 575

Tyr Gln Gln Asn Thr Pro Ile Gly Asp Gly Pro Val Leu Leu Pro Asp
580 585 590

Asn His Tyr Leu Ser Tyr Gln Ser Ala Leu Ser Lys Asp Pro Asn Glu
595 600 605

Lys Arg Asp His Met Val Leu Leu Glu Phe Val Thr Ala Ala Gly Ile
610 615 620

Thr Leu Gly Met Asp Glu Leu Tyr Lys
625 630

<210> 5
<211> 1863
<212> DNA
<213> Artificial sequence

<220>
<223> calcium binding moiety: csTnC 15-163

<400> 5

```
atggtgagca agggcgagga gctgttcacc ggggtggtgc ccatcctggt cgagctggac      60
ggcgacgtaa acggccacag gttcagcgtg tccggcgagg gcgagggcga tgccacctac     120
ggcaagctga ccctgaagtt catctgcacc accggcaagc tgcccgtgcc ctggcccacc     180
ctcgtgacca ccctgacctg gggcgtgcag tgcttcagcc gctaccccga ccacatgaag     240
cagcacgact tcttcaagtc cgccatgccc gaaggctacg tccaggagcg taccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacat cagccacaac gtctatatca ccgccgacaa gcagaagaac     480
ggcatcaagg cccacttcaa gatccgccac aacatcgagg acggcagcgt gcagctcgcc     540
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgcccgcatg ctcagcgagg agatgattgc tgagttcaaa     720
gctgcctttg acatgtttga tgcggacggt ggtggggaca tcagcaccaa ggagttgggc     780
acggtgatga ggatgctggg ccagaacccc accaaagagg agctggatgc catcatcgag     840
gaggtggacg aggatggcag cggcaccatc gacttcgagg agttcctggt gatgatggtg     900
cgccagatga aagaggacgc caagggcaag tctgaggagg agctggccaa ctgcttccgc     960
atcttcgcca agaacgctga tgggttcatc gacatcgagg agctgggtga gattctcagg    1020
gccactgggg agcacgtcat cgaggaggac atagaagacc tcatgaagga ttcagacaag    1080
aacaatgacg gccgcattga cttcgatgag ttcctgaaga tgatggaggg tgtgcaggag    1140
ctcatggtga gcaagggcga ggagctgttc accggggtgg tgcccatcct ggtcgagctg    1200
gacggcgacg taaacggcca caagttcagc gtgtccggcg agggcgaggg cgatgccacc    1260
tacggcaagc tgaccctgaa gttcatctgc accaccggca agctgcccgt gccctggccc    1320
accctcgtga ccaccttcgg ctacggcctg atgtgcttcg cccgctaccc cgaccacatg    1380
cgccagcacg acttcttcaa gtccgccatg cccgaaggct acgtccagga gcgcaccatc    1440
ttcttcaagg acgacggcaa ctacaagacc cgcgccgagg tgaagttcga gggcgacacc    1500
ctggtgaacc gcatcgagct gaagggcatc gacttcaagg aggacggcaa catcctgggg    1560
cacaagctgg agtacaacta caacagccac aacgtctata tcatggccga caagcagaag    1620
aacggcatca aggccaactt caagatccgc cacaacatcg aggacggcag cgtgcagctc    1680
gccgaccact accagcagaa cacccccatc ggcgacggcc ccgtgctgct gcccgacaac    1740
cactacctga gctaccagtc cgccctgagc aaagacccca acgagaagcg cgatcacatg    1800
gtcctgctgg agttcgtgac cgccgccggg atcactctcg gcatggacga gctgtacaag    1860
taa                                                                  1863
```

<210> 6
<211> 620
<212> PRT

<213> Artificial sequence

<220>
<223> calcium binding moiety: csTnC 15-163

<400> 6

```
Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205
```

Ser Lys Asp Pro Asn Glu Lys Arg Asp His Met Val Leu Leu Glu Phe
210 215 220

Val Thr Ala Ala Arg Met Leu Ser Glu Glu Met Ile Ala Glu Phe Lys
225 230 235 240

Ala Ala Phe Asp Met Phe Asp Ala Asp Gly Gly Gly Asp Ile Ser Thr
245 250 255

Lys Glu Leu Gly Thr Val Met Arg Met Leu Gly Gln Asn Pro Thr Lys
260 265 270

Glu Glu Leu Asp Ala Ile Ile Glu Glu Val Asp Glu Asp Gly Ser Gly
275 280 285

Thr Ile Asp Phe Glu Glu Phe Leu Val Met Met Val Arg Gln Met Lys
290 295 300

Glu Asp Ala Lys Gly Lys Ser Glu Glu Glu Leu Ala Asn Cys Phe Arg
305 310 315 320

Ile Phe Ala Lys Asn Ala Asp Gly Phe Ile Asp Ile Glu Glu Leu Gly
325 330 335

Glu Ile Leu Arg Ala Thr Gly Glu His Val Ile Glu Glu Asp Ile Glu
340 345 350

Asp Leu Met Lys Asp Ser Asp Lys Asn Asn Asp Gly Arg Ile Asp Phe
355 360 365

Asp Glu Phe Leu Lys Met Met Glu Gly Val Gln Glu Leu Met Val Ser
370 375 380

Lys Gly Glu Glu Leu Phe Thr Gly Val Val Pro Ile Leu Val Glu Leu
385 390 395 400

Asp Gly Asp Val Asn Gly His Lys Phe Ser Val Ser Gly Glu Gly Glu
405 410 415

Gly Asp Ala Thr Tyr Gly Lys Leu Thr Leu Lys Phe Ile Cys Thr Thr
420 425 430

Gly Lys Leu Pro Val Pro Trp Pro Thr Leu Val Thr Thr Phe Gly Tyr
435 440 445

Gly Leu Met Cys Phe Ala Arg Tyr Pro Asp His Met Arg Gln His Asp
450 455 460

Phe Phe Lys Ser Ala Met Pro Glu Gly Tyr Val Gln Glu Arg Thr Ile
465 470 475 480

Phe Phe Lys Asp Asp Gly Asn Tyr Lys Thr Arg Ala Glu Val Lys Phe
485 490 495

Glu Gly Asp Thr Leu Val Asn Arg Ile Glu Leu Lys Gly Ile Asp Phe
500 505 510

Lys Glu Asp Gly Asn Ile Leu Gly His Lys Leu Glu Tyr Asn Tyr Asn
515 520 525

Ser His Asn Val Tyr Ile Met Ala Asp Lys Gln Lys Asn Gly Ile Lys
530 535 540

Ala Asn Phe Lys Ile Arg His Asn Ile Glu Asp Gly Ser Val Gln Leu
545 550 555 560

Ala Asp His Tyr Gln Gln Asn Thr Pro Ile Gly Asp Gly Pro Val Leu
565 570 575

Leu Pro Asp Asn His Tyr Leu Ser Tyr Gln Ser Ala Leu Ser Lys Asp
580 585 590

Pro Asn Glu Lys Arg Asp His Met Val Leu Leu Glu Phe Val Thr Ala
595 600 605

Ala Gly Ile Thr Leu Gly Met Asp Glu Leu Tyr Lys
610 615 620

<210> 7
<211> 1908
<212> DNA
<213> Artificial Sequence

<220>
<223> calcium binding moiety: csTnC

<400> 7

atggtgagca agggcgagga gctgttcacc ggggtggtgc ccatcctggt cgagctggac 60
ggcgacgtaa acggccacag gttcagcgtg tccggcgagg gcgagggcga tgccacctac 120
ggcaagctga ccctgaagtt catctgcacc accggcaagc tgcccgtgcc ctggcccacc 180
ctcgtgacca ccctgacctg gggcgtgcag tgcttcagcc gctaccccga ccacatgaag 240
cagcacgact tcttcaagtc cgccatgccc gaaggctacg tccaggagcg taccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg cccacttcaa gatccgccac aacatcgagg acggcagcgt gcagctcgcc 540

gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgcccgcatg ctaatggcgt caatgacgga ccagcaggcg 720
gaggcccgcg ccttcctcag cgaggagatg attgctgagt tcaaagctgc ctttgacatg 780
tttgatgcgg acggtggtgg ggacatcagc accaaggagt tgggcacggt gatgaggatg 840
ctgggccaga accccaccaa agaggagctg gatgccatca tcgaggaggt ggacgaggat 900
ggcagcggca ccatcgactt cgaggagttc ctggtgatga tggtgcgcca gatgaaagag 960
gacgccaagg gcaagtctga ggaggagctg gccaactgct tccgcatctt cgacaagaac 1020
gctgatgggt tcatcgacat cgaggagctg ggtgagattc tcagggccac tggggagcac 1080
gtcatcgagg aggacataga agacctcatg aaggattcag acaagaacaa tgacggccgc 1140
attgacttcg atgagttcct gaagatgatg gagggtgtgc aggagctcat ggtgagcaag 1200
ggcgaggagc tgttcaccgg ggtggtgccc atcctggtcg agctggacgg cgacgtaaac 1260
ggccacaagt tcagcgtgtc cggcgagggc gagggcgatg ccacctacgg caagctgacc 1320
ctgaagttca tctgcaccac cggcaagctg cccgtgccct ggcccaccct cgtgaccacc 1380
ttcggctacg gcctgatgtg cttcgcccgc taccccgacc acatgcgcca gcacgacttc 1440
ttcaagtccg ccatgcccga aggctacgtc caggagcgca ccatcttctt caaggacgac 1500
ggcaactaca agacccgcgc cgaggtgaag ttcgagggcg acaccctggt gaaccgcatc 1560
gagctgaagg gcatcgactt caaggaggac ggcaacatcc tggggcacaa gctggagtac 1620
aactacaaca gccacaacgt ctatatcatg gccgacaagc agaagaacgg catcaaggcc 1680
aacttcaaga tccgccacaa catcgaggac ggcagcgtgc agctcgccga ccactaccag 1740
cagaacaccc ccatcggcga cggccccgtg ctgctgcccg acaaccacta cctgagctac 1800
cagtccgccc tgagcaaaga ccccaacgag aagcgcgatc acatggtcct gctggagttc 1860
gtgaccgccg ccgggatcac tctcggcatg gacgagctgt acaagtaa 1908

<210> 8
<211> 635
<212> PRT
<213> Artificial Sequence

<220>
<223> calcium binding moiety: csTnC

<400> 8

Met Val Ser Lys Gly Glu Glu Leu Phe Thr Gly Val Val Pro Ile Leu
1 5 10 15

```
Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Met Ala Ser Met Thr Asp Gln Gln Ala
225                 230                 235                 240

Glu Ala Arg Ala Phe Leu Ser Glu Glu Met Ile Ala Glu Phe Lys Ala
                245                 250                 255

Ala Phe Asp Met Phe Asp Ala Asp Gly Gly Gly Asp Ile Ser Thr Lys
            260                 265                 270

Glu Leu Gly Thr Val Met Arg Met Leu Gly Gln Asn Pro Thr Lys Glu
        275                 280                 285
```

Glu Leu Asp Ala Ile Ile Glu Glu Val Asp Glu Asp Gly Ser Gly Thr
290 295 300

Ile Asp Phe Glu Glu Phe Leu Val Met Met Val Arg Gln Met Lys Glu
305 310 315 320

Asp Ala Lys Gly Lys Ser Glu Glu Glu Leu Ala Asn Cys Phe Arg Ile
325 330 335

Phe Asp Lys Asn Ala Asp Gly Phe Ile Asp Ile Glu Glu Leu Gly Glu
340 345 350

Ile Leu Arg Ala Thr Gly Glu His Val Ile Glu Glu Asp Ile Glu Asp
355 360 365

Leu Met Lys Asp Ser Asp Lys Asn Asn Asp Gly Arg Ile Asp Phe Asp
370 375 380

Glu Phe Leu Lys Met Met Glu Gly Val Gln Glu Leu Met Val Ser Lys
385 390 395 400

Gly Glu Glu Leu Phe Thr Gly Val Val Pro Ile Leu Val Glu Leu Asp
405 410 415

Gly Asp Val Asn Gly His Lys Phe Ser Val Ser Gly Glu Gly Glu Gly
420 425 430

Asp Ala Thr Tyr Gly Lys Leu Thr Leu Lys Phe Ile Cys Thr Thr Gly
435 440 445

Lys Leu Pro Val Pro Trp Pro Thr Leu Val Thr Thr Phe Gly Tyr Gly
450 455 460

Leu Met Cys Phe Ala Arg Tyr Pro Asp His Met Arg Gln His Asp Phe
465 470 475 480

Phe Lys Ser Ala Met Pro Glu Gly Tyr Val Gln Glu Arg Thr Ile Phe
485 490 495

Phe Lys Asp Asp Gly Asn Tyr Lys Thr Arg Ala Glu Val Lys Phe Glu
500 505 510

Gly Asp Thr Leu Val Asn Arg Ile Glu Leu Lys Gly Ile Asp Phe Lys
515 520 525

Glu Asp Gly Asn Ile Leu Gly His Lys Leu Glu Tyr Asn Tyr Asn Ser
530 535 540

His Asn Val Tyr Ile Met Ala Asp Lys Gln Lys Asn Gly Ile Lys Ala
545 550 555 560

Asn Phe Lys Ile Arg His Asn Ile Glu Asp Gly Ser Val Gln Leu Ala
565 570 575

Asp His Tyr Gln Gln Asn Thr Pro Ile Gly Asp Gly Pro Val Leu Leu
580 585 590

Pro Asp Asn His Tyr Leu Ser Tyr Gln Ser Ala Leu Ser Lys Asp Pro
595 600 605

Asn Glu Lys Arg Asp His Met Val Leu Leu Glu Phe Val Thr Ala Ala
610 615 620

Gly Ile Thr Leu Gly Met Asp Glu Leu Tyr Lys
625 630 635

<210> 9
<211> 1542
<212> DNA
<213> Artificial Sequence

<220>
<223> calcium binding moiety: csTnC EF-hand 2, 51-91

<400> 9

atggtgagca agggcgagga gctgttcacc ggggtggtgc ccatcctggt cgagctggac 60
ggcgacgtaa acggccacag gttcagcgtg tccggcgagg gcgagggcga tgccacctac 120
ggcaagctga ccctgaagtt catctgcacc accggcaagc tgcccgtgcc ctggcccacc 180
ctcgtgacca ccctgacctg gggcgtgcag tgcttcagcc gctaccccga ccacatgaag 240
cagcacgact tcttcaagtc cgccatgccc gaaggctacg tccaggagcg taccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg cccacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgcccgcatg ctaggccaga accccaccaa agaggagctg 720
gatgccatca tcgaggaggt ggacgaggat ggcagcggca ccatcgactt cgaggagttc 780
ctggtgatga tggtgcgcca gatgaaagag gacgccgagc tcatggtgag caagggcgag 840
gagctgttca ccggggtggt gcccatcctg gtcgagctgg acggcgacgt aaacggccac 900
aagttcagcg tgtccggcga gggcgagggc gatgccacct acggcaagct gaccctgaag 960
ttcatctgca ccaccggcaa gctgcccgtg ccctggccca ccctcgtgac caccttcggc 1020

```
tacggcctga tgtgcttcgc ccgctacccc gaccacatgc gccagcacga cttcttcaag    1080
tccgccatgc ccgaaggcta cgtccaggag cgcaccatct tcttcaagga cgacggcaac    1140
tacaagaccc gcgccgaggt gaagttcgag ggcgacaccc tggtgaaccg catcgagctg    1200
aagggcatcg acttcaagga ggacggcaac atcctggggc acaagctgga gtacaactac    1260
aacagccaca acgtctatat catggccgac aagcagaaga acggcatcaa ggccaacttc    1320
aagatccgcc acaacatcga ggacggcagc gtgcagctcg ccgaccacta ccagcagaac    1380
acccccatcg gcgacggccc cgtgctgctg cccgacaacc actacctgag ctaccagtcc    1440
gccctgagca aagaccccaa cgagaagcgc gatcacatgg tcctgctgga gttcgtgacc    1500
gccgccggga tcactctcgg catggacgag ctgtacaagt aa                      1542
```

<210> 10
<211> 513
<212> PRT
<213> Artificial sequence

<220>
<223> calcium binding moiety: csTnC EF-hand 2, 51-91

<400> 10

```
Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125
```

```
Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Gly Gln Asn Pro Thr Lys Glu Glu Leu
225                 230                 235                 240

Asp Ala Ile Ile Glu Glu Val Asp Glu Asp Gly Ser Gly Thr Ile Asp
                245                 250                 255

Phe Glu Glu Phe Leu Val Met Met Val Arg Gln Met Lys Glu Asp Ala
            260                 265                 270

Glu Leu Met Val Ser Lys Gly Glu Glu Leu Phe Thr Gly Val Val Pro
        275                 280                 285

Ile Leu Val Glu Leu Asp Gly Asp Val Asn Gly His Lys Phe Ser Val
    290                 295                 300

Ser Gly Glu Gly Glu Gly Asp Ala Thr Tyr Gly Lys Leu Thr Leu Lys
305                 310                 315                 320

Phe Ile Cys Thr Thr Gly Lys Leu Pro Val Pro Trp Pro Thr Leu Val
                325                 330                 335

Thr Thr Phe Gly Tyr Gly Leu Met Cys Phe Ala Arg Tyr Pro Asp His
            340                 345                 350

Met Arg Gln His Asp Phe Phe Lys Ser Ala Met Pro Glu Gly Tyr Val
        355                 360                 365

Gln Glu Arg Thr Ile Phe Phe Lys Asp Asp Gly Asn Tyr Lys Thr Arg
    370                 375                 380

Ala Glu Val Lys Phe Glu Gly Asp Thr Leu Val Asn Arg Ile Glu Leu
385                 390                 395                 400
```

Lys Gly Ile Asp Phe Lys Glu Asp Gly Asn Ile Leu Gly His Lys Leu
405 410 415

Glu Tyr Asn Tyr Asn Ser His Asn Val Tyr Ile Met Ala Asp Lys Gln
420 425 430

Lys Asn Gly Ile Lys Ala Asn Phe Lys Ile Arg His Asn Ile Glu Asp
435 440 445

Gly Ser Val Gln Leu Ala Asp His Tyr Gln Gln Asn Thr Pro Ile Gly
450 455 460

Asp Gly Pro Val Leu Leu Pro Asp Asn His Tyr Leu Ser Tyr Gln Ser
465 470 475 480

Ala Leu Ser Lys Asp Pro Asn Glu Lys Arg Asp His Met Val Leu Leu
485 490 495

Glu Phe Val Thr Ala Ala Gly Ile Thr Leu Gly Met Asp Glu Leu Tyr
500 505 510

Lys

<210> 11
<211> 2469
<212> DNA
<213> Artificial Sequence

<220>
<223> Calcium binding moiety: csTnC - Gly-Gly - csTnI

<400> 11

atggtgagca agggcgagga gctgttcacc ggggtggtgc ccatcctggt cgagctggac 60
ggcgacgtaa acggccacag gttcagcgtg tccggcgagg gcgagggcga tgccacctac 120
ggcaagctga ccctgaagtt catctgcacc accggcaagc tgcccgtgcc ctggcccacc 180
ctcgtgacca ccctgacctg gggcgtgcag tgcttcagcc gctaccccga ccacatgaag 240
cagcacgact tcttcaagtc cgccatgccc gaaggctacg tccaggagcg taccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg cccacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660

```
ctgctggagt tcgtgaccgc cgcccgcatg ctaatggcgt caatgacgga ccagcaggcg    720
gaggcccgcg ccttcctcag cgaggagatg attgctgagt tcaaagctgc ctttgacatg    780
tttgatgcgg acggtggtgg ggacatcagc accaaggagt tgggcacggt gatgaggatg    840
ctgggccaga accccaccaa agaggagctg gatgccatca tcgaggaggt ggacgaggat    900
ggcagcggca ccatcgactt cgaggagttc ctggtgatga tggtgcgcca gatgaaagag    960
gacgccaagg gcaagtctga ggaggagctg gccaactgct tccgcatctt cgacaagaac   1020
gctgatgggt tcatcgacat cgaggagctg ggtgagattc tcagggccac tggggagcac   1080
gtcatcgagg aggacataga agacctcatg aaggattcag acaagaacaa tgacggccgc   1140
attgacttcg atgagttcct gaagatgatg gagggtgtgc aggagctcgg cggcatgtct   1200
gatgaagaga aaaagcgtcg tgcagccacc gcccgtcgtc agcacctgaa gagtgctatg   1260
ctccagcttg ctgtcactga aatagaaaaa gaagcagctg ctaaagaagt ggaaaagcaa   1320
aactacctgg cagagcatag ccctcctctg tccctcccag ggtccatgca ggaacttcag   1380
gaactgagca aaaaacttca tgccaagata gactcagtgg atgaggaaag gtatgacaca   1440
gaggtgaagc tacagaagac taacaaggag ctggaggacc tgagccagaa gctgtttgac   1500
ctgaggggca agttcaagag gccacctctg cgccgggtgc gcatgtctgc tgatgccatg   1560
ctgcgtgccc tgctgggctc caagcacaag gtcaacatgg acctccgggc caacctgaag   1620
caagtcaaga aggaggacac ggagaaggag aaggacctcc gcgatgtggg tgactggagg   1680
aagaacattg aggagaaatc tggcatggag ggcaggaaga agatgtttga ggccggcgag   1740
tccgagctca tggtgagcaa gggcgaggag ctgttcaccg ggtggtgcc catcctggtc    1800
gagctggacg gcgacgtaaa cggccacaag ttcagcgtgt ccggcgaggg cgagggcgat   1860
gccacctacg gcaagctgac cctgaagttc atctgcacca ccggcaagct gcccgtgccc   1920
tggcccaccc tcgtgaccac cttcggctac ggcctgatgt gcttcgcccg ctaccccgac   1980
cacatgcgcc agcacgactt cttcaagtcc gccatgcccg aaggctacgt ccaggagcgc   2040
accatcttct tcaaggacga cggcaactac aagacccgcg ccgaggtgaa gttcgagggc   2100
gacaccctgg tgaaccgcat cgagctgaag ggcatcgact tcaaggagga cggcaacatc   2160
ctggggcaca agctggagta caactacaac agccacaacg tctatatcat ggccgacaag   2220
cagaagaacg gcatcaaggc caacttcaag atccgccaca acatcgagga cggcagcgtg   2280
cagctcgccg accactacca gcagaacacc cccatcggcg acggccccgt gctgctgccc   2340
gacaaccact acctgagcta ccagtccgcc ctgagcaaag accccaacga gaagcgcgat   2400
cacatggtcc tgctggagtt cgtgaccgcc gccgggatca ctctcggcat ggacgagctg   2460
tacaagtaa                                                          2469
```

<210> 12
<211> 822
<212> PRT
<213> Artificial Sequence

<220>

<223> Calcium binding moiety: csTnC - Gly-Gly - csTnI

<400> 12

```
Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220
```

Val Thr Ala Ala Arg Met Leu Met Ala Ser Met Thr Asp Gln Gln Ala
225 230 235 240

Glu Ala Arg Ala Phe Leu Ser Glu Glu Met Ile Ala Glu Phe Lys Ala
245 250 255

Ala Phe Asp Met Phe Asp Ala Asp Gly Gly Gly Asp Ile Ser Thr Lys
260 265 270

Glu Leu Gly Thr Val Met Arg Met Leu Gly Gln Asn Pro Thr Lys Glu
275 280 285

Glu Leu Asp Ala Ile Ile Glu Glu Val Asp Glu Asp Gly Ser Gly Thr
290 295 300

Ile Asp Phe Glu Glu Phe Leu Val Met Met Val Arg Gln Met Lys Glu
305 310 315 320

Asp Ala Lys Gly Lys Ser Glu Glu Glu Leu Ala Asn Cys Phe Arg Ile
325 330 335

Phe Asp Lys Asn Ala Asp Gly Phe Ile Asp Ile Glu Glu Leu Gly Glu
340 345 350

Ile Leu Arg Ala Thr Gly Glu His Val Ile Glu Glu Asp Ile Glu Asp
355 360 365

Leu Met Lys Asp Ser Asp Lys Asn Asn Asp Gly Arg Ile Asp Phe Asp
370 375 380

Glu Phe Leu Lys Met Met Glu Gly Val Gln Glu Leu Gly Gly Met Ser
385 390 395 400

Asp Glu Glu Lys Lys Arg Arg Ala Ala Thr Ala Arg Arg Gln His Leu
405 410 415

Lys Ser Ala Met Leu Gln Leu Ala Val Thr Glu Ile Glu Lys Glu Ala
420 425 430

Ala Ala Lys Glu Val Glu Lys Gln Asn Tyr Leu Ala Glu His Ser Pro
435 440 445

Pro Leu Ser Leu Pro Gly Ser Met Gln Glu Leu Gln Glu Leu Ser Lys
450 455 460

Lys Leu His Ala Lys Ile Asp Ser Val Asp Glu Glu Arg Tyr Asp Thr
465 470 475 480

Glu Val Lys Leu Gln Lys Thr Asn Lys Glu Leu Glu Asp Leu Ser Gln
485 490 495

Lys Leu Phe Asp Leu Arg Gly Lys Phe Lys Arg Pro Pro Leu Arg Arg
500 505 510

Val Arg Met Ser Ala Asp Ala Met Leu Arg Ala Leu Leu Gly Ser Lys
515 520 525

His Lys Val Asn Met Asp Leu Arg Ala Asn Leu Lys Gln Val Lys Lys
530 535 540

Glu Asp Thr Glu Lys Glu Lys Asp Leu Arg Asp Val Gly Asp Trp Arg
545 550 555 560

Lys Asn Ile Glu Glu Lys Ser Gly Met Glu Gly Arg Lys Lys Met Phe
565 570 575

Glu Ala Gly Glu Ser Glu Leu Met Val Ser Lys Gly Glu Glu Leu Phe
580 585 590

Thr Gly Val Val Pro Ile Leu Val Glu Leu Asp Gly Asp Val Asn Gly
595 600 605

His Lys Phe Ser Val Ser Gly Glu Gly Glu Gly Asp Ala Thr Tyr Gly
610 615 620

Lys Leu Thr Leu Lys Phe Ile Cys Thr Thr Gly Lys Leu Pro Val Pro
625 630 635 640

Trp Pro Thr Leu Val Thr Thr Phe Gly Tyr Gly Leu Met Cys Phe Ala
645 650 655

Arg Tyr Pro Asp His Met Arg Gln His Asp Phe Phe Lys Ser Ala Met
660 665 670

Pro Glu Gly Tyr Val Gln Glu Arg Thr Ile Phe Phe Lys Asp Asp Gly
675 680 685

Asn Tyr Lys Thr Arg Ala Glu Val Lys Phe Glu Gly Asp Thr Leu Val
690 695 700

Asn Arg Ile Glu Leu Lys Gly Ile Asp Phe Lys Glu Asp Gly Asn Ile
705 710 715 720

Leu Gly His Lys Leu Glu Tyr Asn Tyr Asn Ser His Asn Val Tyr Ile
725 730 735

Met Ala Asp Lys Gln Lys Asn Gly Ile Lys Ala Asn Phe Lys Ile Arg
740 745 750

His Asn Ile Glu Asp Gly Ser Val Gln Leu Ala Asp His Tyr Gln Gln
755 760 765

Asn Thr Pro Ile Gly Asp Gly Pro Val Leu Leu Pro Asp Asn His Tyr
770 775 780

Leu Ser Tyr Gln Ser Ala Leu Ser Lys Asp Pro Asn Glu Lys Arg Asp
785 790 795 800

His Met Val Leu Leu Glu Phe Val Thr Ala Ala Gly Ile Thr Leu Gly
805 810 815

Met Asp Glu Leu Tyr Lys
820

<210> 13
<211> 1959
<212> DNA
<213> Artificial Sequence

<220>
<223> calcium binding moiety: csTnI 116-135 - Gly-Gly - csTnc

<400> 13

```
atggtgagca agggcgagga gctgttcacc ggggtggtgc ccatcctggt cgagctggac     60
ggcgacgtaa acggccacag gttcagcgtg tccggcgagg gcgagggcga tgccacctac    120
ggcaagctga ccctgaagtt catctgcacc accggcaagc tgcccgtgcc ctggcccacc    180
ctcgtgacca ccctgacctg gggcgtgcag tgcttcagcc gctaccccga ccacatgaag    240
cagcacgact tcttcaagtc cgccatgccc gaaggctacg tccaggagcg taccatcttc    300
ttcaaggacg acggcaacta caagacccgc gccgaggtga agttcgaggg cgacaccctg    360
gtgaaccgca tcgagctgaa gggcatcgac ttcaaggagg acggcaacat cctggggcac    420
aagctggagt acaactacat cagccacaac gtctatatca ccgccgacaa gcagaagaac    480
ggcatcaagg cccacttcaa gatccgccac aacatcgagg acggcagcgt gcagctcgcc    540
gaccactacc agcagaacac ccccatcggc gacggccccg tgctgctgcc cgacaaccac    600
tacctgagca cccagtccgc cctgagcaaa gaccccaacg agaagcgcga tcacatggtc    660
ctgctggagt tcgtgaccgc cgcccgcatg ctcgctgatg ccatgctgcg tgccctgctg    720
ggctccaagc acaaggtcaa cggcggcgcg tcaatgacgg accagcaggc ggaggcccgc    780
gccttcctca gcgaggagat gattgctgag ttcaaagctg cctttgacat gtttgatgcg    840
gacggtggtg gggacatcag caccaaggag ttgggcacgg tgatgaggat gctgggccag    900
aaccccacca aagaggagct ggatgccatc atcgaggagg tggacgagga tggcagcggc    960
accatcgact tcgaggagtt cctggtgatg atggtgcgcc agatgaaaga ggacgccaag   1020
ggcaagtctg aggaggagct ggccaactgc ttccgcatct tcgacaagaa cgctgatggg   1080
ttcatcgaca tcgaggagct gggtgagatt ctcagggcca ctggggagca cgtcatcgag   1140
```

gaggacatag aagacctcat gaaggattca gacaagaaca atgacggccg cattgacttc 1200
gatgagttcc tgaagatgat ggagggtgtg caggagctca tggtgagcaa gggcgaggag 1260
ctgttcaccg gggtggtgcc catcctggtc gagctggacg gcgacgtaaa cggccacaag 1320
ttcagcgtgt ccggcgaggg cgagggcgat gccacctacg gcaagctgac cctgaagttc 1380
atctgcacca ccggcaagct gcccgtgccc tggcccaccc tcgtgaccac cttcggctac 1440
ggcctgatgt gcttcgcccg ctaccccgac cacatgcgcc agcacgactt cttcaagtcc 1500
gccatgcccg aaggctacgt ccaggagcgc accatcttct tcaaggacga cggcaactac 1560
aagacccgcg ccgaggtgaa gttcgagggc gacaccctgg tgaaccgcat cgagctgaag 1620
ggcatcgact tcaaggagga cggcaacatc ctggggcaca agctggagta caactacaac 1680
agccacaacg tctatatcat ggccgacaag cagaagaacg gcatcaaggc caacttcaag 1740
atccgccaca acatcgagga cggcagcgtg cagctcgccg accactacca gcagaacacc 1800
cccatcggcg acggccccgt gctgctgccc gacaaccact acctgagcta ccagtccgcc 1860
ctgagcaaag accccaacga gaagcgcgat cacatggtcc tgctggagtt cgtgaccgcc 1920
gccgggatca ctctcggcat ggacgagctg tacaagtaa 1959

<210> 14
<211> 652
<212> PRT
<213> Artificial Sequence

<220>
<223> calcium binding moiety: csTnI 116-135 - Gly-Gly - csTnC

<400> 14

Met Val Ser Lys Gly Glu Glu Leu Phe Thr Gly Val Val Pro Ile Leu
1 5 10 15

Val Glu Leu Asp Gly Asp Val Asn Gly His Arg Phe Ser Val Ser Gly
20 25 30

Glu Gly Glu Gly Asp Ala Thr Tyr Gly Lys Leu Thr Leu Lys Phe Ile
35 40 45

Cys Thr Thr Gly Lys Leu Pro Val Pro Trp Pro Thr Leu Val Thr Thr
50 55 60

Leu Thr Trp Gly Val Gln Cys Phe Ser Arg Tyr Pro Asp His Met Lys
65 70 75 80

Gln His Asp Phe Phe Lys Ser Ala Met Pro Glu Gly Tyr Val Gln Glu
85 90 95

Arg Thr Ile Phe Phe Lys Asp Asp Gly Asn Tyr Lys Thr Arg Ala Glu
100 105 110

Val Lys Phe Glu Gly Asp Thr Leu Val Asn Arg Ile Glu Leu Lys Gly
115 120 125

Ile Asp Phe Lys Glu Asp Gly Asn Ile Leu Gly His Lys Leu Glu Tyr
130 135 140

Asn Tyr Ile Ser His Asn Val Tyr Ile Thr Ala Asp Lys Gln Lys Asn
145 150 155 160

Gly Ile Lys Ala His Phe Lys Ile Arg His Asn Ile Glu Asp Gly Ser
165 170 175

Val Gln Leu Ala Asp His Tyr Gln Gln Asn Thr Pro Ile Gly Asp Gly
180 185 190

Pro Val Leu Leu Pro Asp Asn His Tyr Leu Ser Thr Gln Ser Ala Leu
195 200 205

Ser Lys Asp Pro Asn Glu Lys Arg Asp His Met Val Leu Leu Glu Phe
210 215 220

Val Thr Ala Ala Arg Met Leu Ala Asp Ala Met Leu Arg Ala Leu Leu
225 230 235 240

Gly Ser Lys His Lys Val Asn Gly Gly Ala Ser Met Thr Asp Gln Gln
245 250 255

Ala Glu Ala Arg Ala Phe Leu Ser Glu Glu Met Ile Ala Glu Phe Lys
260 265 270

Ala Ala Phe Asp Met Phe Asp Ala Asp Gly Gly Gly Asp Ile Ser Thr
275 280 285

Lys Glu Leu Gly Thr Val Met Arg Met Leu Gly Gln Asn Pro Thr Lys
290 295 300

Glu Glu Leu Asp Ala Ile Ile Glu Glu Val Asp Glu Asp Gly Ser Gly
305 310 315 320

Thr Ile Asp Phe Glu Glu Phe Leu Val Met Met Val Arg Gln Met Lys
325 330 335

Glu Asp Ala Lys Gly Lys Ser Glu Glu Glu Leu Ala Asn Cys Phe Arg
340 345 350

Ile Phe Asp Lys Asn Ala Asp Gly Phe Ile Asp Ile Glu Glu Leu Gly
355 360 365

Glu Ile Leu Arg Ala Thr Gly Glu His Val Ile Glu Glu Asp Ile Glu
370 375 380

Asp Leu Met Lys Asp Ser Asp Lys Asn Asn Asp Gly Arg Ile Asp Phe
385 390 395 400

Asp Glu Phe Leu Lys Met Met Glu Gly Val Gln Glu Leu Met Val Ser
405 410 415

Lys Gly Glu Glu Leu Phe Thr Gly Val Val Pro Ile Leu Val Glu Leu
420 425 430

Asp Gly Asp Val Asn Gly His Lys Phe Ser Val Ser Gly Glu Gly Glu
435 440 445

Gly Asp Ala Thr Tyr Gly Lys Leu Thr Leu Lys Phe Ile Cys Thr Thr
450 455 460

Gly Lys Leu Pro Val Pro Trp Pro Thr Leu Val Thr Thr Phe Gly Tyr
465 470 475 480

Gly Leu Met Cys Phe Ala Arg Tyr Pro Asp His Met Arg Gln His Asp
485 490 495

Phe Phe Lys Ser Ala Met Pro Glu Gly Tyr Val Gln Glu Arg Thr Ile
500 505 510

Phe Phe Lys Asp Asp Gly Asn Tyr Lys Thr Arg Ala Glu Val Lys Phe
515 520 525

Glu Gly Asp Thr Leu Val Asn Arg Ile Glu Leu Lys Gly Ile Asp Phe
530 535 540

Lys Glu Asp Gly Asn Ile Leu Gly His Lys Leu Glu Tyr Asn Tyr Asn
545 550 555 560

Ser His Asn Val Tyr Ile Met Ala Asp Lys Gln Lys Asn Gly Ile Lys
565 570 575

Ala Asn Phe Lys Ile Arg His Asn Ile Glu Asp Gly Ser Val Gln Leu
580 585 590

Ala Asp His Tyr Gln Gln Asn Thr Pro Ile Gly Asp Gly Pro Val Leu
595 600 605

Leu Pro Asp Asn His Tyr Leu Ser Tyr Gln Ser Ala Leu Ser Lys Asp
610 615 620

Pro Asn Glu Lys Arg Asp His Met Val Leu Leu Glu Phe Val Thr Ala
625 630 635 640

```
Ala Gly Ile Thr Leu Gly Met Asp Glu Leu Tyr Lys
                645                 650
```

<210> 15
<211> 1827
<212> DNA
<213> Artificial Sequence

<220>
<223> calcium binding moiety: csTnI 95-131 - Gly-Ser-Gly - csTnC 1-91

<400> 15

```
atggtgagca agggcgagga gctgttcacc ggggtggtgc ccatcctggt cgagctggac     60
ggcgacgtaa acggccacag gttcagcgtg tccggcgagg gcgagggcga tgccacctac    120
ggcaagctga ccctgaagtt catctgcacc accggcaagc tgcccgtgcc ctggcccacc    180
ctcgtgacca ccctgacctg gggcgtgcag tgcttcagcc gctaccccga ccacatgaag    240
cagcacgact tcttcaagtc cgccatgccc gaaggctacg tccaggagcg taccatcttc    300
ttcaaggacg acggcaacta caagacccgc gccgaggtga agttcgaggg cgacaccctg    360
gtgaaccgca tcgagctgaa gggcatcgac ttcaaggagg acggcaacat cctggggcac    420
aagctggagt acaactacat cagccacaac gtctatatca ccgccgacaa gcagaagaac    480
ggcatcaagg cccacttcaa gatccgccac aacatcgagg acggcagcgt gcagctcgcc    540
gaccactacc agcagaacac ccccatcggc gacggccccg tgctgctgcc cgacaaccac    600
tacctgagca cccagtccgc cctgagcaaa gaccccaacg agaagcgcga tcacatggtc    660
ctgctggagt tcgtgaccgc cgcccgcatg ctagacctga gccagaagct gtttgacctg    720
aggggcaagt tcaagaggcc acctctgcgc cgggtgcgca tgtctgctga tgccatgctg    780
cgtgccctgc tgggctccaa gcacaaggtc ggcagcggca gcatgctaat ggcgtcaatg    840
acggaccagc aggcggaggc ccgcgccttc ctcagcgagg agatgattgc tgagttcaaa    900
gctgcctttg acatgtttga tgcggacggt ggtggggaca tcagcaccaa ggagttgggc    960
acggtgatga ggatgctggg ccagaacccc accaaagagg agctggatgc catcatcgag   1020
gaggtggacg aggatggcag cggcaccatc gacttcgagg agttcctggt gatgatggtg   1080
cgccagatga aagaggacgc cgagctcatg gtgagcaagg gcgaggagct gttcaccggg   1140
gtggtgccca tcctggtcga gctggacggc gacgtaaacg gccacaagtt cagcgtgtcc   1200
ggcgagggcg agggcgatgc cacctacggc aagctgaccc tgaagttcat ctgcaccacc   1260
ggcaagctgc ccgtgccctg gcccaccctc gtgaccacct tcggctacgg cctgatgtgc   1320
ttcgcccgct accccgacca catgcgccag cacgacttct tcaagtccgc catgcccgaa   1380
ggctacgtcc aggagcgcac catcttcttc aaggacgacg gcaactacaa gacccgcgcc   1440
```

gaggtgaagt tcgagggcga caccctggtg aaccgcatcg agctgaaggg catcgacttc 1500
aaggaggacg gcaacatcct ggggcacaag ctggagtaca actacaacag ccacaacgtc 1560
tatatcatgg ccgacaagca gaagaacggc atcaaggcca acttcaagat ccgccacaac 1620
atcgaggacg gcagcgtgca gctcgccgac cactaccagc agaacacccc catcggcgac 1680
ggccccgtgc tgctgcccga caaccactac ctgagctacc agtccgccct gagcaaagac 1740
cccaacgaga agcgcgatca catggtcctg ctggagttcg tgaccgccgc cgggatcact 1800
ctcggcatgg acgagctgta caagtaa 1827

<210> 16
<211> 608
<212> PRT
<213> Artificial Sequence

<220>
<223> Calcium binding moiety: csTnI 95-131 - Gly-Ser-Gly - csTnC 1-91

<400> 16

Met Val Ser Lys Gly Glu Glu Leu Phe Thr Gly Val Val Pro Ile Leu
1 5 10 15

Val Glu Leu Asp Gly Asp Val Asn Gly His Arg Phe Ser Val Ser Gly
20 25 30

Glu Gly Glu Gly Asp Ala Thr Tyr Gly Lys Leu Thr Leu Lys Phe Ile
35 40 45

Cys Thr Thr Gly Lys Leu Pro Val Pro Trp Pro Thr Leu Val Thr Thr
50 55 60

Leu Thr Trp Gly Val Gln Cys Phe Ser Arg Tyr Pro Asp His Met Lys
65 70 75 80

Gln His Asp Phe Phe Lys Ser Ala Met Pro Glu Gly Tyr Val Gln Glu
85 90 95

Arg Thr Ile Phe Phe Lys Asp Asp Gly Asn Tyr Lys Thr Arg Ala Glu
100 105 110

Val Lys Phe Glu Gly Asp Thr Leu Val Asn Arg Ile Glu Leu Lys Gly
115 120 125

Ile Asp Phe Lys Glu Asp Gly Asn Ile Leu Gly His Lys Leu Glu Tyr
130 135 140

Asn Tyr Ile Ser His Asn Val Tyr Ile Thr Ala Asp Lys Gln Lys Asn
145 150 155 160

Gly Ile Lys Ala His Phe Lys Ile Arg His Asn Ile Glu Asp Gly Ser
165 170 175

Val Gln Leu Ala Asp His Tyr Gln Gln Asn Thr Pro Ile Gly Asp Gly
180 185 190

Pro Val Leu Leu Pro Asp Asn His Tyr Leu Ser Thr Gln Ser Ala Leu
195 200 205

Ser Lys Asp Pro Asn Glu Lys Arg Asp His Met Val Leu Leu Glu Phe
210 215 220

Val Thr Ala Ala Arg Met Leu Asp Leu Ser Gln Lys Leu Phe Asp Leu
225 230 235 240

Arg Gly Lys Phe Lys Arg Pro Pro Leu Arg Arg Val Arg Met Ser Ala
245 250 255

Asp Ala Met Leu Arg Ala Leu Leu Gly Ser Lys His Lys Val Gly Ser
260 265 270

Gly Ser Met Leu Met Ala Ser Met Thr Asp Gln Gln Ala Glu Ala Arg
275 280 285

Ala Phe Leu Ser Glu Glu Met Ile Ala Glu Phe Lys Ala Ala Phe Asp
290 295 300

Met Phe Asp Ala Asp Gly Gly Gly Asp Ile Ser Thr Lys Glu Leu Gly
305 310 315 320

Thr Val Met Arg Met Leu Gly Gln Asn Pro Thr Lys Glu Glu Leu Asp
325 330 335

Ala Ile Ile Glu Glu Val Asp Glu Asp Gly Ser Gly Thr Ile Asp Phe
340 345 350

Glu Glu Phe Leu Val Met Met Val Arg Gln Met Lys Glu Asp Ala Glu
355 360 365

Leu Met Val Ser Lys Gly Glu Glu Leu Phe Thr Gly Val Val Pro Ile
370 375 380

Leu Val Glu Leu Asp Gly Asp Val Asn Gly His Lys Phe Ser Val Ser
385 390 395 400

Gly Glu Gly Glu Gly Asp Ala Thr Tyr Gly Lys Leu Thr Leu Lys Phe
405 410 415

Ile Cys Thr Thr Gly Lys Leu Pro Val Pro Trp Pro Thr Leu Val Thr
420 425 430

Thr Phe Gly Tyr Gly Leu Met Cys Phe Ala Arg Tyr Pro Asp His Met
435 440 445

Arg Gln His Asp Phe Phe Lys Ser Ala Met Pro Glu Gly Tyr Val Gln
450 455 460

Glu Arg Thr Ile Phe Phe Lys Asp Asp Gly Asn Tyr Lys Thr Arg Ala
465 470 475 480

Glu Val Lys Phe Glu Gly Asp Thr Leu Val Asn Arg Ile Glu Leu Lys
485 490 495

Gly Ile Asp Phe Lys Glu Asp Gly Asn Ile Leu Gly His Lys Leu Glu
500 505 510

Tyr Asn Tyr Asn Ser His Asn Val Tyr Ile Met Ala Asp Lys Gln Lys
515 520 525

Asn Gly Ile Lys Ala Asn Phe Lys Ile Arg His Asn Ile Glu Asp Gly
530 535 540

Ser Val Gln Leu Ala Asp His Tyr Gln Gln Asn Thr Pro Ile Gly Asp
545 550 555 560

Gly Pro Val Leu Leu Pro Asp Asn His Tyr Leu Ser Tyr Gln Ser Ala
565 570 575

Leu Ser Lys Asp Pro Asn Glu Lys Arg Asp His Met Val Leu Leu Glu
580 585 590

Phe Val Thr Ala Ala Gly Ile Thr Leu Gly Met Asp Glu Leu Tyr Lys
595 600 605

<210> 17
<211> 1869
<212> DNA
<213> Artificial Sequence

<220>
<223> Calcium binding moiety: csTnI 12-161

<400> 17

atggtgagca agggcgagga gctgttcacc ggggtggtgc ccatcctggt cgagctggac 60
ggcgacgtaa acggccacag gttcagcgtg tccggcgagg gcgagggcga tgccacctac 120
ggcaagctga ccctgaagtt catctgcacc accggcaagc tgcccgtgcc ctggcccacc 180

ctcgtgacca ccctgacctg gggcgtgcag tgcttcagcc gctaccccga ccacatgaag 240
cagcacgact tcttcaagtc cgccatgccc gaaggctacg tccaggagcg taccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg cccacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgcccgcatg ctgctgacag aagagcagaa aaatgagttc 720
aaggcagcct tcgacatctt cgtgctgggc gctgaggatg gctgcatcag caccaaggag 780
ctgggcaagg tgatgaggat gctgggccag aaccccaccc ctgaggagct gcaggagatg 840
atcgatgagg tggacgagga cggcagcggc acggtggact ttgatgagtt cctggtcatg 900
atggttcggt gcatgaagga cgacagcaaa gggaaatctg aggaggagct gtctgacctc 960
ttccgcatgt ttgacaaaaa tgctgatggc tacatcgacc tggatgagct gaagataatg 1020
ctgcaggcta caggcgagac catcacggag gacgacatcg aggaactcat gaaggacgga 1080
gacaagaaca acgacggccg catcgactat gatgagttcc tggagttcat gaagggtgtg 1140
gaggagctca tggtgagcaa gggcgaggag ctgttcaccg gggtggtgcc catcctggtc 1200
gagctggacg gcgacgtaaa cggccacaag ttcagcgtgt ccggcgaggg cgagggcgat 1260
gccacctacg gcaagctgac cctgaagttc atctgcacca ccggcaagct gcccgtgccc 1320
tggcccaccc tcgtgaccac cttcggctac ggcctgatgt gcttcgcccg ctaccccgac 1380
cacatgcgcc agcacgactt cttcaagtcc gccatgcccg aaggctacgt ccaggagcgc 1440
accatcttct tcaaggacga cggcaactac aagacccgcg ccgaggtgaa gttcgagggc 1500
gacaccctgg tgaaccgcat cgagctgaag ggcatcgact tcaaggagga cggcaacatc 1560
ctggggcaca agctggagta caactacaac agccacaacg tctatatcat ggccgacaag 1620
cagaagaacg gcatcaaggc caacttcaag atccgccaca acatcgagga cggcagcgtg 1680
cagctcgccg accactacca gcagaacacc cccatcggcg acggccccgt gctgctgccc 1740
gacaaccact acctgagcta ccagtccgcc ctgagcaaag accccaacga gaagcgcgat 1800
cacatggtcc tgctggagtt cgtgaccgcc gccgggatca ctctcggcat ggacgagctg 1860
tacaagtaa 1869

<210> 18
<211> 622
<212> PRT
<213> Artificial sequence

<220>
<223> Calcium binding moiety: csTnI 12-161

<400> 18

Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Leu Thr Glu Glu Gln Lys Asn Glu Phe
225                 230                 235                 240

```
Lys Ala Ala Phe Asp Ile Phe Val Leu Gly Ala Glu Asp Gly Cys Ile
                245                 250                 255

Ser Thr Lys Glu Leu Gly Lys Val Met Arg Met Leu Gly Gln Asn Pro
            260                 265                 270

Thr Pro Glu Glu Leu Gln Glu Met Ile Asp Glu Val Asp Glu Asp Gly
        275                 280                 285

Ser Gly Thr Val Asp Phe Asp Glu Phe Leu Val Met Met Val Arg Cys
    290                 295                 300

Met Lys Asp Asp Ser Lys Gly Lys Ser Glu Glu Glu Leu Ser Asp Leu
305                 310                 315                 320

Phe Arg Met Phe Asp Lys Asn Ala Asp Gly Tyr Ile Asp Leu Asp Glu
                325                 330                 335

Leu Lys Ile Met Leu Gln Ala Thr Gly Glu Thr Ile Thr Glu Asp Asp
            340                 345                 350

Ile Glu Glu Leu Met Lys Asp Gly Asp Lys Asn Asn Asp Gly Arg Ile
        355                 360                 365

Asp Tyr Asp Glu Phe Leu Glu Phe Met Lys Gly Val Glu Glu Leu Met
    370                 375                 380

Val Ser Lys Gly Glu Glu Leu Phe Thr Gly Val Val Pro Ile Leu Val
385                 390                 395                 400

Glu Leu Asp Gly Asp Val Asn Gly His Lys Phe Ser Val Ser Gly Glu
                405                 410                 415

Gly Glu Gly Asp Ala Thr Tyr Gly Lys Leu Thr Leu Lys Phe Ile Cys
            420                 425                 430

Thr Thr Gly Lys Leu Pro Val Pro Trp Pro Thr Leu Val Thr Thr Phe
        435                 440                 445

Gly Tyr Gly Leu Met Cys Phe Ala Arg Tyr Pro Asp His Met Arg Gln
    450                 455                 460

His Asp Phe Phe Lys Ser Ala Met Pro Glu Gly Tyr Val Gln Glu Arg
465                 470                 475                 480

Thr Ile Phe Phe Lys Asp Asp Gly Asn Tyr Lys Thr Arg Ala Glu Val
                485                 490                 495

Lys Phe Glu Gly Asp Thr Leu Val Asn Arg Ile Glu Leu Lys Gly Ile
            500                 505                 510
```

Asp Phe Lys Glu Asp Gly Asn Ile Leu Gly His Lys Leu Glu Tyr Asn
515 520 525

Tyr Asn Ser His Asn Val Tyr Ile Met Ala Asp Lys Gln Lys Asn Gly
530 535 540

Ile Lys Ala Asn Phe Lys Ile Arg His Asn Ile Glu Asp Gly Ser Val
545 550 555 560

Gln Leu Ala Asp His Tyr Gln Gln Asn Thr Pro Ile Gly Asp Gly Pro
565 570 575

Val Leu Leu Pro Asp Asn His Tyr Leu Ser Tyr Gln Ser Ala Leu Ser
580 585 590

Lys Asp Pro Asn Glu Lys Arg Asp His Met Val Leu Leu Glu Phe Val
595 600 605

Thr Ala Ala Gly Ile Thr Leu Gly Met Asp Glu Leu Tyr Lys
610 615 620

<210> 19
<211> 486
<212> DNA
<213> Homo sapiens

<400> 19

atggatgaca tctacaaggc tgcggtagag cagctgacag aagagcagaa aaatgagttc 60
aaggcagcct tcgacatctt cgtgctgggc gctgaggatg gctgcatcag caccaaggag 120
ctgggcaagg tgatgaggat gctgggccag aaccccaccc ctgaggagct gcaggagatg 180
atcgatgagg tggacgagga cggcagcggc acggtggact ttgatgagtt cctggtcatg 240
atggttcggt gcatgaagga cgacagcaaa gggaaatctg aggaggagct gtctgacctc 300
ttccgcatgt ttgacaaaaa tgctgatggc tacatcgacc tggatgagct gaagataatg 360
ctgcaggcta caggcgagac catcacggag gacgacatcg aggagctcat gaaggacgga 420
gacaagaaca acgacggccg catcgactat gatgagttcc tggagttcat gaagggtgtg 480
gagtag 486

<210> 20
<211> 161
<212> PRT
<213> Homo sapiens

<400> 20

Met Asp Asp Ile Tyr Lys Ala Ala Val Glu Gln Leu Thr Glu Glu Gln
1 5 10 15

Lys Asn Glu Phe Lys Ala Ala Phe Asp Ile Phe Val Leu Gly Ala Glu
20 25 30

Asp Gly Cys Ile Ser Thr Lys Glu Leu Gly Lys Val Met Arg Met Leu
35 40 45

Gly Gln Asn Pro Thr Pro Glu Glu Leu Gln Glu Met Ile Asp Glu Val
50 55 60

Asp Glu Asp Gly Ser Gly Thr Val Asp Phe Asp Glu Phe Leu Val Met
65 70 75 80

Met Val Arg Cys Met Lys Asp Asp Ser Lys Gly Lys Ser Glu Glu Glu
85 90 95

Leu Ser Asp Leu Phe Arg Met Phe Asp Lys Asn Ala Asp Gly Tyr Ile
100 105 110

Asp Leu Asp Glu Leu Lys Ile Met Leu Gln Ala Thr Gly Glu Thr Ile
115 120 125

Thr Glu Asp Asp Ile Glu Glu Leu Met Lys Asp Gly Asp Lys Asn Asn
130 135 140

Asp Gly Arg Ile Asp Tyr Asp Glu Phe Leu Glu Phe Met Lys Gly Val
145 150 155 160

Glu

<210> 21
<211> 633
<212> DNA
<213> Homo sapiens

<400> 21

atggcggatg ggagcagcga tgcggctagg gaacctcgcc ctgcaccagc cccaatcaga 60
cgccgctcct ccaactaccg cgcttatgcc acggagccgc acgccaagaa aaaatctaag 120
atctccgcct cgagaaaatt gcagctgaag actctgctgc tgcagattgc aaagcaagag 180
ctggagcgag aggcggagga gcggcgcgga gagaaggggc gcgctctgag cacccgctgc 240
cagccgctgg agttgaccgg gctgggcttc gcggagctgc aggacttgtg ccgacagctc 300
cacgcccgtg tggacaaggt ggatgaagag agatacgaca tagaggcaaa agtcaccaag 360

```
aacatcacgg agattgcaga tctgactcag aagatctttg accttcgagg caagtttaag     420
cggcccaccc tgcggagagt gaggatctct gcagatgcca tgatgcaggc gctgctgggg     480
gcccgggcta aggagtccct ggacctgcgg gcccacctca agcaggtgaa gaaggaggac     540
accgagaagg aaaaccggga ggtgggagac tggcggaaga acatcgatgc actgagtgga     600
atggagggcc gcaagaaaaa gtttgagagc tga                                  633
```

<210> 22
<211> 210
<212> PRT
<213> Homo sapiens

<400> 22

```
Met Ala Asp Gly Ser Ser Asp Ala Ala Arg Glu Pro Arg Pro Ala Pro
1               5                   10                  15

Ala Pro Ile Arg Arg Arg Ser Ser Asn Tyr Arg Ala Tyr Ala Thr Glu
            20                  25                  30

Pro His Ala Lys Lys Lys Ser Lys Ile Ser Ala Ser Arg Lys Leu Gln
        35                  40                  45

Leu Lys Thr Leu Leu Leu Gln Ile Ala Lys Gln Glu Leu Glu Arg Glu
    50                  55                  60

Ala Glu Glu Arg Arg Gly Glu Lys Gly Arg Ala Leu Ser Thr Arg Cys
65                  70                  75                  80

Gln Pro Leu Glu Leu Thr Gly Leu Gly Phe Ala Glu Leu Gln Asp Leu
                85                  90                  95

Cys Arg Gln Leu His Ala Arg Val Asp Lys Val Asp Glu Glu Arg Tyr
            100                 105                 110

Asp Ile Glu Ala Lys Val Thr Lys Asn Ile Thr Glu Ile Ala Asp Leu
        115                 120                 125

Thr Gln Lys Ile Phe Asp Leu Arg Gly Lys Phe Lys Arg Pro Thr Leu
    130                 135                 140

Arg Arg Val Arg Ile Ser Ala Asp Ala Met Met Gln Ala Leu Leu Gly
145                 150                 155                 160

Ala Arg Ala Lys Glu Ser Leu Asp Leu Arg Ala His Leu Lys Gln Val
                165                 170                 175
```

Lys Lys Glu Asp Thr Glu Lys Glu Asn Arg Glu Val Gly Asp Trp Arg
180 185 190

Lys Asn Ile Asp Ala Leu Ser Gly Met Glu Gly Arg Lys Lys Lys Phe
195 200 205

Glu Ser
210

<210> 23
<211> 483
<212> DNA
<213> Homo sapiens

<400> 23

atgacggacc agcaggctga ggccaggtcc tacctcagcg aagagatgat cgctgagttc 60
aaggctgcct ttgacatgtt tgatgctgat ggtggtgggg acatcagcgt caaggagttg 120
ggcacggtga tgaggatgct gggccagaca cccaccaagg aggagctgga cgccatcatc 180
gaggaggtgg atgaggacgg cagcggcacc atcgacttcg aggagttctt ggtcatgatg 240
gtgcgccaga tgaaagagga cgcgaaaggg aagagcgagg aggagctggc cgagtgcttc 300
cgcatcttcg acaggaatgc agacggctac atcgacccgg aggagctggc tgagattttc 360
agggcctccg gggagcacgt gactgacgag gagatcgaat ctctgatgaa agacggcgac 420
aagaacaacg acggccgcat tgacttcgac gagttcctga agatgatgga gggcgtgcag 480
taa 483

<210> 24
<211> 160
<212> PRT
<213> Homo sapiens

<400> 24

Met Thr Asp Gln Gln Ala Glu Ala Arg Ser Tyr Leu Ser Glu Glu Met
1 5 10 15

Ile Ala Glu Phe Lys Ala Ala Phe Asp Met Phe Asp Ala Asp Gly Gly
20 25 30

Gly Asp Ile Ser Val Lys Glu Leu Gly Thr Val Met Arg Met Leu Gly
35 40 45

Gln Thr Pro Thr Lys Glu Glu Leu Asp Ala Ile Ile Glu Glu Val Asp
50 55 60

Glu Asp Gly Ser Gly Thr Ile Asp Phe Glu Glu Phe Leu Val Met Met
65 70 75 80

Val Arg Gln Met Lys Glu Asp Ala Lys Gly Lys Ser Glu Glu Glu Leu
85 90 95

Ala Glu Cys Phe Arg Ile Phe Asp Arg Asn Ala Asp Gly Tyr Ile Asp
100 105 110

Pro Glu Glu Leu Ala Glu Ile Phe Arg Ala Ser Gly Glu His Val Thr
115 120 125

Asp Glu Glu Ile Glu Ser Leu Met Lys Asp Gly Asp Lys Asn Asn Asp
130 135 140

Gly Arg Ile Asp Phe Asp Glu Phe Leu Lys Met Met Glu Gly Val Gln
145 150 155 160

<210> 25
<211> 492
<212> DNA
<213> Gallus gallus

<400> 25

atggcgtcaa tgacggacca gcaggcggag gcccgcgcct tcctcagcga ggagatgatt 60
gctgagttca aagctgcctt tgacatgttt gatgcggacg gtggtgggga catcagcacc 120
aaggagttgg gcacggtgat gaggatgctg ggccagaacc ccaccaaaga ggagctggat 180
gccatcatcg aggaggtgga cgaggatggc agcggcacca tcgacttcga ggagttcctg 240
gtgatgatgg tgcgccagat gaaagaggac gccaagggca agtctgagga ggagctggcc 300
aactgcttcc gcatcttcga caagaacgct gatgggttca tcgacatcga ggagctgggt 360
gagattctca gggccactgg ggagcacgtc atcgaggagg acatagaaga cctcatgaag 420
gattcagaca agaacaatga cggccgcatt gacttcgatg agttcctgaa gatgatggag 480
ggtgtgcagt aa 492

<210> 26
<211> 163
<212> PRT
<213> Gallus gallus

<400> 26

Met Ala Ser Met Thr Asp Gln Gln Ala Glu Ala Arg Ala Phe Leu Ser
1 5 10 15

Glu Glu Met Ile Ala Glu Phe Lys Ala Ala Phe Asp Met Phe Asp Ala
20 25 30

Asp Gly Gly Gly Asp Ile Ser Thr Lys Glu Leu Gly Thr Val Met Arg
35 40 45

Met Leu Gly Gln Asn Pro Thr Lys Glu Glu Leu Asp Ala Ile Ile Glu
50 55 60

Glu Val Asp Glu Asp Gly Ser Gly Thr Ile Asp Phe Glu Glu Phe Leu
65 70 75 80

Val Met Met Val Arg Gln Met Lys Glu Asp Ala Lys Gly Lys Ser Glu
85 90 95

Glu Glu Leu Ala Asn Cys Phe Arg Ile Phe Asp Lys Asn Ala Asp Gly
100 105 110

Phe Ile Asp Ile Glu Glu Leu Gly Glu Ile Leu Arg Ala Thr Gly Glu
115 120 125

His Val Ile Glu Glu Asp Ile Glu Asp Leu Met Lys Asp Ser Asp Lys
130 135 140

Asn Asn Asp Gly Arg Ile Asp Phe Asp Glu Phe Leu Lys Met Met Glu
145 150 155 160

Gly Val Gln

<210> 27
<211> 552
<212> DNA
<213> Gallus gallus

<400> 27

atgtctgatg aagagaaaaa gaggagggca gccaccgccc ggcgccagca cctgaagagt 60
gctatgctcc agcttgctgt cactgaaata gaaaaagaag cagctgctaa agaagtggaa 120
aagcaaaact acctggcaga gcattgccct cctctgtccc tcccaggatc catgcaggaa 180
cttcaggaac tgtgcaaaaa gcttcatgcc aagatagact cagtggatga ggaaaggtat 240
gacacagagg tgaagctaca gaagactaac aaggagctgg aggacctgag ccagaagctg 300
tttgacctga ggggcaagtt caagaggcca cctctgcgcc gggtgcgcat gtctgctgat 360
gccatgctgc gtgccctgct gggctccaag cacaaggtca acatggacct ccgggccaac 420

ctgaagcaag tcaagaagga ggacacggag aaggagaagg acctccgcga tgtgggtgac 480

tggaggaaga acattgagga gaaatctggc atggagggca ggaagaagat gtttgaggcc 540

ggcgagtcct aa 552

<210> 28
<211> 183
<212> PRT
<213> Gallus gallus

<400> 28

Met Ser Asp Glu Glu Lys Lys Arg Arg Ala Ala Thr Ala Arg Arg Gln
1 5 10 15

His Leu Lys Ser Ala Met Leu Gln Leu Ala Val Thr Glu Ile Glu Lys
20 25 30

Glu Ala Ala Ala Lys Glu Val Glu Lys Gln Asn Tyr Leu Ala Glu His
35 40 45

Cys Pro Pro Leu Ser Leu Pro Gly Ser Met Gln Glu Leu Gln Glu Leu
50 55 60

Cys Lys Lys Leu His Ala Lys Ile Asp Ser Val Asp Glu Glu Arg Tyr
65 70 75 80

Asp Thr Glu Val Lys Leu Gln Lys Thr Asn Lys Glu Leu Glu Asp Leu
85 90 95

Ser Gln Lys Leu Phe Asp Leu Arg Gly Lys Phe Lys Arg Pro Pro Leu
100 105 110

Arg Arg Val Arg Met Ser Ala Asp Ala Met Leu Arg Ala Leu Leu Gly
115 120 125

Ser Lys His Lys Val Asn Met Asp Leu Arg Ala Asn Leu Lys Gln Val
130 135 140

Lys Lys Glu Asp Thr Glu Lys Glu Lys Asp Leu Arg Asp Val Gly Asp
145 150 155 160

Trp Arg Lys Asn Ile Glu Glu Lys Ser Gly Met Glu Gly Arg Lys Lys
165 170 175

Met Phe Glu Ala Gly Glu Ser
180

<210> 29
<211> 486
<212> DNA

<213> Gallus gallus

<400> 29

```
atggatgaca tctataaggc ggcggttgag cagttgacag aagaacaaaa aaatgagttt      60
aaggctgcct tcgacatctt cgtgctgggg gcagaggatg gctgcatcag caccaaggag     120
ctggggaagg tgatgaggat gctggggcag aaccccaccc ctgaggagct gcaggagatg     180
attgatgagg tggatgagga tggcagtggc actgtggact ttgatgagtt ccttgttatg     240
atggttcggt gtatgaaaga tgacagcaaa ggaaaaactg aagaggagct ctcagatctc     300
ttcaggatgt ttgataagaa tgctgatggc tacatcgatc ttgaggaact gaagatcatg     360
ctacaggcaa ctggagagac gatcactgag gatgacatag aagaactgat gaaagatggg     420
gacaaaaaca atgatggcag gattgactat gacgagttcc tggagttcat gaagggagtt     480
gaataa                                                                486
```

<210> 30
<211> 161
<212> PRT
<213> Gallus gallus

<400> 30

```
Met Asp Asp Ile Tyr Lys Ala Ala Val Glu Gln Leu Thr Glu Glu Gln
1               5                   10                  15

Lys Asn Glu Phe Lys Ala Ala Phe Asp Ile Phe Val Leu Gly Ala Glu
            20                  25                  30

Asp Gly Cys Ile Ser Thr Lys Glu Leu Gly Lys Val Met Arg Met Leu
        35                  40                  45

Gly Gln Asn Pro Thr Pro Glu Glu Leu Gln Glu Met Ile Asp Glu Val
    50                  55                  60

Asp Glu Asp Gly Ser Gly Thr Val Asp Phe Asp Glu Phe Leu Val Met
65                  70                  75                  80

Met Val Arg Cys Met Lys Asp Asp Ser Lys Gly Lys Thr Glu Glu Glu
                85                  90                  95

Leu Ser Asp Leu Phe Arg Met Phe Asp Lys Asn Ala Asp Gly Tyr Ile
            100                 105                 110
```

Asp Leu Glu Glu Leu Lys Ile Met Leu Gln Ala Thr Gly Glu Thr Ile
115 120 125

Thr Glu Asp Asp Ile Glu Glu Leu Met Lys Asp Gly Asp Lys Asn Asn
130 135 140

Asp Gly Arg Ile Asp Tyr Asp Glu Phe Leu Glu Phe Met Lys Gly Val
145 150 155 160

Glu

SEQUENCE LISTING

<110> Max-Planck-Gesellschaft

<120> Novel genetically encoded bioindicators of calcium-ions

<130> G62276EP

<160> 30

<170> PatentIn version 3.1

<210> 1
<211> 1863
<212> DNA
<213> Artificial Sequence

<220>
<223> Calcium binding moiety: csTnC 15-163

<400> 1

```
atggtgagca agggcgagga gctgttcacc ggggtggtgc ccatcctggt cgagctggac     60
ggcgacgtaa acggccacag gttcagcgtg tccggcgagg gcgagggcga tgccacctac    120
ggcaagctga ccctgaagtt catctgcacc accggcaagc tgcccgtgcc ctggcccacc    180
ctcgtgacca ccctgacctg gggcgtgcag tgcttcagcc gctaccccga ccacatgaag    240
cagcacgact tcttcaagtc cgccatgccc gaaggctacg tccaggagcg taccatcttc    300
ttcaaggacg acggcaacta caagacccgc gccgaggtga agttcgaggg cgacaccctg    360
gtgaaccgca tcgagctgaa gggcatcgac ttcaaggagg acggcaacat cctggggcac    420
aagctggagt acaactacat cagccacaac gtctatatca ccgccgacaa gcagaagaac    480
ggcatcaagg cccacttcaa gatccgccac aacatcgagg acggcagcgt gcagctcgcc    540
gaccactacc agcagaacac ccccatcggc gacggccccg tgctgctgcc cgacaaccac    600
tacctgagca cccagtccgc cctgagcaaa gaccccaacg agaagcgcga tcacatggtc    660
ctgctggagt tcgtgaccgc cgcccgcatg ctcagcgagg agatgattgc tgagttcaaa    720
gctgcctttg acatgtttga tgcggacggt ggtggggaca tcagcaccaa ggagttgggc    780
acggtgatga ggatgctggg ccagaacccc accaaagagg agctggatgc catcatcgag    840
gaggtggacg aggatggcag cggcaccatc gacttcgagg agttcctggt gatgatggtg    900

cgccagatga aagaggacgc caagggcaag tctgaggagg agctggccaa ctgcttccgc    960
atcttcgaca agaacgctga tgggttcatc gacatcgagg agctgggtga gattctcagg   1020
gccactgggg agcacgtcat cgaggaggac atagaagacc tcatgaagga ttcagacaag   1080
aacaatgacg gccgcattga cttcgatgag ttcctgaaga tgatggaggg tgtgcaggag   1140
ctcatggtga gcaagggcga ggagctgttc accggggtgg tgcccatcct ggtcgagctg   1200
gacggcgacg taaacggcca caagttcagc gtgtccggcg agggcgaggg cgatgccacc   1260
tacggcaagc tgaccctgaa gttcatctgc accaccggca agctgcccgt gccctggccc   1320
accctcgtga ccaccttcgg ctacggcctg atgtgcttcg cccgctaccc cgaccacatg   1380
cgccagcacg acttcttcaa gtccgccatg cccgaaggct acgtccagga gcgcaccatc   1440
ttcttcaagg acgacggcaa ctacaagacc cgcgccgagg tgaagttcga gggcgacacc   1500
ctggtgaacc gcatcgagct gaagggcatc gacttcaagg aggacggcaa catcctgggg   1560
cacaagctgg agtacaacta caacagccac aacgtctata tcatggccga caagcagaag   1620
aacggcatca aggccaactt caagatccgc cacaacatcg aggacggcag cgtgcagctc   1680
gccgaccact accagcagaa cacccccatc ggcgacggcc ccgtgctgct gcccgacaac   1740
cactacctga gctaccagtc cgccctgagc aaagacccca acgagaagcg cgatcacatg   1800
gtcctgctgg agttcgtgac cgccgccggg atcactctcg gcatggacga gctgtacaag   1860
taa                                                                 1863
```

<210> 2
<211> 620
<212> PRT
<213> Artificial sequence

<220>
<223> Calcium binding moiety: csTnC 15-163

<400> 2

```
Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60
```

Leu Thr Trp Gly Val Gln Cys Phe Ser Arg Tyr Pro Asp His Met Lys
65 70 75 80

Gln His Asp Phe Phe Lys Ser Ala Met Pro Glu Gly Tyr Val Gln Glu
85 90 95

Arg Thr Ile Phe Phe Lys Asp Asp Gly Asn Tyr Lys Thr Arg Ala Glu
100 105 110

Val Lys Phe Glu Gly Asp Thr Leu Val Asn Arg Ile Glu Leu Lys Gly
115 120 125

Ile Asp Phe Lys Glu Asp Gly Asn Ile Leu Gly His Lys Leu Glu Tyr
130 135 140

Asn Tyr Ile Ser His Asn Val Tyr Ile Thr Ala Asp Lys Gln Lys Asn
145 150 155 160

Gly Ile Lys Ala His Phe Lys Ile Arg His Asn Ile Glu Asp Gly Ser
165 170 175

Val Gln Leu Ala Asp His Tyr Gln Gln Asn Thr Pro Ile Gly Asp Gly
180 185 190

Pro Val Leu Leu Pro Asp Asn His Tyr Leu Ser Thr Gln Ser Ala Leu
195 200 205

Ser Lys Asp Pro Asn Glu Lys Arg Asp His Met Val Leu Leu Glu Phe
210 215 220

Val Thr Ala Ala Arg Met Leu Ser Glu Glu Met Ile Ala Glu Phe Lys
225 230 235 240

Ala Ala Phe Asp Met Phe Asp Ala Asp Gly Gly Gly Asp Ile Ser Thr
245 250 255

Lys Glu Leu Gly Thr Val Met Arg Met Leu Gly Gln Asn Pro Thr Lys
260 265 270

Glu Glu Leu Asp Ala Ile Ile Glu Glu Val Asp Glu Asp Gly Ser Gly
275 280 285

Thr Ile Asp Phe Glu Glu Phe Leu Val Met Met Val Arg Gln Met Lys
290 295 300

Glu Asp Ala Lys Gly Lys Ser Glu Glu Glu Leu Ala Asn Cys Phe Arg
305 310 315 320

Ile Phe Asp Lys Asn Ala Asp Gly Phe Ile Asp Ile Glu Glu Leu Gly
325 330 335

```
Glu Ile Leu Arg Ala Thr Gly Glu His Val Ile Glu Glu Asp Ile Glu
            340                 345                 350

Asp Leu Met Lys Asp Ser Asp Lys Asn Asn Asp Gly Arg Ile Asp Phe
        355                 360                 365

Asp Glu Phe Leu Lys Met Met Glu Gly Val Gln Glu Leu Met Val Ser
    370                 375                 380

Lys Gly Glu Glu Leu Phe Thr Gly Val Val Pro Ile Leu Val Glu Leu
385                 390                 395                 400

Asp Gly Asp Val Asn Gly His Lys Phe Ser Val Ser Gly Glu Gly Glu
                405                 410                 415

Gly Asp Ala Thr Tyr Gly Lys Leu Thr Leu Lys Phe Ile Cys Thr Thr
            420                 425                 430

Gly Lys Leu Pro Val Pro Trp Pro Thr Leu Val Thr Thr Phe Gly Tyr
        435                 440                 445

Gly Leu Met Cys Phe Ala Arg Tyr Pro Asp His Met Arg Gln His Asp
    450                 455                 460

Phe Phe Lys Ser Ala Met Pro Glu Gly Tyr Val Gln Glu Arg Thr Ile
465                 470                 475                 480

Phe Phe Lys Asp Asp Gly Asn Tyr Lys Thr Arg Ala Glu Val Lys Phe
                485                 490                 495

Glu Gly Asp Thr Leu Val Asn Arg Ile Glu Leu Lys Gly Ile Asp Phe
            500                 505                 510

Lys Glu Asp Gly Asn Ile Leu Gly His Lys Leu Glu Tyr Asn Tyr Asn
        515                 520                 525

Ser His Asn Val Tyr Ile Met Ala Asp Lys Gln Lys Asn Gly Ile Lys
    530                 535                 540

Ala Asn Phe Lys Ile Arg His Asn Ile Glu Asp Gly Ser Val Gln Leu
545                 550                 555                 560

Ala Asp His Tyr Gln Gln Asn Thr Pro Ile Gly Asp Gly Pro Val Leu
                565                 570                 575

Leu Pro Asp Asn His Tyr Leu Ser Tyr Gln Ser Ala Leu Ser Lys Asp
            580                 585                 590

Pro Asn Glu Lys Arg Asp His Met Val Leu Leu Glu Phe Val Thr Ala
        595                 600                 605
```

```
Ala Gly Ile Thr Leu Gly Met Asp Glu Leu Tyr Lys
    610                 615                 620
```

<210> 3
<211> 1902
<212> DNA
<213> Artificial sequence

<220>
<223> Calcium binding moiety: humcTnC

<400> 3

atggtgagca agggcgagga gctgttcacc ggggtggtgc ccatcctggt cgagctggac 60
ggcgacgtaa acggccacag gttcagcgtg tccggcgagg gcgagggcga tgccacctac 120
ggcaagctga ccctgaagtt catctgcacc accggcaagc tgcccgtgcc ctggcccacc 180
ctcgtgacca ccctgacctg gggcgtgcag tgcttcagcc gctaccccga ccacatgaag 240
cagcacgact tcttcaagtc cgccatgccc gaaggctacg tccaggagcg taccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg cccacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgcccgcatg ctaatggatg acatctacaa ggctgcggta 720
gagcagctga cagaagagca gaaaaatgag ttcaaggcag ccttcgacat cttcgtgctg 780
ggcgctgagg atggctgcat cagcaccaag gagctgggca aggtgatgag gatgctgggc 840
cagaacccca cccctgagga gctgcaggag atgatcgatg aggtggacga ggacggcagc 900
ggcacggtgg actttgatga gttcctggtc atgatggttc ggtgcatgaa ggacgacagc 960
aaagggaaat ctgaggagga gctgtctgac ctcttccgca tgtttgacaa aaatgctgat 1020
ggctacatcg acctggatga gctgaagata atgctgcagg ctacaggcga gaccatcacg 1080
gaggacgaca tcgaggaact catgaaggac ggagacaaga acaacgacgg ccgcatcgac 1140
tatgatgagt tcctggagtt catgaagggt gtggaggagc tcatggtgag caagggcgag 1200
gagctgttca ccggggtggt gcccatcctg gtcgagctgg acggcgacgt aaacggccac 1260
aagttcagcg tgtccggcga gggcgagggc gatgccacct acggcaagct gaccctgaag 1320
ttcatctgca ccaccggcaa gctgcccgtg ccctggccca ccctcgtgac caccttcggc 1380
tacggcctga tgtgcttcgc ccgctacccc gaccacatgc gccagcacga cttcttcaag 1440
tccgccatgc ccgaaggcta cgtccaggag cgcaccatct tcttcaagga cgacggcaac 1500

tacaagaccc gcgccgaggt gaagttcgag ggcgacaccc tggtgaaccg catcgagctg 1560
aagggcatcg acttcaagga ggacggcaac atcctggggc acaagctgga gtacaactac 1620
aacagccaca acgtctatat catggccgac aagcagaaga acggcatcaa ggccaacttc 1680
aagatccgcc acaacatcga ggacggcagc gtgcagctcg ccgaccacta ccagcagaac 1740
acccccatcg gcgacggccc cgtgctgctg cccgacaacc actacctgag ctaccagtcc 1800
gccctgagca aagaccccaa cgagaagcgc gatcacatgg tcctgctgga gttcgtgacc 1860
gccgccggga tcactctcgg catggacgag ctgtacaagt aa 1902

<210> 4
<211> 633
<212> PRT
<213> Artificial Sequence

<220>
<223> Calcium binding moiety: humcTnc

<400> 4

```
Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140
```

```
Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Met Asp Asp Ile Tyr Lys Ala Ala Val
225                 230                 235                 240

Glu Gln Leu Thr Glu Glu Gln Lys Asn Glu Phe Lys Ala Ala Phe Asp
                245                 250                 255

Ile Phe Val Leu Gly Ala Glu Asp Gly Cys Ile Ser Thr Lys Glu Leu
            260                 265                 270

Gly Lys Val Met Arg Met Leu Gly Gln Asn Pro Thr Pro Glu Glu Leu
        275                 280                 285

Gln Glu Met Ile Asp Glu Val Asp Glu Asp Gly Ser Gly Thr Val Asp
    290                 295                 300

Phe Asp Glu Phe Leu Val Met Met Val Arg Cys Met Lys Asp Asp Ser
305                 310                 315                 320

Lys Gly Lys Ser Glu Glu Glu Leu Ser Asp Leu Phe Arg Met Phe Asp
                325                 330                 335

Lys Asn Ala Asp Gly Tyr Ile Asp Leu Asp Glu Leu Lys Ile Met Leu
            340                 345                 350

Gln Ala Thr Gly Glu Thr Ile Thr Glu Asp Asp Ile Glu Glu Leu Met
        355                 360                 365

Lys Asp Gly Asp Lys Asn Asn Asp Gly Arg Ile Asp Tyr Asp Glu Phe
    370                 375                 380

Leu Glu Phe Met Lys Gly Val Glu Glu Leu Met Val Ser Lys Gly Glu
385                 390                 395                 400

Glu Leu Phe Thr Gly Val Val Pro Ile Leu Val Glu Leu Asp Gly Asp
                405                 410                 415
```

Val Asn Gly His Lys Phe Ser Val Ser Gly Glu Gly Glu Gly Asp Ala
420 425 430

Thr Tyr Gly Lys Leu Thr Leu Lys Phe Ile Cys Thr Thr Gly Lys Leu
435 440 445

Pro Val Pro Trp Pro Thr Leu Val Thr Thr Phe Gly Tyr Gly Leu Met
450 455 460

Cys Phe Ala Arg Tyr Pro Asp His Met Arg Gln His Asp Phe Phe Lys
465 470 475 480

Ser Ala Met Pro Glu Gly Tyr Val Gln Glu Arg Thr Ile Phe Phe Lys
485 490 495

Asp Asp Gly Asn Tyr Lys Thr Arg Ala Glu Val Lys Phe Glu Gly Asp
500 505 510

Thr Leu Val Asn Arg Ile Glu Leu Lys Gly Ile Asp Phe Lys Glu Asp
515 520 525

Gly Asn Ile Leu Gly His Lys Leu Glu Tyr Asn Tyr Asn Ser His Asn
530 535 540

Val Tyr Ile Met Ala Asp Lys Gln Lys Asn Gly Ile Lys Ala Asn Phe
545 550 555 560

Lys Ile Arg His Asn Ile Glu Asp Gly Ser Val Gln Leu Ala Asp His
565 570 575

Tyr Gln Gln Asn Thr Pro Ile Gly Asp Gly Pro Val Leu Leu Pro Asp
580 585 590

Asn His Tyr Leu Ser Tyr Gln Ser Ala Leu Ser Lys Asp Pro Asn Glu
595 600 605

Lys Arg Asp His Met Val Leu Leu Glu Phe Val Thr Ala Ala Gly Ile
610 615 620

Thr Leu Gly Met Asp Glu Leu Tyr Lys
625 630

<210> 5
<211> 1863
<212> DNA
<213> Artificial Sequence

<220>
<223> Calcium binding moiety: csTnC 15-163

<400> 5

```
atggtgagca agggcgagga gctgttcacc ggggtggtgc ccatcctggt cgagctggac      60
ggcgacgtaa acggccacag gttcagcgtg tccggcgagg gcgagggcga tgccacctac     120
ggcaagctga ccctgaagtt catctgcacc accggcaagc tgcccgtgcc ctggcccacc     180
ctcgtgacca ccctgacctg gggcgtgcag tgcttcagcc gctaccccga ccacatgaag     240
cagcacgact tcttcaagtc cgccatgccc gaaggctacg tccaggagcg taccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacat cagccacaac gtctatatca ccgccgacaa gcagaagaac     480
ggcatcaagg cccacttcaa gatccgccac aacatcgagg acggcagcgt gcagctcgcc     540
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgcccgcatg ctcagcgagg agatgattgc tgagttcaaa     720
gctgcctttg acatgtttga tgcggacggt ggtggggaca tcagcaccaa ggagttgggc     780
acggtgatga ggatgctggg ccagaacccc accaaagagg agctggatgc catcatcgag     840
gaggtggacg aggatggcag cggcaccatc gacttcgagg agttcctggt gatgatggtg     900
cgccagatga aagaggacgc caagggcaag tctgaggagg agctggccaa ctgcttccgc     960
atcttcgcca agaacgctga tgggttcatc gacatcgagg agctgggtga gattctcagg    1020
gccactgggg agcacgtcat cgaggaggac atagaagacc tcatgaagga ttcagacaag    1080
aacaatgacg gccgcattga cttcgatgag ttcctgaaga tgatggaggg tgtgcaggag    1140
ctcatggtga gcaagggcga ggagctgttc accggggtgg tgcccatcct ggtcgagctg    1200
gacggcgacg taaacggcca caagttcagc gtgtccggcg agggcgaggg cgatgccacc    1260
tacggcaagc tgaccctgaa gttcatctgc accaccggca agctgcccgt gccctggccc    1320
accctcgtga ccaccttcgg ctacggcctg atgtgcttcg cccgctaccc cgaccacatg    1380
cgccagcacg acttcttcaa gtccgccatg cccgaaggct acgtccagga gcgcaccatc    1440
ttcttcaagg acgacggcaa ctacaagacc cgcgccgagg tgaagttcga gggcgacacc    1500
ctggtgaacc gcatcgagct gaagggcatc gacttcaagg aggacggcaa catcctgggg    1560
cacaagctgg agtacaacta caacagccac aacgtctata tcatggccga caagcagaag    1620
aacggcatca aggccaactt caagatccgc cacaacatcg aggacggcag cgtgcagctc    1680
gccgaccact accagcagaa cacccccatc ggcgacggcc ccgtgctgct gcccgacaac    1740
cactacctga gctaccagtc cgccctgagc aaagacccca acgagaagcg cgatcacatg    1800
gtcctgctgg agttcgtgac cgccgccggg atcactctcg gcatggacga gctgtacaag    1860
taa                                                                  1863
```

<210> 6
<211> 620
<212> PRT
<213> Artificial Sequence

<220>
<223> calcium binding moiety: csTnC 15-163

<400> 6

```
Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205
```

```
Ser Lys Asp Pro Asn Glu Lys Arg Asp His Met Val Leu Leu Glu Phe
    210                 215                 220

Val Thr Ala Ala Arg Met Leu Ser Glu Glu Met Ile Ala Glu Phe Lys
225                 230                 235                 240

Ala Ala Phe Asp Met Phe Asp Ala Asp Gly Gly Gly Asp Ile Ser Thr
                245                 250                 255

Lys Glu Leu Gly Thr Val Met Arg Met Leu Gly Gln Asn Pro Thr Lys
            260                 265                 270

Glu Glu Leu Asp Ala Ile Ile Glu Glu Val Asp Glu Asp Gly Ser Gly
        275                 280                 285

Thr Ile Asp Phe Glu Glu Phe Leu Val Met Met Val Arg Gln Met Lys
    290                 295                 300

Glu Asp Ala Lys Gly Lys Ser Glu Glu Glu Leu Ala Asn Cys Phe Arg
305                 310                 315                 320

Ile Phe Ala Lys Asn Ala Asp Gly Phe Ile Asp Ile Glu Glu Leu Gly
                325                 330                 335

Glu Ile Leu Arg Ala Thr Gly Glu His Val Ile Glu Glu Asp Ile Glu
            340                 345                 350

Asp Leu Met Lys Asp Ser Asp Lys Asn Asn Asp Gly Arg Ile Asp Phe
        355                 360                 365

Asp Glu Phe Leu Lys Met Met Glu Gly Val Gln Glu Leu Met Val Ser
    370                 375                 380

Lys Gly Glu Glu Leu Phe Thr Gly Val Val Pro Ile Leu Val Glu Leu
385                 390                 395                 400

Asp Gly Asp Val Asn Gly His Lys Phe Ser Val Ser Gly Glu Gly Glu
                405                 410                 415

Gly Asp Ala Thr Tyr Gly Lys Leu Thr Leu Lys Phe Ile Cys Thr Thr
            420                 425                 430

Gly Lys Leu Pro Val Pro Trp Pro Thr Leu Val Thr Thr Phe Gly Tyr
        435                 440                 445

Gly Leu Met Cys Phe Ala Arg Tyr Pro Asp His Met Arg Gln His Asp
    450                 455                 460

Phe Phe Lys Ser Ala Met Pro Glu Gly Tyr Val Gln Glu Arg Thr Ile
465                 470                 475                 480
```

```
Phe Phe Lys Asp Asp Gly Asn Tyr Lys Thr Arg Ala Glu Val Lys Phe
                485                 490                 495

Glu Gly Asp Thr Leu Val Asn Arg Ile Glu Leu Lys Gly Ile Asp Phe
            500                 505                 510

Lys Glu Asp Gly Asn Ile Leu Gly His Lys Leu Glu Tyr Asn Tyr Asn
        515                 520                 525

Ser His Asn Val Tyr Ile Met Ala Asp Lys Gln Lys Asn Gly Ile Lys
    530                 535                 540

Ala Asn Phe Lys Ile Arg His Asn Ile Glu Asp Gly Ser Val Gln Leu
545                 550                 555                 560

Ala Asp His Tyr Gln Gln Asn Thr Pro Ile Gly Asp Gly Pro Val Leu
                565                 570                 575

Leu Pro Asp Asn His Tyr Leu Ser Tyr Gln Ser Ala Leu Ser Lys Asp
            580                 585                 590

Pro Asn Glu Lys Arg Asp His Met Val Leu Leu Glu Phe Val Thr Ala
        595                 600                 605

Ala Gly Ile Thr Leu Gly Met Asp Glu Leu Tyr Lys
    610                 615                 620
```

<210> 7
<211> 1908
<212> DNA
<213> Artificial sequence

<220>
<223> Calcium binding moiety: csTnC

<400> 7

```
atggtgagca agggcgagga gctgttcacc ggggtggtgc ccatcctggt cgagctggac      60
ggcgacgtaa acggccacag gttcagcgtg tccggcgagg gcgagggcga tgccacctac     120
ggcaagctga ccctgaagtt catctgcacc accggcaagc tgcccgtgcc ctggcccacc     180
ctcgtgacca ccctgacctg gggcgtgcag tgcttcagcc gctaccccga ccacatgaag     240
cagcacgact tcttcaagtc cgccatgccc gaaggctacg tccaggagcg taccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacat cagccacaac gtctatatca ccgccgacaa gcagaagaac     480
ggcatcaagg cccacttcaa gatccgccac aacatcgagg acggcagcgt gcagctcgcc     540
```

```
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgcccgcatg ctaatggcgt caatgacgga ccagcaggcg     720
gaggcccgcg ccttcctcag cgaggagatg attgctgagt tcaaagctgc ctttgacatg     780
tttgatgcgg acggtggtgg ggacatcagc accaaggagt tgggcacggt gatgaggatg     840
ctgggccaga accccaccaa agaggagctg gatgccatca tcgaggaggt ggacgaggat     900
ggcagcggca ccatcgactt cgaggagttc ctggtgatga tggtgcgcca gatgaaagag     960
gacgccaagg gcaagtctga ggaggagctg gccaactgct tccgcatctt cgacaagaac    1020
gctgatgggt tcatcgacat cgaggagctg ggtgagattc tcagggccac tggggagcac    1080
gtcatcgagg aggacataga agacctcatg aaggattcag acaagaacaa tgacggccgc    1140
attgacttcg atgagttcct gaagatgatg gagggtgtgc aggagctcat ggtgagcaag    1200
ggcgaggagc tgttcaccgg ggtggtgccc atcctggtcg agctggacgg cgacgtaaac    1260
ggccacaagt tcagcgtgtc cggcgagggc gagggcgatg ccacctacgg caagctgacc    1320
ctgaagttca tctgcaccac cggcaagctg cccgtgccct ggcccaccct cgtgaccacc    1380
ttcggctacg gcctgatgtg cttcgcccgc taccccgacc acatgcgcca gcacgacttc    1440
ttcaagtccg ccatgcccga aggctacgtc caggagcgca ccatcttctt caaggacgac    1500
ggcaactaca agacccgcgc cgaggtgaag ttcgagggcg acaccctggt gaaccgcatc    1560
gagctgaagg gcatcgactt caaggaggac ggcaacatcc tggggcacaa gctggagtac    1620
aactacaaca gccacaacgt ctatatcatg gccgacaagc agaagaacgg catcaaggcc    1680
aacttcaaga tccgccacaa catcgaggac ggcagcgtgc agctcgccga ccactaccag    1740
cagaacaccc ccatcggcga cggccccgtg ctgctgcccg acaaccacta cctgagctac    1800
cagtccgccc tgagcaaaga ccccaacgag aagcgcgatc acatggtcct gctggagttc    1860
gtgaccgccg ccgggatcac tctcggcatg gacgagctgt acaagtaa                 1908
```

<210> 8
<211> 635
<212> PRT
<213> Artificial sequence

<220>
<223> calcium binding moiety: csTnC

<400> 8

```
Met Val Ser Lys Gly Glu Glu Leu Phe Thr Gly Val Val Pro Ile Leu
1               5                   10                  15
```

```
Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Met Ala Ser Met Thr Asp Gln Gln Ala
225                 230                 235                 240

Glu Ala Arg Ala Phe Leu Ser Glu Glu Met Ile Ala Glu Phe Lys Ala
                245                 250                 255

Ala Phe Asp Met Phe Asp Ala Asp Gly Gly Gly Asp Ile Ser Thr Lys
            260                 265                 270

Glu Leu Gly Thr Val Met Arg Met Leu Gly Gln Asn Pro Thr Lys Glu
        275                 280                 285
```

```
Glu Leu Asp Ala Ile Ile Glu Glu Val Asp Glu Asp Gly Ser Gly Thr
    290                 295                 300

Ile Asp Phe Glu Glu Phe Leu Val Met Met Val Arg Gln Met Lys Glu
305                 310                 315                 320

Asp Ala Lys Gly Lys Ser Glu Glu Glu Leu Ala Asn Cys Phe Arg Ile
                325                 330                 335

Phe Asp Lys Asn Ala Asp Gly Phe Ile Asp Ile Glu Glu Leu Gly Glu
            340                 345                 350

Ile Leu Arg Ala Thr Gly Glu His Val Ile Glu Glu Asp Ile Glu Asp
        355                 360                 365

Leu Met Lys Asp Ser Asp Lys Asn Asn Asp Gly Arg Ile Asp Phe Asp
    370                 375                 380

Glu Phe Leu Lys Met Met Glu Gly Val Gln Glu Leu Met Val Ser Lys
385                 390                 395                 400

Gly Glu Glu Leu Phe Thr Gly Val Val Pro Ile Leu Val Glu Leu Asp
                405                 410                 415

Gly Asp Val Asn Gly His Lys Phe Ser Val Ser Gly Glu Gly Glu Gly
            420                 425                 430

Asp Ala Thr Tyr Gly Lys Leu Thr Leu Lys Phe Ile Cys Thr Thr Gly
        435                 440                 445

Lys Leu Pro Val Pro Trp Pro Thr Leu Val Thr Thr Phe Gly Tyr Gly
    450                 455                 460

Leu Met Cys Phe Ala Arg Tyr Pro Asp His Met Arg Gln His Asp Phe
465                 470                 475                 480

Phe Lys Ser Ala Met Pro Glu Gly Tyr Val Gln Glu Arg Thr Ile Phe
                485                 490                 495

Phe Lys Asp Asp Gly Asn Tyr Lys Thr Arg Ala Glu Val Lys Phe Glu
            500                 505                 510

Gly Asp Thr Leu Val Asn Arg Ile Glu Leu Lys Gly Ile Asp Phe Lys
        515                 520                 525

Glu Asp Gly Asn Ile Leu Gly His Lys Leu Glu Tyr Asn Tyr Asn Ser
    530                 535                 540

His Asn Val Tyr Ile Met Ala Asp Lys Gln Lys Asn Gly Ile Lys Ala
545                 550                 555                 560
```

```
Asn Phe Lys Ile Arg His Asn Ile Glu Asp Gly Ser Val Gln Leu Ala
                565                 570                 575

Asp His Tyr Gln Gln Asn Thr Pro Ile Gly Asp Gly Pro Val Leu Leu
            580                 585                 590

Pro Asp Asn His Tyr Leu Ser Tyr Gln Ser Ala Leu Ser Lys Asp Pro
        595                 600                 605

Asn Glu Lys Arg Asp His Met Val Leu Leu Glu Phe Val Thr Ala Ala
    610                 615                 620

Gly Ile Thr Leu Gly Met Asp Glu Leu Tyr Lys
625                 630                 635
```

<210> 9
<211> 1542
<212> DNA
<213> Artificial sequence

<220>
<223> Calcium binding moiety: csTnC EF-hand 2, 51-91

<400> 9

```
atggtgagca agggcgagga gctgttcacc ggggtggtgc ccatcctggt cgagctggac      60
ggcgacgtaa acggccacag gttcagcgtg tccggcgagg gcgagggcga tgccacctac     120
ggcaagctga ccctgaagtt catctgcacc accggcaagc tgcccgtgcc ctggcccacc     180
ctcgtgacca ccctgacctg gggcgtgcag tgcttcagcc gctaccccga ccacatgaag     240
cagcacgact tcttcaagtc cgccatgccc gaaggctacg tccaggagcg taccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacat cagccacaac gtctatatca ccgccgacaa gcagaagaac     480
ggcatcaagg cccacttcaa gatccgccac aacatcgagg acggcagcgt gcagctcgcc     540
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgcccgcatg ctaggccaga accccaccaa agaggagctg     720
gatgccatca tcgaggaggt ggacgaggat ggcagcggca ccatcgactt cgaggagttc     780
ctggtgatga tggtgcgcca gatgaaagag gacgccgagc tcatggtgag caagggcgag     840
gagctgttca ccggggtggt gcccatcctg gtcgagctgg acggcgacgt aaacggccac     900
aagttcagcg tgtccggcga gggcgagggc gatgccacct acggcaagct gaccctgaag     960
ttcatctgca ccaccggcaa gctgcccgtg ccctggccca ccctcgtgac caccttcggc    1020
```

tacggcctga tgtgcttcgc ccgctacccc gaccacatgc gccagcacga cttcttcaag 1080
tccgccatgc ccgaaggcta cgtccaggag cgcaccatct tcttcaagga cgacggcaac 1140
tacaagaccc gcgccgaggt gaagttcgag ggcgacaccc tggtgaaccg catcgagctg 1200
aagggcatcg acttcaagga ggacggcaac atcctggggc acaagctgga gtacaactac 1260
aacagccaca acgtctatat catggccgac aagcagaaga acggcatcaa ggccaacttc 1320
aagatccgcc acaacatcga ggacggcagc gtgcagctcg ccgaccacta ccagcagaac 1380
acccccatcg gcgacggccc cgtgctgctg cccgacaacc actacctgag ctaccagtcc 1440
gccctgagca aagaccccaa cgagaagcgc gatcacatgg tcctgctgga gttcgtgacc 1500
gccgccggga tcactctcgg catggacgag ctgtacaagt aa 1542

<210> 10
<211> 513
<212> PRT
<213> Artificial Sequence

<220>
<223> Calcium binding moiety: csTnc EF-hand 2, 51-91

<400> 10

Met Val Ser Lys Gly Glu Glu Leu Phe Thr Gly Val Val Pro Ile Leu
1 5 10 15

Val Glu Leu Asp Gly Asp Val Asn Gly His Arg Phe Ser Val Ser Gly
20 25 30

Glu Gly Glu Gly Asp Ala Thr Tyr Gly Lys Leu Thr Leu Lys Phe Ile
35 40 45

Cys Thr Thr Gly Lys Leu Pro Val Pro Trp Pro Thr Leu Val Thr Thr
50 55 60

Leu Thr Trp Gly Val Gln Cys Phe Ser Arg Tyr Pro Asp His Met Lys
65 70 75 80

Gln His Asp Phe Phe Lys Ser Ala Met Pro Glu Gly Tyr Val Gln Glu
85 90 95

Arg Thr Ile Phe Phe Lys Asp Asp Gly Asn Tyr Lys Thr Arg Ala Glu
100 105 110

Val Lys Phe Glu Gly Asp Thr Leu Val Asn Arg Ile Glu Leu Lys Gly
115 120 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Gly Gln Asn Pro Thr Lys Glu Glu Leu
225                 230                 235                 240

Asp Ala Ile Ile Glu Glu Val Asp Glu Asp Gly Ser Gly Thr Ile Asp
                245                 250                 255

Phe Glu Glu Phe Leu Val Met Met Val Arg Gln Met Lys Glu Asp Ala
            260                 265                 270

Glu Leu Met Val Ser Lys Gly Glu Glu Leu Phe Thr Gly Val Val Pro
        275                 280                 285

Ile Leu Val Glu Leu Asp Gly Asp Val Asn Gly His Lys Phe Ser Val
    290                 295                 300

Ser Gly Glu Gly Glu Gly Asp Ala Thr Tyr Gly Lys Leu Thr Leu Lys
305                 310                 315                 320

Phe Ile Cys Thr Thr Gly Lys Leu Pro Val Pro Trp Pro Thr Leu Val
                325                 330                 335

Thr Thr Phe Gly Tyr Gly Leu Met Cys Phe Ala Arg Tyr Pro Asp His
            340                 345                 350

Met Arg Gln His Asp Phe Phe Lys Ser Ala Met Pro Glu Gly Tyr Val
        355                 360                 365

Gln Glu Arg Thr Ile Phe Phe Lys Asp Asp Gly Asn Tyr Lys Thr Arg
    370                 375                 380

Ala Glu Val Lys Phe Glu Gly Asp Thr Leu Val Asn Arg Ile Glu Leu
385                 390                 395                 400

Lys Gly Ile Asp Phe Lys Glu Asp Gly Asn Ile Leu Gly His Lys Leu
405 410 415

Glu Tyr Asn Tyr Asn Ser His Asn Val Tyr Ile Met Ala Asp Lys Gln
420 425 430

Lys Asn Gly Ile Lys Ala Asn Phe Lys Ile Arg His Asn Ile Glu Asp
435 440 445

Gly Ser Val Gln Leu Ala Asp His Tyr Gln Gln Asn Thr Pro Ile Gly
450 455 460

Asp Gly Pro Val Leu Leu Pro Asp Asn His Tyr Leu Ser Tyr Gln Ser
465 470 475 480

Ala Leu Ser Lys Asp Pro Asn Glu Lys Arg Asp His Met Val Leu Leu
485 490 495

Glu Phe Val Thr Ala Ala Gly Ile Thr Leu Gly Met Asp Glu Leu Tyr
500 505 510

Lys

<210> 11
<211> 2469
<212> DNA
<213> Artificial sequence

<220>
<223> Calcium binding moiety: csTnC - Gly-Gly - csTnI

<400> 11

atggtgagca agggcgagga gctgttcacc ggggtggtgc ccatcctggt cgagctggac 60
ggcgacgtaa acggccacag gttcagcgtg tccggcgagg gcgagggcga tgccacctac 120
ggcaagctga ccctgaagtt catctgcacc accggcaagc tgcccgtgcc ctggcccacc 180
ctcgtgacca ccctgacctg gggcgtgcag tgcttcagcc gctaccccga ccacatgaag 240
cagcacgact tcttcaagtc cgccatgccc gaaggctacg tccaggagcg taccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg cccacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660

```
ctgctggagt tcgtgaccgc cgcccgcatg ctaatggcgt caatgacgga ccagcaggcg     720
gaggcccgcg ccttcctcag cgaggagatg attgctgagt tcaaagctgc ctttgacatg     780
tttgatgcgg acggtggtgg ggacatcagc accaaggagt tgggcacggt gatgaggatg     840
ctgggccaga accccaccaa agaggagctg gatgccatca tcgaggaggt ggacgaggat     900
ggcagcggca ccatcgactt cgaggagttc ctggtgatga tggtgcgcca gatgaaagag     960
gacgccaagg gcaagtctga ggaggagctg gccaactgct tccgcatctt cgacaagaac    1020
gctgatgggt tcatcgacat cgaggagctg ggtgagattc tcagggccac tggggagcac    1080
gtcatcgagg aggacataga agacctcatg aaggattcag acaagaacaa tgacggccgc    1140
attgacttcg atgagttcct gaagatgatg gagggtgtgc aggagctcgg cggcatgtct    1200
gatgaagaga aaaagcgtcg tgcagccacc gcccgtcgtc agcacctgaa gagtgctatg    1260
ctccagcttg ctgtcactga aatagaaaaa gaagcagctg ctaaagaagt ggaaaagcaa    1320
aactacctgg cagagcatag ccctcctctg tccctcccag ggtccatgca ggaacttcag    1380
gaactgagca aaaaacttca tgccaagata gactcagtgg atgaggaaag gtatgacaca    1440
gaggtgaagc tacagaagac taacaaggag ctggaggacc tgagccagaa gctgtttgac    1500
ctgaggggca agttcaagag gccacctctg cgccgggtgc gcatgtctgc tgatgccatg    1560
ctgcgtgccc tgctgggctc caagcacaag gtcaacatgg acctccgggc caacctgaag    1620
caagtcaaga aggaggacac ggagaaggag aaggacctcc gcgatgtggg tgactggagg    1680
aagaacattg aggagaaatc tggcatggag ggcaggaaga agatgtttga ggccggcgag    1740
tccgagctca tggtgagcaa gggcgaggag ctgttcaccg ggtggtgcc catcctggtc    1800
gagctggacg gcgacgtaaa cggccacaag ttcagcgtgt ccggcgaggg cgagggcgat    1860
gccacctacg gcaagctgac cctgaagttc atctgcacca ccggcaagct gcccgtgccc    1920
tggcccaccc tcgtgaccac cttcggctac ggcctgatgt gcttcgcccg ctaccccgac    1980
cacatgcgcc agcacgactt cttcaagtcc gccatgcccg aaggctacgt ccaggagcgc    2040
accatcttct tcaaggacga cggcaactac aagacccgcg ccgaggtgaa gttcgagggc    2100
gacaccctgg tgaaccgcat cgagctgaag ggcatcgact tcaaggagga cggcaacatc    2160
ctggggcaca agctggagta caactacaac agccacaacg tctatatcat ggccgacaag    2220
cagaagaacg gcatcaaggc caacttcaag atccgccaca acatcgagga cggcagcgtg    2280
cagctcgccg accactacca gcagaacacc cccatcggcg acggccccgt gctgctgccc    2340
gacaaccact acctgagcta ccagtccgcc ctgagcaaag accccaacga gaagcgcgat    2400
cacatggtcc tgctggagtt cgtgaccgcc gccgggatca ctctcggcat ggacgagctg    2460
tacaagtaa                                                            2469
```

<210> 12
<211> 822
<212> PRT

<213> Artificial Sequence

<220>
<223> calcium binding moiety: csTnC - Gly-Gly - csTnI

<400> 12

```
Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220
```

```
Val Thr Ala Ala Arg Met Leu Met Ala Ser Met Thr Asp Gln Gln Ala
225                 230                 235                 240

Glu Ala Arg Ala Phe Leu Ser Glu Glu Met Ile Ala Glu Phe Lys Ala
                245                 250                 255

Ala Phe Asp Met Phe Asp Ala Asp Gly Gly Gly Asp Ile Ser Thr Lys
            260                 265                 270

Glu Leu Gly Thr Val Met Arg Met Leu Gly Gln Asn Pro Thr Lys Glu
        275                 280                 285

Glu Leu Asp Ala Ile Ile Glu Glu Val Asp Glu Asp Gly Ser Gly Thr
    290                 295                 300

Ile Asp Phe Glu Glu Phe Leu Val Met Met Val Arg Gln Met Lys Glu
305                 310                 315                 320

Asp Ala Lys Gly Lys Ser Glu Glu Glu Leu Ala Asn Cys Phe Arg Ile
                325                 330                 335

Phe Asp Lys Asn Ala Asp Gly Phe Ile Asp Ile Glu Glu Leu Gly Glu
            340                 345                 350

Ile Leu Arg Ala Thr Gly Glu His Val Ile Glu Glu Asp Ile Glu Asp
        355                 360                 365

Leu Met Lys Asp Ser Asp Lys Asn Asn Asp Gly Arg Ile Asp Phe Asp
    370                 375                 380

Glu Phe Leu Lys Met Met Glu Gly Val Gln Glu Leu Gly Gly Met Ser
385                 390                 395                 400

Asp Glu Glu Lys Lys Arg Arg Ala Ala Thr Ala Arg Arg Gln His Leu
                405                 410                 415

Lys Ser Ala Met Leu Gln Leu Ala Val Thr Glu Ile Glu Lys Glu Ala
            420                 425                 430

Ala Ala Lys Glu Val Glu Lys Gln Asn Tyr Leu Ala Glu His Ser Pro
        435                 440                 445

Pro Leu Ser Leu Pro Gly Ser Met Gln Glu Leu Gln Glu Leu Ser Lys
    450                 455                 460

Lys Leu His Ala Lys Ile Asp Ser Val Asp Glu Glu Arg Tyr Asp Thr
465                 470                 475                 480

Glu Val Lys Leu Gln Lys Thr Asn Lys Glu Leu Glu Asp Leu Ser Gln
                485                 490                 495
```

```
Lys Leu Phe Asp Leu Arg Gly Lys Phe Lys Arg Pro Pro Leu Arg Arg
            500                 505                 510

Val Arg Met Ser Ala Asp Ala Met Leu Arg Ala Leu Leu Gly Ser Lys
        515                 520                 525

His Lys Val Asn Met Asp Leu Arg Ala Asn Leu Lys Gln Val Lys Lys
    530                 535                 540

Glu Asp Thr Glu Lys Glu Lys Asp Leu Arg Asp Val Gly Asp Trp Arg
545                 550                 555                 560

Lys Asn Ile Glu Glu Lys Ser Gly Met Glu Gly Arg Lys Lys Met Phe
                565                 570                 575

Glu Ala Gly Glu Ser Glu Leu Met Val Ser Lys Gly Glu Glu Leu Phe
            580                 585                 590

Thr Gly Val Val Pro Ile Leu Val Glu Leu Asp Gly Asp Val Asn Gly
        595                 600                 605

His Lys Phe Ser Val Ser Gly Glu Gly Glu Gly Asp Ala Thr Tyr Gly
    610                 615                 620

Lys Leu Thr Leu Lys Phe Ile Cys Thr Thr Gly Lys Leu Pro Val Pro
625                 630                 635                 640

Trp Pro Thr Leu Val Thr Thr Phe Gly Tyr Gly Leu Met Cys Phe Ala
                645                 650                 655

Arg Tyr Pro Asp His Met Arg Gln His Asp Phe Phe Lys Ser Ala Met
            660                 665                 670

Pro Glu Gly Tyr Val Gln Glu Arg Thr Ile Phe Phe Lys Asp Asp Gly
        675                 680                 685

Asn Tyr Lys Thr Arg Ala Glu Val Lys Phe Glu Gly Asp Thr Leu Val
    690                 695                 700

Asn Arg Ile Glu Leu Lys Gly Ile Asp Phe Lys Glu Asp Gly Asn Ile
705                 710                 715                 720

Leu Gly His Lys Leu Glu Tyr Asn Tyr Asn Ser His Asn Val Tyr Ile
                725                 730                 735

Met Ala Asp Lys Gln Lys Asn Gly Ile Lys Ala Asn Phe Lys Ile Arg
            740                 745                 750

His Asn Ile Glu Asp Gly Ser Val Gln Leu Ala Asp His Tyr Gln Gln
        755                 760                 765
```

Asn Thr Pro Ile Gly Asp Gly Pro Val Leu Leu Pro Asp Asn His Tyr
770 775 780

Leu Ser Tyr Gln Ser Ala Leu Ser Lys Asp Pro Asn Glu Lys Arg Asp
785 790 795 800

His Met Val Leu Leu Glu Phe Val Thr Ala Ala Gly Ile Thr Leu Gly
805 810 815

Met Asp Glu Leu Tyr Lys
820

<210> 13
<211> 1959
<212> DNA
<213> Artificial sequence

<220>
<223> calcium binding moiety: csTnI 116-135 - Gly-Gly - csTnC

<400> 13

atggtgagca agggcgagga gctgttcacc ggggtggtgc ccatcctggt cgagctggac 60
ggcgacgtaa acggccacag gttcagcgtg tccggcgagg gcgagggcga tgccacctac 120
ggcaagctga ccctgaagtt catctgcacc accggcaagc tgcccgtgcc ctggcccacc 180
ctcgtgacca ccctgacctg gggcgtgcag tgcttcagcc gctaccccga ccacatgaag 240
cagcacgact tcttcaagtc cgccatgccc gaaggctacg tccaggagcg taccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg cccacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgcccgcatg ctcgctgatg ccatgctgcg tgccctgctg 720
ggctccaagc acaaggtcaa cggcggcgcg tcaatgacgg accagcaggc ggaggcccgc 780
gccttcctca gcgaggagat gattgctgag ttcaaagctg cctttgacat gtttgatgcg 840
gacggtggtg gggacatcag caccaaggag ttgggcacgg tgatgaggat gctgggccag 900
aaccccacca aagaggagct ggatgccatc atcgaggagg tggacgagga tggcagcggc 960
accatcgact tcgaggagtt cctggtgatg atggtgcgcc agatgaaaga ggacgccaag 1020
ggcaagtctg aggaggagct ggccaactgc ttccgcatct tcgacaagaa cgctgatggg 1080
ttcatcgaca tcgaggagct gggtgagatt ctcagggcca ctggggagca cgtcatcgag 1140

gaggacatag aagacctcat gaaggattca gacaagaaca atgacggccg cattgacttc 1200
gatgagttcc tgaagatgat ggagggtgtg caggagctca tggtgagcaa gggcgaggag 1260
ctgttcaccg gggtggtgcc catcctggtc gagctggacg gcgacgtaaa cggccacaag 1320
ttcagcgtgt ccggcgaggg cgagggcgat gccacctacg gcaagctgac cctgaagttc 1380
atctgcacca ccggcaagct gcccgtgccc tggcccaccc tcgtgaccac cttcggctac 1440
ggcctgatgt gcttcgcccg ctaccccgac cacatgcgcc agcacgactt cttcaagtcc 1500
gccatgcccg aaggctacgt ccaggagcgc accatcttct tcaaggacga cggcaactac 1560
aagacccgcg ccgaggtgaa gttcgagggc gacaccctgg tgaaccgcat cgagctgaag 1620
ggcatcgact tcaaggagga cggcaacatc ctggggcaca agctggagta caactacaac 1680
agccacaacg tctatatcat ggccgacaag cagaagaacg gcatcaaggc caacttcaag 1740
atccgccaca acatcgagga cggcagcgtg cagctcgccg accactacca gcagaacacc 1800
cccatcggcg acggccccgt gctgctgccc gacaaccact acctgagcta ccagtccgcc 1860
ctgagcaaag accccaacga gaagcgcgat cacatggtcc tgctggagtt cgtgaccgcc 1920
gccgggatca ctctcggcat ggacgagctg tacaagtaa 1959

<210> 14
<211> 652
<212> PRT
<213> Artificial sequence

<220>
<223> calcium binding moiety: csTnI 116-135 - Gly-Gly - csTnC

<400> 14

Met Val Ser Lys Gly Glu Glu Leu Phe Thr Gly Val Val Pro Ile Leu
1 5 10 15

Val Glu Leu Asp Gly Asp Val Asn Gly His Arg Phe Ser Val Ser Gly
20 25 30

Glu Gly Glu Gly Asp Ala Thr Tyr Gly Lys Leu Thr Leu Lys Phe Ile
35 40 45

Cys Thr Thr Gly Lys Leu Pro Val Pro Trp Pro Thr Leu Val Thr Thr
50 55 60

Leu Thr Trp Gly Val Gln Cys Phe Ser Arg Tyr Pro Asp His Met Lys
65 70 75 80

Gln His Asp Phe Phe Lys Ser Ala Met Pro Glu Gly Tyr Val Gln Glu
85 90 95

```
Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Ala Asp Ala Met Leu Arg Ala Leu Leu
225                 230                 235                 240

Gly Ser Lys His Lys Val Asn Gly Gly Ala Ser Met Thr Asp Gln Gln
                245                 250                 255

Ala Glu Ala Arg Ala Phe Leu Ser Glu Glu Met Ile Ala Glu Phe Lys
            260                 265                 270

Ala Ala Phe Asp Met Phe Asp Ala Asp Gly Gly Gly Asp Ile Ser Thr
        275                 280                 285

Lys Glu Leu Gly Thr Val Met Arg Met Leu Gly Gln Asn Pro Thr Lys
    290                 295                 300

Glu Glu Leu Asp Ala Ile Ile Glu Glu Val Asp Glu Asp Gly Ser Gly
305                 310                 315                 320

Thr Ile Asp Phe Glu Glu Phe Leu Val Met Met Val Arg Gln Met Lys
                325                 330                 335

Glu Asp Ala Lys Gly Lys Ser Glu Glu Glu Leu Ala Asn Cys Phe Arg
            340                 345                 350

Ile Phe Asp Lys Asn Ala Asp Gly Phe Ile Asp Ile Glu Glu Leu Gly
        355                 360                 365
```

```
Glu Ile Leu Arg Ala Thr Gly Glu His Val Ile Glu Glu Asp Ile Glu
    370                 375                 380

Asp Leu Met Lys Asp Ser Asp Lys Asn Asn Asp Gly Arg Ile Asp Phe
385                 390                 395                 400

Asp Glu Phe Leu Lys Met Met Glu Gly Val Gln Glu Leu Met Val Ser
                405                 410                 415

Lys Gly Glu Glu Leu Phe Thr Gly Val Val Pro Ile Leu Val Glu Leu
            420                 425                 430

Asp Gly Asp Val Asn Gly His Lys Phe Ser Val Ser Gly Glu Gly Glu
        435                 440                 445

Gly Asp Ala Thr Tyr Gly Lys Leu Thr Leu Lys Phe Ile Cys Thr Thr
    450                 455                 460

Gly Lys Leu Pro Val Pro Trp Pro Thr Leu Val Thr Thr Phe Gly Tyr
465                 470                 475                 480

Gly Leu Met Cys Phe Ala Arg Tyr Pro Asp His Met Arg Gln His Asp
                485                 490                 495

Phe Phe Lys Ser Ala Met Pro Glu Gly Tyr Val Gln Glu Arg Thr Ile
            500                 505                 510

Phe Phe Lys Asp Asp Gly Asn Tyr Lys Thr Arg Ala Glu Val Lys Phe
        515                 520                 525

Glu Gly Asp Thr Leu Val Asn Arg Ile Glu Leu Lys Gly Ile Asp Phe
    530                 535                 540

Lys Glu Asp Gly Asn Ile Leu Gly His Lys Leu Glu Tyr Asn Tyr Asn
545                 550                 555                 560

Ser His Asn Val Tyr Ile Met Ala Asp Lys Gln Lys Asn Gly Ile Lys
                565                 570                 575

Ala Asn Phe Lys Ile Arg His Asn Ile Glu Asp Gly Ser Val Gln Leu
            580                 585                 590

Ala Asp His Tyr Gln Gln Asn Thr Pro Ile Gly Asp Gly Pro Val Leu
        595                 600                 605

Leu Pro Asp Asn His Tyr Leu Ser Tyr Gln Ser Ala Leu Ser Lys Asp
    610                 615                 620

Pro Asn Glu Lys Arg Asp His Met Val Leu Leu Glu Phe Val Thr Ala
625                 630                 635                 640
```

Ala Gly Ile Thr Leu Gly Met Asp Glu Leu Tyr Lys
645 650

<210> 15
<211> 1827
<212> DNA
<213> Artificial Sequence

<220>
<223> calcium binding moiety: csTnI 95-131 - Gly-ser-Gly - csTnc 1-91

<400> 15

atggtgagca agggcgagga gctgttcacc ggggtggtgc ccatcctggt cgagctggac 60

ggcgacgtaa acggccacag gttcagcgtg tccggcgagg gcgagggcga tgccacctac 120

ggcaagctga ccctgaagtt catctgcacc accggcaagc tgcccgtgcc ctggcccacc 180

ctcgtgacca ccctgacctg gggcgtgcag tgcttcagcc gctaccccga ccacatgaag 240

cagcacgact tcttcaagtc cgccatgccc gaaggctacg tccaggagcg taccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacat cagccacaac gtctatatca ccgccgacaa gcagaagaac 480

ggcatcaagg cccacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgcccgcatg ctagacctga gccagaagct gtttgacctg 720

aggggcaagt tcaagaggcc acctctgcgc cgggtgcgca tgtctgctga tgccatgctg 780

cgtgccctgc tgggctccaa gcacaaggtc ggcagcggca gcatgctaat ggcgtcaatg 840

acggaccagc aggcggaggc ccgcgccttc ctcagcgagg agatgattgc tgagttcaaa 900

gctgcctttg acatgtttga tgcggacggt ggtggggaca tcagcaccaa ggagttgggc 960

acggtgatga ggatgctggg ccagaacccc accaaagagg agctggatgc catcatcgag 1020

gaggtggacg aggatggcag cggcaccatc gacttcgagg agttcctggt gatgatggtg 1080

cgccagatga aagaggacgc cgagctcatg gtgagcaagg gcgaggagct gttcaccggg 1140

gtggtgccca tcctggtcga gctggacggc gacgtaaacg gccacaagtt cagcgtgtcc 1200

ggcgagggcg agggcgatgc cacctacggc aagctgaccc tgaagttcat ctgcaccacc 1260

ggcaagctgc ccgtgccctg gcccaccctc gtgaccacct tcggctacgg cctgatgtgc 1320

ttcgcccgct accccgacca catgcgccag cacgacttct tcaagtccgc catgcccgaa 1380

ggctacgtcc aggagcgcac catcttcttc aaggacgacg gcaactacaa gacccgcgcc 1440

gaggtgaagt tcgagggcga caccctggtg aaccgcatcg agctgaaggg catcgacttc 1500

aaggaggacg gcaacatcct ggggcacaag ctggagtaca actacaacag ccacaacgtc 1560

tatatcatgg ccgacaagca gaagaacggc atcaaggcca acttcaagat ccgccacaac 1620

atcgaggacg gcagcgtgca gctcgccgac cactaccagc agaacacccc catcggcgac 1680

ggccccgtgc tgctgcccga caaccactac ctgagctacc agtccgccct gagcaaagac 1740

cccaacgaga agcgcgatca catggtcctg ctggagttcg tgaccgccgc cgggatcact 1800

ctcggcatgg acgagctgta caagtaa 1827

<210> 16
<211> 608
<212> PRT
<213> Artificial Sequence

<220>
<223> Calcium binding moiety: csTnI 95-131 - Gly-Ser-Gly - csTnC 1-91

<400> 16

Met Val Ser Lys Gly Glu Glu Leu Phe Thr Gly Val Val Pro Ile Leu
1 5 10 15

Val Glu Leu Asp Gly Asp Val Asn Gly His Arg Phe Ser Val Ser Gly
20 25 30

Glu Gly Glu Gly Asp Ala Thr Tyr Gly Lys Leu Thr Leu Lys Phe Ile
35 40 45

Cys Thr Thr Gly Lys Leu Pro Val Pro Trp Pro Thr Leu Val Thr Thr
50 55 60

Leu Thr Trp Gly Val Gln Cys Phe Ser Arg Tyr Pro Asp His Met Lys
65 70 75 80

Gln His Asp Phe Phe Lys Ser Ala Met Pro Glu Gly Tyr Val Gln Glu
85 90 95

Arg Thr Ile Phe Phe Lys Asp Asp Gly Asn Tyr Lys Thr Arg Ala Glu
100 105 110

Val Lys Phe Glu Gly Asp Thr Leu Val Asn Arg Ile Glu Leu Lys Gly
115 120 125

Ile Asp Phe Lys Glu Asp Gly Asn Ile Leu Gly His Lys Leu Glu Tyr
130 135 140

```
Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Asp Leu Ser Gln Lys Leu Phe Asp Leu
225                 230                 235                 240

Arg Gly Lys Phe Lys Arg Pro Pro Leu Arg Arg Val Arg Met Ser Ala
                245                 250                 255

Asp Ala Met Leu Arg Ala Leu Leu Gly Ser Lys His Lys Val Gly Ser
            260                 265                 270

Gly Ser Met Leu Met Ala Ser Met Thr Asp Gln Gln Ala Glu Ala Arg
        275                 280                 285

Ala Phe Leu Ser Glu Glu Met Ile Ala Glu Phe Lys Ala Ala Phe Asp
    290                 295                 300

Met Phe Asp Ala Asp Gly Gly Gly Asp Ile Ser Thr Lys Glu Leu Gly
305                 310                 315                 320

Thr Val Met Arg Met Leu Gly Gln Asn Pro Thr Lys Glu Glu Leu Asp
                325                 330                 335

Ala Ile Ile Glu Glu Val Asp Glu Asp Gly Ser Gly Thr Ile Asp Phe
            340                 345                 350

Glu Glu Phe Leu Val Met Met Val Arg Gln Met Lys Glu Asp Ala Glu
        355                 360                 365

Leu Met Val Ser Lys Gly Glu Glu Leu Phe Thr Gly Val Val Pro Ile
    370                 375                 380

Leu Val Glu Leu Asp Gly Asp Val Asn Gly His Lys Phe Ser Val Ser
385                 390                 395                 400

Gly Glu Gly Glu Gly Asp Ala Thr Tyr Gly Lys Leu Thr Leu Lys Phe
                405                 410                 415
```

```
Ile Cys Thr Thr Gly Lys Leu Pro Val Pro Trp Pro Thr Leu Val Thr
            420                 425                 430

Thr Phe Gly Tyr Gly Leu Met Cys Phe Ala Arg Tyr Pro Asp His Met
        435                 440                 445

Arg Gln His Asp Phe Phe Lys Ser Ala Met Pro Glu Gly Tyr Val Gln
    450                 455                 460

Glu Arg Thr Ile Phe Phe Lys Asp Asp Gly Asn Tyr Lys Thr Arg Ala
465                 470                 475                 480

Glu Val Lys Phe Glu Gly Asp Thr Leu Val Asn Arg Ile Glu Leu Lys
                485                 490                 495

Gly Ile Asp Phe Lys Glu Asp Gly Asn Ile Leu Gly His Lys Leu Glu
            500                 505                 510

Tyr Asn Tyr Asn Ser His Asn Val Tyr Ile Met Ala Asp Lys Gln Lys
        515                 520                 525

Asn Gly Ile Lys Ala Asn Phe Lys Ile Arg His Asn Ile Glu Asp Gly
    530                 535                 540

Ser Val Gln Leu Ala Asp His Tyr Gln Gln Asn Thr Pro Ile Gly Asp
545                 550                 555                 560

Gly Pro Val Leu Leu Pro Asp Asn His Tyr Leu Ser Tyr Gln Ser Ala
                565                 570                 575

Leu Ser Lys Asp Pro Asn Glu Lys Arg Asp His Met Val Leu Leu Glu
            580                 585                 590

Phe Val Thr Ala Ala Gly Ile Thr Leu Gly Met Asp Glu Leu Tyr Lys
        595                 600                 605
```

<210> 17
<211> 1869
<212> DNA
<213> Artificial Sequence

<220>
<223> Calcium binding moiety: csTnI 12-161

<400> 17

atggtgagca agggcgagga gctgttcacc ggggtggtgc ccatcctggt cgagctggac 60
ggcgacgtaa acggccacag gttcagcgtg tccggcgagg gcgagggcga tgccacctac 120
ggcaagctga ccctgaagtt catctgcacc accggcaagc tgcccgtgcc ctggcccacc 180

ctcgtgacca ccctgacctg ggcgtgcag tgcttcagcc gctaccccga ccacatgaag 240
cagcacgact tcttcaagtc cgccatgccc gaaggctacg tccaggagcg taccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacat cagccacaac gtctatatca ccgccgacaa gcagaagaac 480
ggcatcaagg cccacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgcccgcatg ctgctgacag aagagcagaa aaatgagttc 720
aaggcagcct tcgacatctt cgtgctgggc gctgaggatg gctgcatcag caccaaggag 780
ctgggcaagg tgatgaggat gctgggccag aaccccaccc ctgaggagct gcaggagatg 840
atcgatgagg tggacgagga cggcagcggc acggtggact ttgatgagtt cctggtcatg 900
atggttcggt gcatgaagga cgacagcaaa gggaaatctg aggaggagct gtctgacctc 960
ttccgcatgt ttgacaaaaa tgctgatggc tacatcgacc tggatgagct gaagataatg 1020
ctgcaggcta caggcgagac catcacggag gacgacatcg aggaactcat gaaggacgga 1080
gacaagaaca acgacggccg catcgactat gatgagttcc tggagttcat gaagggtgtg 1140
gaggagctca tggtgagcaa gggcgaggag ctgttcaccg gggtggtgcc catcctggtc 1200
gagctggacg gcgacgtaaa cggccacaag ttcagcgtgt ccggcgaggg cgagggcgat 1260
gccacctacg gcaagctgac cctgaagttc atctgcacca ccggcaagct gcccgtgccc 1320
tggcccaccc tcgtgaccac cttcggctac ggcctgatgt gcttcgcccg ctaccccgac 1380
cacatgcgcc agcacgactt cttcaagtcc gccatgcccg aaggctacgt ccaggagcgc 1440
accatcttct tcaaggacga cggcaactac aagacccgcg ccgaggtgaa gttcgagggc 1500
gacaccctgg tgaaccgcat cgagctgaag ggcatcgact tcaaggagga cggcaacatc 1560
ctggggcaca agctggagta caactacaac agccacaacg tctatatcat ggccgacaag 1620
cagaagaacg gcatcaaggc caacttcaag atccgccaca acatcgagga cggcagcgtg 1680
cagctcgccg accactacca gcagaacacc cccatcggcg acggccccgt gctgctgccc 1740
gacaaccact acctgagcta ccagtccgcc ctgagcaaag accccaacga gaagcgcgat 1800
cacatggtcc tgctggagtt cgtgaccgcc gccgggatca ctctcggcat ggacgagctg 1860
tacaagtaa 1869

<210> 18
<211> 622
<212> PRT
<213> Artificial Sequence

<220>

<223> calcium binding moiety: csTnI 12-161

<400> 18

```
Met Val Ser Lys Gly Glu Glu Leu Phe Thr Gly Val Val Pro Ile Leu
1               5                   10                  15

Val Glu Leu Asp Gly Asp Val Asn Gly His Arg Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Trp Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Ile Ser His Asn Val Tyr Ile Thr Ala Asp Lys Gln Lys Asn
145                 150                 155                 160

Gly Ile Lys Ala His Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Arg Met Leu Leu Thr Glu Glu Gln Lys Asn Glu Phe
225                 230                 235                 240
```

Lys Ala Ala Phe Asp Ile Phe Val Leu Gly Ala Glu Asp Gly Cys Ile
245 250 255

Ser Thr Lys Glu Leu Gly Lys Val Met Arg Met Leu Gly Gln Asn Pro
260 265 270

Thr Pro Glu Glu Leu Gln Glu Met Ile Asp Glu Val Asp Glu Asp Gly
275 280 285

Ser Gly Thr Val Asp Phe Asp Glu Phe Leu Val Met Met Val Arg Cys
290 295 300

Met Lys Asp Asp Ser Lys Gly Lys Ser Glu Glu Glu Leu Ser Asp Leu
305 310 315 320

Phe Arg Met Phe Asp Lys Asn Ala Asp Gly Tyr Ile Asp Leu Asp Glu
325 330 335

Leu Lys Ile Met Leu Gln Ala Thr Gly Glu Thr Ile Thr Glu Asp Asp
340 345 350

Ile Glu Glu Leu Met Lys Asp Gly Asp Lys Asn Asn Asp Gly Arg Ile
355 360 365

Asp Tyr Asp Glu Phe Leu Glu Phe Met Lys Gly Val Glu Glu Leu Met
370 375 380

Val Ser Lys Gly Glu Glu Leu Phe Thr Gly Val Val Pro Ile Leu Val
385 390 395 400

Glu Leu Asp Gly Asp Val Asn Gly His Lys Phe Ser Val Ser Gly Glu
405 410 415

Gly Glu Gly Asp Ala Thr Tyr Gly Lys Leu Thr Leu Lys Phe Ile Cys
420 425 430

Thr Thr Gly Lys Leu Pro Val Pro Trp Pro Thr Leu Val Thr Thr Phe
435 440 445

Gly Tyr Gly Leu Met Cys Phe Ala Arg Tyr Pro Asp His Met Arg Gln
450 455 460

His Asp Phe Phe Lys Ser Ala Met Pro Glu Gly Tyr Val Gln Glu Arg
465 470 475 480

Thr Ile Phe Phe Lys Asp Asp Gly Asn Tyr Lys Thr Arg Ala Glu Val
485 490 495

Lys Phe Glu Gly Asp Thr Leu Val Asn Arg Ile Glu Leu Lys Gly Ile
500 505 510

Asp Phe Lys Glu Asp Gly Asn Ile Leu Gly His Lys Leu Glu Tyr Asn
515 520 525

Tyr Asn Ser His Asn Val Tyr Ile Met Ala Asp Lys Gln Lys Asn Gly
530 535 540

Ile Lys Ala Asn Phe Lys Ile Arg His Asn Ile Glu Asp Gly Ser Val
545 550 555 560

Gln Leu Ala Asp His Tyr Gln Gln Asn Thr Pro Ile Gly Asp Gly Pro
565 570 575

Val Leu Leu Pro Asp Asn His Tyr Leu Ser Tyr Gln Ser Ala Leu Ser
580 585 590

Lys Asp Pro Asn Glu Lys Arg Asp His Met Val Leu Leu Glu Phe Val
595 600 605

Thr Ala Ala Gly Ile Thr Leu Gly Met Asp Glu Leu Tyr Lys
610 615 620

<210> 19
<211> 486
<212> DNA
<213> Homo sapiens

<400> 19

atggatgaca tctacaaggc tgcggtagag cagctgacag aagagcagaa aaatgagttc 60
aaggcagcct tcgacatctt cgtgctgggc gctgaggatg gctgcatcag caccaaggag 120
ctgggcaagg tgatgaggat gctgggccag aaccccaccc ctgaggagct gcaggagatg 180
atcgatgagg tggacgagga cggcagcggc acggtggact ttgatgagtt cctggtcatg 240
atggttcggt gcatgaagga cgacagcaaa gggaaatctg aggaggagct gtctgacctc 300
ttccgcatgt ttgacaaaaa tgctgatggc tacatcgacc tggatgagct gaagataatg 360
ctgcaggcta caggcgagac catcacggag gacgacatcg aggagctcat gaaggacgga 420
gacaagaaca acgacggccg catcgactat gatgagttcc tggagttcat gaagggtgtg 480
gagtag 486

<210> 20
<211> 161
<212> PRT
<213> Homo sapiens

<400> 20

```
Met Asp Asp Ile Tyr Lys Ala Ala Val Glu Gln Leu Thr Glu Glu Gln
1               5                   10                  15

Lys Asn Glu Phe Lys Ala Ala Phe Asp Ile Phe Val Leu Gly Ala Glu
            20                  25                  30

Asp Gly Cys Ile Ser Thr Lys Glu Leu Gly Lys Val Met Arg Met Leu
        35                  40                  45

Gly Gln Asn Pro Thr Pro Glu Glu Leu Gln Glu Met Ile Asp Glu Val
    50                  55                  60

Asp Glu Asp Gly Ser Gly Thr Val Asp Phe Asp Glu Phe Leu Val Met
65                  70                  75                  80

Met Val Arg Cys Met Lys Asp Asp Ser Lys Gly Lys Ser Glu Glu Glu
                85                  90                  95

Leu Ser Asp Leu Phe Arg Met Phe Asp Lys Asn Ala Asp Gly Tyr Ile
            100                 105                 110

Asp Leu Asp Glu Leu Lys Ile Met Leu Gln Ala Thr Gly Glu Thr Ile
        115                 120                 125

Thr Glu Asp Asp Ile Glu Glu Leu Met Lys Asp Gly Asp Lys Asn Asn
    130                 135                 140

Asp Gly Arg Ile Asp Tyr Asp Glu Phe Leu Glu Phe Met Lys Gly Val
145                 150                 155                 160

Glu
```

<210> 21
<211> 633
<212> DNA
<213> Homo sapiens

<400> 21

```
atggcggatg ggagcagcga tgcggctagg gaacctcgcc ctgcaccagc cccaatcaga      60
cgccgctcct ccaactaccg cgcttatgcc acggagccgc acgccaagaa aaaatctaag     120
atctccgcct cgagaaaatt gcagctgaag actctgctgc tgcagattgc aaagcaagag     180
ctggagcgag aggcggagga gcggcgcgga gagaaggggc gcgctctgag cacccgctgc     240
cagccgctgg agttgaccgg gctgggcttc gcggagctgc aggacttgtg ccgacagctc     300
cacgcccgtg tggacaaggt ggatgaagag agatacgaca tagaggcaaa agtcaccaag     360
```

aacatcacgg agattgcaga tctgactcag aagatctttg accttcgagg caagtttaag 420
cggcccaccc tgcggagagt gaggatctct gcagatgcca tgatgcaggc gctgctgggg 480
gcccgggcta aggagtccct ggacctgcgg gcccacctca agcaggtgaa gaaggaggac 540
accgagaagg aaaaccggga ggtgggagac tggcggaaga acatcgatgc actgagtgga 600
atggagggcc gcaagaaaaa gtttgagagc tga 633

<210> 22
<211> 210
<212> PRT
<213> Homo sapiens

<400> 22

Met Ala Asp Gly Ser Ser Asp Ala Ala Arg Glu Pro Arg Pro Ala Pro
1 5 10 15

Ala Pro Ile Arg Arg Arg Ser Ser Asn Tyr Arg Ala Tyr Ala Thr Glu
20 25 30

Pro His Ala Lys Lys Lys Ser Lys Ile Ser Ala Ser Arg Lys Leu Gln
35 40 45

Leu Lys Thr Leu Leu Leu Gln Ile Ala Lys Gln Glu Leu Glu Arg Glu
50 55 60

Ala Glu Glu Arg Arg Gly Glu Lys Gly Arg Ala Leu Ser Thr Arg Cys
65 70 75 80

Gln Pro Leu Glu Leu Thr Gly Leu Gly Phe Ala Glu Leu Gln Asp Leu
85 90 95

Cys Arg Gln Leu His Ala Arg Val Asp Lys Val Asp Glu Glu Arg Tyr
100 105 110

Asp Ile Glu Ala Lys Val Thr Lys Asn Ile Thr Glu Ile Ala Asp Leu
115 120 125

Thr Gln Lys Ile Phe Asp Leu Arg Gly Lys Phe Lys Arg Pro Thr Leu
130 135 140

Arg Arg Val Arg Ile Ser Ala Asp Ala Met Met Gln Ala Leu Leu Gly
145 150 155 160

Ala Arg Ala Lys Glu Ser Leu Asp Leu Arg Ala His Leu Lys Gln Val
165 170 175

```
Lys Lys Glu Asp Thr Glu Lys Glu Asn Arg Glu Val Gly Asp Trp Arg
            180                 185                 190

Lys Asn Ile Asp Ala Leu Ser Gly Met Glu Gly Arg Lys Lys Lys Phe
        195                 200                 205

Glu Ser
    210
```

<210> 23
<211> 483
<212> DNA
<213> Homo sapiens

<400> 23

```
atgacggacc agcaggctga ggccaggtcc tacctcagcg aagagatgat cgctgagttc    60
aaggctgcct ttgacatgtt tgatgctgat ggtggtgggg acatcagcgt caaggagttg   120
ggcacggtga tgaggatgct gggccagaca cccaccaagg aggagctgga cgccatcatc   180
gaggaggtgg atgaggacgg cagcggcacc atcgacttcg aggagttctt ggtcatgatg   240
gtgcgccaga tgaaagagga cgcgaaaggg aagagcgagg aggagctggc cgagtgcttc   300
cgcatcttcg acaggaatgc agacggctac atcgacccgg aggagctggc tgagattttc   360
agggcctccg gggagcacgt gactgacgag gagatcgaat ctctgatgaa agacggcgac   420
aagaacaacg acggccgcat tgacttcgac gagttcctga agatgatgga gggcgtgcag   480
taa                                                                 483
```

<210> 24
<211> 160
<212> PRT
<213> Homo sapiens

<400> 24

```
Met Thr Asp Gln Gln Ala Glu Ala Arg Ser Tyr Leu Ser Glu Glu Met
1               5                   10                  15

Ile Ala Glu Phe Lys Ala Ala Phe Asp Met Phe Asp Ala Asp Gly Gly
            20                  25                  30

Gly Asp Ile Ser Val Lys Glu Leu Gly Thr Val Met Arg Met Leu Gly
        35                  40                  45

Gln Thr Pro Thr Lys Glu Glu Leu Asp Ala Ile Ile Glu Glu Val Asp
    50                  55                  60
```

```
Glu Asp Gly Ser Gly Thr Ile Asp Phe Glu Glu Phe Leu Val Met Met
65                  70                  75                  80

Val Arg Gln Met Lys Glu Asp Ala Lys Gly Lys Ser Glu Glu Glu Leu
                85                  90                  95

Ala Glu Cys Phe Arg Ile Phe Asp Arg Asn Ala Asp Gly Tyr Ile Asp
            100                 105                 110

Pro Glu Glu Leu Ala Glu Ile Phe Arg Ala Ser Gly Glu His Val Thr
        115                 120                 125

Asp Glu Glu Ile Glu Ser Leu Met Lys Asp Gly Asp Lys Asn Asn Asp
    130                 135                 140

Gly Arg Ile Asp Phe Asp Glu Phe Leu Lys Met Met Glu Gly Val Gln
145                 150                 155                 160
```

<210> 25
<211> 492
<212> DNA
<213> Gallus gallus

<400> 25

```
atggcgtcaa tgacggacca gcaggcggag gcccgcgcct tcctcagcga ggagatgatt     60
gctgagttca aagctgcctt tgacatgttt gatgcggacg gtggtgggga catcagcacc    120
aaggagttgg gcacggtgat gaggatgctg ggccagaacc ccaccaaaga ggagctggat    180
gccatcatcg aggaggtgga cgaggatggc agcggcacca tcgacttcga ggagttcctg    240
gtgatgatgg tgcgccagat gaaagaggac gccaagggca agtctgagga ggagctggcc    300
aactgcttcc gcatcttcga caagaacgct gatgggttca tcgacatcga ggagctgggt    360
gagattctca gggccactgg ggagcacgtc atcgaggagg acatagaaga cctcatgaag    420
gattcagaca agaacaatga cggccgcatt gacttcgatg agttcctgaa gatgatggag    480
ggtgtgcagt aa                                                        492
```

<210> 26
<211> 163
<212> PRT
<213> Gallus gallus

<400> 26

```
Met Ala Ser Met Thr Asp Gln Gln Ala Glu Ala Arg Ala Phe Leu Ser
1               5                   10                  15

Glu Glu Met Ile Ala Glu Phe Lys Ala Ala Phe Asp Met Phe Asp Ala
            20                  25                  30

Asp Gly Gly Gly Asp Ile Ser Thr Lys Glu Leu Gly Thr Val Met Arg
        35                  40                  45

Met Leu Gly Gln Asn Pro Thr Lys Glu Glu Leu Asp Ala Ile Ile Glu
    50                  55                  60

Glu Val Asp Glu Asp Gly Ser Gly Thr Ile Asp Phe Glu Glu Phe Leu
65                  70                  75                  80

Val Met Met Val Arg Gln Met Lys Glu Asp Ala Lys Gly Lys Ser Glu
                85                  90                  95

Glu Glu Leu Ala Asn Cys Phe Arg Ile Phe Asp Lys Asn Ala Asp Gly
            100                 105                 110

Phe Ile Asp Ile Glu Glu Leu Gly Glu Ile Leu Arg Ala Thr Gly Glu
        115                 120                 125

His Val Ile Glu Glu Asp Ile Glu Asp Leu Met Lys Asp Ser Asp Lys
    130                 135                 140

Asn Asn Asp Gly Arg Ile Asp Phe Asp Glu Phe Leu Lys Met Met Glu
145                 150                 155                 160

Gly Val Gln
```

<210> 27
<211> 552
<212> DNA
<213> Gallus gallus

<400> 27

```
atgtctgatg aagagaaaaa gaggagggca gccaccgccc ggcgccagca cctgaagagt      60
gctatgctcc agcttgctgt cactgaaata gaaaaagaag cagctgctaa agaagtggaa     120
aagcaaaact acctggcaga gcattgccct cctctgtccc tcccaggatc catgcaggaa     180
cttcaggaac tgtgcaaaaa gcttcatgcc aagatagact cagtggatga ggaaaggtat     240
gacacagagg tgaagctaca gaagactaac aaggagctgg aggacctgag ccagaagctg     300
tttgacctga ggggcaagtt caagaggcca cctctgcgcc gggtgcgcat gtctgctgat     360
gccatgctgc gtgccctgct gggctccaag cacaaggtca acatggacct ccgggccaac     420
```

```
ctgaagcaag tcaagaagga ggacacggag aaggagaagg acctccgcga tgtgggtgac    480
tggaggaaga acattgagga gaaatctggc atggagggca ggaagaagat gtttgaggcc    540
ggcgagtcct aa                                                        552
```

<210> 28
<211> 183
<212> PRT
<213> Gallus gallus

<400> 28

```
Met Ser Asp Glu Glu Lys Lys Arg Arg Ala Ala Thr Ala Arg Arg Gln
1               5                   10                  15

His Leu Lys Ser Ala Met Leu Gln Leu Ala Val Thr Glu Ile Glu Lys
            20                  25                  30

Glu Ala Ala Ala Lys Glu Val Glu Lys Gln Asn Tyr Leu Ala Glu His
        35                  40                  45

Cys Pro Pro Leu Ser Leu Pro Gly Ser Met Gln Glu Leu Gln Glu Leu
    50                  55                  60

Cys Lys Lys Leu His Ala Lys Ile Asp Ser Val Asp Glu Glu Arg Tyr
65                  70                  75                  80

Asp Thr Glu Val Lys Leu Gln Lys Thr Asn Lys Glu Leu Glu Asp Leu
                85                  90                  95

Ser Gln Lys Leu Phe Asp Leu Arg Gly Lys Phe Lys Arg Pro Pro Leu
            100                 105                 110

Arg Arg Val Arg Met Ser Ala Asp Ala Met Leu Arg Ala Leu Leu Gly
        115                 120                 125

Ser Lys His Lys Val Asn Met Asp Leu Arg Ala Asn Leu Lys Gln Val
    130                 135                 140

Lys Lys Glu Asp Thr Glu Lys Glu Lys Asp Leu Arg Asp Val Gly Asp
145                 150                 155                 160

Trp Arg Lys Asn Ile Glu Glu Lys Ser Gly Met Glu Gly Arg Lys Lys
                165                 170                 175

Met Phe Glu Ala Gly Glu Ser
            180
```

<210> 29

<211> 486
<212> DNA
<213> Gallus gallus

<400> 29

```
atggatgaca tctataaggc ggcggttgag cagttgacag aagaacaaaa aaatgagttt      60
aaggctgcct tcgacatctt cgtgctgggg gcagaggatg gctgcatcag caccaaggag     120
ctggggaagg tgatgaggat gctggggcag aaccccaccc ctgaggagct gcaggagatg     180
attgatgagg tggatgagga tggcagtggc actgtggact ttgatgagtt ccttgttatg     240
atggttcggt gtatgaaaga tgacagcaaa ggaaaaactg aagaggagct ctcagatctc     300
ttcaggatgt ttgataagaa tgctgatggc tacatcgatc ttgaggaact gaagatcatg     360
ctacaggcaa ctggagagac gatcactgag gatgacatag aagaactgat gaaagatggg     420
gacaaaaaca atgatggcag gattgactat gacgagttcc tggagttcat gaagggagtt     480
gaataa                                                                486
```

<210> 30
<211> 161
<212> PRT
<213> Gallus gallus

<400> 30

```
Met Asp Asp Ile Tyr Lys Ala Ala Val Glu Gln Leu Thr Glu Glu Gln
1               5                   10                  15

Lys Asn Glu Phe Lys Ala Ala Phe Asp Ile Phe Val Leu Gly Ala Glu
            20                  25                  30

Asp Gly Cys Ile Ser Thr Lys Glu Leu Gly Lys Val Met Arg Met Leu
        35                  40                  45

Gly Gln Asn Pro Thr Pro Glu Glu Leu Gln Glu Met Ile Asp Glu Val
    50                  55                  60

Asp Glu Asp Gly Ser Gly Thr Val Asp Phe Asp Glu Phe Leu Val Met
65                  70                  75                  80

Met Val Arg Cys Met Lys Asp Asp Ser Lys Gly Lys Thr Glu Glu Glu
                85                  90                  95

Leu Ser Asp Leu Phe Arg Met Phe Asp Lys Asn Ala Asp Gly Tyr Ile
            100                 105                 110
```

```
Asp Leu Glu Glu Leu Lys Ile Met Leu Gln Ala Thr Gly Glu Thr Ile
        115                 120                 125

Thr Glu Asp Asp Ile Glu Glu Leu Met Lys Asp Gly Asp Lys Asn Asn
    130                 135                 140

Asp Gly Arg Ile Asp Tyr Asp Glu Phe Leu Glu Phe Met Lys Gly Val
145                 150                 155                 160

Glu
```

## Description

### BACKGROUND OF THE INVENTION

The use of genetically encoded fluorescent indicators for visualizing cellular calcium levels promises many advantages over fluorescent Ca-indicating dyes that have to be applied externally. Genetically encoded indicators are generated *in situ* inside cells after transfection, do not require cofactors, can in theory be specifically targeted to cell organelles and cellular microenvironments and do not leak out of cells during longer recording sessions. Furthermore, they should be expressible within intact tissues of transgenic organisms and thus should solve the problem of loading an indicator dye into tissue, while allowing to label specific subsets of cells of interest (for review see Zhang J., et al. "Creating new fluorescent probes for cell biology." Nat. Rev. Mol. Biol. 3, 906-918 (2002)).

Two classes of GFP-based calcium indicators have been described so far: first, ratiometric indicators termed "Cameleons" consisting of a pair of fluorescent proteins engineered for fluorescence resonance energy transfer (FRET) carrying the calcium binding protein calmodulin as well as a calmodulin target peptide sandwiched between the GFPs (see for example Miyawaki, A. et al. "Fluorescent indicators for Ca2+ based on green fluorescent proteins and calmodulin." Nature 388, 882-887 (1997); Miyawaki, A. et al. "Dynamic and quantitative calcium measurements using improved cameleons." Proc. Natl. Acad. Sci. USA 96, 2135-2140 (1999) and Truong et al. "FRET-based in vivo Ca2+ imaging by a new calmodulin-GFP fusion molecule." Nat. Struct. Biol. 8, 1069-1073 (2001)). Second, various non-ratiometric indicators with calmodulin directly inserted into a single fluorescent protein (see Baird, G.S. et al. "Circular permutation and receptor insertion within green fluorescent proteins." Proc. Natl. Acad. USA 96, 11241-11246 (1999); Nagai, T. et al. "Circularly permuted green fluorescent proteins engineered to sense Ca2+." Proc. Natl. Acad Sci. USA 98, 3197-3202 (2001); Nakai, J. et al. "A high signal-to-noise Ca2+ probe composed of a single green fluorescent protein." Nat. Biotechnol. 19, 137-141 (2001); and Griesbeck, O. et al. "Reducing the environmental sensitivity of yellow fluorescent protein: mechanism and applications." J. Biol. Chem. 276, 29188-29194 (2001)).

However, calmodulin-based indicators show deficiencies in certain applications, e.g. they display only a reduced dynamic range in transgenic invertebrates compared to *in vitro* data of the purified indicator proteins and acute transfections (see Reiff, D.F. et al. "Differential regulation of active zone density during long-term strengthening of Drosophila neuromuscular junctions." J. Neurosci. 22, 9399-9409; Kerr R. et al. "Optical imaging of calcium transients in neurons and pharyngeal muscle of C. elegans." Neuron 26, 583-594; and Fiala et al. "Genetically expressed cameleon in Drosophila melanogaster is used to visualize olfactory information in projection neurons." Curr. Biol. 12, 1877-1884 (2002) ). Further, they fail to show calcium responses when targeted to certain sites within cells. No useful transgenic expression in mammals has been reported yet. Calmodulin is an ubiquitous signal protein in cell metabolism and thus under stringent regulation involving a plethora of calmodulin-binding proteins (for review see Jurado, L.A. et al. "Apocalmodulin." Physiol. Rev. 79, 661-682 (1999)). It activates numerous kinases and phosphatases, modulates ion channels (Saimi, Y. & Kung, C. "Calmodulin as an ion channel subunit." Ann. Rev. Physiol. 64, 289-311 (2002) and is itself extensively phosphorylated by multiple protein serine/threonine kinases and protein tyrosine kinases (Benaim, G. & Villalobo, A. "Phosphorylation of calmodulin." Eur. J Biochem. 269, 3619-3725 (2002).

The present inventors therefore explored ways of constructing new types of calcium probes with more specialized calcium binding proteins that are minimally influenced by the cellular regulatory protein network.

### SUMMARY OF THE INVENTION

Troponin C (TnC or TNC) is a dumbbell-shaped calcium binding protein with two globular domains connected by a central linker. It was found that novel types of calcium probes that are based on Troponin C are superior for dynamic imaging within live cells than prior art genetic calcium sensors. In particular, the calcium sensors based on Troponin C function in subcellular environments in which prior art calcium sensors have demonstrated only poor behaviour, for example when tethered to a cellular membrane. Moreover, the novel Troponin-C-based calcium sensors can be used in a multitude of cell types and even in transgenic animals, which is a further advantage compared with prior art Calcium sensors. Moreover, the Troponin-C-based calcium sensors of the invention do not interfere with intracellular Ca-signalling, in particular, they do not interfere with the important calmodulin pathway. The Troponin-C-based calcium sensors do not show any sign of unfavourable aggregation and have the further advantage that they do not interact in an unfavourable way with cytosolic components.

This invention therefore relates to modified polypeptides comprising three functional components: a first chromophor of a donor-acceptor-pair for FRET, a calcium-binding polypeptide with an identity of at least 80% to a 30 amino acid long polypeptide sequence of human Troponin C or chicken skeletal muscle Troponin C, and a second chromophor of a donor-acceptor-pair for FRET. Such modified calcium-binding polypeptides function as superior intracellular calcium sensors because upon calcium binding the calcium-binding polypeptide changes its conformation leading to a spatial redistribution of the two chromophores of the polypeptide of the invention. This spatial redistribution can then be detected by a change of the fluorescence properties of the overall polypeptide. Another aspect of the invention relates to nucleic acid molecules comprising a nucleotide sequence encoding a fusion polypeptide, where both the first chromophore and the second chromophore of the donor-acceptor-pair for FRET of the modified calcium-binding polypeptide of the invention are themselves polypeptides. The functionality of the above mentioned modified polypeptides and fusion proteins can readily be determined by assaying the respective molecule for its Ca-binding ability as described further below. Another aspect of the invention relates to recombinant expression vectors and host cells comprising the nucleic acid molecules of the inventions. In yet another aspect the invention provides a method for the detection of changes in local calcium concentrations. In a further aspect the invention provides a method for detecting the binding of a small chemical compound or a polypeptide to a calcium-binding polypeptide with a homology of at least 80 % over a stretch of 30 amino acids to human Troponin C or chicken skeletal muscle Troponin C. The modified polypeptides of the invention are useful for the detection of local calcium concentrations, particularly local calcium concentration changes occurring close to a cellular membrane.

## DEFINITIONS

A "polypeptide" as used herein is a molecule comprising more than 30, and in particular more than 35, 40, 45 or even more than 50 amino acids, but less than 10,000, in particular less than 9,000, 8,000, 7,000, 6,000, 5,000, 4,000, 3,000, or 2,000, most preferably less than 1,500 amino acids. Polypeptides are usually linear amino acid polymers, wherein the individual amino acids are linked to one another via peptide bonds. Also, polypeptides which contain a low percentage, e.g. less than 5%, 3% or even only up to 1% of modified or non-natural amino acids, are encompassed. Polypeptides can be further modified by chemical modification, e.g. by phosphorylation of serine, threonine, or tyrosine residues, or by glycosylation, e.g. of asparagines or serine residues.

"Peptide" as used herein is a molecule comprising less than 30 amino acids, but preferably more than 4, 5, 6, 7, 8, or even more than 9 amino acids.

A "modified polypeptide" is a polypeptide which is not encoded as such by the genome of a naturally occurring species, in particular a polypeptide that is not identical to one of those polypeptides of the gene bank database as of July 28, 2003 with a naturally occurring species identified as its source. This means that a "modified" polypeptide does not occur as such in nature, but can be, and in particular was, produced by laboratory manipulations, such as genetic engineering techniques or chemical coupling of other molecules to a polypeptide. Examples of modified polypeptides are mutant polypeptides, in particular deletions, truncations, multiple substitutions, and fusion polypeptides, which at one stage were produced by genetic engineering techniques.

A polypeptide is a "calcium-binding polypeptide" if it has a Kd for $Ca^{2+}$ of lower than 800 μM, preferably lower than 600 μM and most preferably from 50 nM to 400 μM. A method for determining the Kd will be described below.

A polypeptide has "at least X % identity with" human Troponin C, SEQ ID NO. 20 or 24, or chicken skeletal muscle Troponin C, SEQ ID NO. 26, if, when a 30 amino acid stretch of its polypeptide sequence is aligned with the best matching sequence of human Troponin C or chicken skeleton muscle Troponin C, the amino acid identity between those two aligned sequences is X %. X can be 80 or more. For example, the corresponding polypeptide sequences in Troponin C molecules from other metazoan species, preferably other chordate species and more preferably other mammalian species, provide a source for such highly homologous polypeptides, which can substitute in the modified polypeptides of the invention for the corresponding sequences of human Troponin C or chicken skeleton muscle Troponin C. Preferably X is 85 or more, more preferably 90 or more, or most preferably 95 or more. It is to be understood that the case of sequence identity, that is 100% identity, is included.

Preferably, the nature of the amino acid residue change by which the polypeptide with at least X% identity to one of the reference sequences differs from said reference sequence is a semiconservative and more preferably a conservative amino acid residue exchange.

| Amino acid | Conservative substitution | Semi-conservative substitution |
|---|---|---|
| A | G; S; T | N; V; C |
| C | A; V; L | M; I; F; G |
| D | E; N; Q | A; S; T; K; R; H |
| E | D; Q; N | A; S; T; K; R; H |
| F | W; Y; L; M; H | I; V; A |
| G | A | S; N; T; D; E; N; Q; |
| H | Y; F; K; R | L; M; A |

(continued)

| Amino acid | Conservative substitution | Semi-conservative substitution |
|---|---|---|
| I | V; L; M; A | F; Y; W; G |
| K | R; H | D; E; N; Q; S; T; A |
| L | M; I; V; A | F; Y; W; H; C |
| M | L; I; V; A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | A; T; G; N | D; E; R; K |
| T | A; S; G; N; V | D; E; R; K; I |
| V | A; L; I | M; T; C; N |
| W | F; Y; H | L; M; I; V; C |
| Y | F; W; H | L; M; I; V; C |

Changing from A, F, H, I, L, M, P, V, W or Y to C is semiconservative if the new cysteine remains as a free thiol. Changing from M to E, R or K is semiconservative if the ionic tip of the new side group can reach the protein surface while the methylene groups make hydrophobic contacts. Changing from P to one of K, R, E or D is semiconservative, if the side group is on the surface of the protein. Furthermore, the skilled person will appreciate that Glycines at sterically demanding positions should not be substituted and that P should not be introduced into parts of the protein which have an alpha-helical or a beta sheet structure. Preferably, the above mentioned 30 amino acid stretch comprises a region with the above mentioned sequence identity with polypeptide sequences corresponding to amino acids 3 to 28, amino acids 28 to 40, 65 to 76, 105 to 116, or 141 to 152 of hTNNC1, which regions contain loops with Ca-binding capabilities.

As used herein, "FRET" relates to the phenomenon known as "fluorescence resonance energy transfer". The principle of FRET has been described for example in J.R. Lakowicz, "Principles of Fluorescence Spectroscopy", 2nd Ed. Plenum Press, New York, 1999. Briefly, FRET can occur if the emission spectrum of a first chromophore (donor chromophore or FRET-donor) overlaps with the absorption spectrum of a second chromophore (acceptor chromophore or FRET-acceptor), so that excitation by lower-wavelength light of the donor chromophore is followed by transfer of part of the excitation energy to the acceptor chromophore. A prerequisite for this phenomenon is the very close proximity of both chromophores. A result of FRET is the decrease/loss of emission by the donor chromophore while at the same time emission by the acceptor chromophore is observed. A pair of 2 chromophores which can interact in the above described manner is called a "donor-acceptor-pair" for FRET.

A "chromophore" as used herein is that part of a molecule responsible for its light-absorbing and light-emitting properties. A chromophore can be an independent chemical entity. Chromophores can be low-molecular substances, for example, the indocyanin chromophores CY3, CY3.5, Cy5, Cy7 (available from Amersham International plc, GB), fluorescein and coumarin (for example, from Molecular Probes). But chromophores can also be fluorescent proteins, like P4-3, EGFP, S65T, BFP, CFP, YFP, to name but a few.

The latter are also commercially available in a variety of forms, for example in the context of expression constructs.

"Human Troponin C" (hTnC or hTNC) comes in two forms: Troponin C from skeletal muscle, which is a 160 amino acid polypeptide with the Swissprot Accession Number P02585, and Troponin C from cardiac muscle, which is a 161 amino acid polypeptide with the Swissprot Accession Number P02590. Troponin C in chicken also comes in two forms, a form from cardiac muscle and a form from skeletal muscle. Troponin C from chicken skeletal muscle is also sometimes used herein and is a 163 amino acid polypeptide with the Swissprot Accession Number P02588 and is herein sometimes referred to as "cs-Troponin C" or "csTnC". Troponin C from chicken cardiac muscle is as defined in SEQ ID NO: 30. As used herein, the human Troponin C from cardiac muscle is sometimes called "hTNNC1" or "hcardTnC", while human Troponin C from skeletal muscle is sometimes called "hTNNC2" or "hsTnC". The structure of hTNNC1 is as follows: A helical region extending from amino acid 3 to amino acid 11 is followed by a second helical region from amino acid 14 to amino acid 28. The region from amino acid 28 to amino acid 40 is an ancestral calcium site which in its present form no longer binds calcium ions. The three calcium-binding regions in hTNNC1 are the EF-hand loop 2 extending from amino acid 65 to amino acid 76, EF-hand loop 3 from amino acid 105 to amino acid 116, and EF-hand loop 4 extending from amino acid 141 to amino acid 152.

The two best performing indicator constructs based on troponin C variants were given the names TN-humTnC for an indicator using the human cardiac troponin C (hcardTnC, SEQ ID NO. 3 and 4) as calcium binding moiety, and TN-L15 for an indicator using a truncated version (amino acids 15-163) of the chicken skeletal muscle troponin C (csTnC, SEQ ID NO. 1 and 2) as calcium binding moiety.

EF-hands are a type of calcium-binding domain shared among many calcium-binding proteins. This type of domain consists of a twelve-residue loop flanked on both sides by a twelve residue alphahelical domain. In an EF-hand the calcium ion is coordinated in a pentagonal-bipyramidal configuration. The six residues involved in the calcium-binding are in positions 1, 3, 5, 7, 9 and 12 of the twelve-residue loop. The invariant Glu or Asp residues at position 12 provide two oxygens for liganding $Ca^{2+}$-ions and work as a bidentate ligand in the coordination of $Ca^{2+}$.

As used herein, a "glycine-rich linker" comprises a peptide sequence with two or more glycine residues or a peptide sequence with alternating glycine and serine residues, in particular the amino acid sequences Gly-Gly, Gly-Ser-Gly, and Gly-Gly-Ser-Gly-Gly. With regard to glycine-rich linkers reference is made to Witchlow M. et al., "An improved linker for single-chain Fv with reduced aggregation and enhanced proteolytic stability", (1993) Prot. Engineering, 6: 989-995.

As used herein, a "localization signal" is a signal, in particular a peptidic signal, which leads to the compartmentalization of the polypeptide carrying it to a particular part of the cell, for example an organelle or a particular topographical localization like the inner or outer face of the cell membrane. Such a localization signal can be a nuclear localization signal, a nuclear export signal, a signal that leads to targeting to the endoplasmic reticulum, the mitochondrium, the Golgi, the peroxisome the cell membrane, or even to localize sub-fractions thereof, like pre- and/or postsynaptic structures.

A "ratio change" as used herein is defined by the following formula

$$\textbf{Ratio change [\%]} = \left( \frac{\left(\dfrac{IntensityYFP}{IntensityCFP}\right) inCa10mM}{\left(\dfrac{IntensityYFP}{IntensityCFP}\right) inCafree} \bullet 100 \right) - 100$$

To obtain the ratio change in % of a modified polypeptide of the invention, the fluorescence emission intensities of the FRET-donor and the FRET-acceptor are measured at their respective emission maxima under suitable conditions. First, the values are determined in a calcium-free buffer solution. For example, the calcium-free buffer solution contains an aliquot of the modified polypeptide of the invention to be tested in 10 mM MOPS pH 7.5, 100 mM KCl and 20 $\mu$M EGTA. After the first measurement a solution of 1 M $CaCl_2$ is added to the mix to a final concentration of 10 mM $CaCl_2$. Then the respective emission maxima of the FRET-donor and the FRET-acceptor are measured again. The concentration of the modified polypeptide of the invention to be tested in this manner should be such that the change in FRET is readily detected. As a guideline, suitable concentrations range from 500 nM to 5 $\mu$M. Reference is made to Miyawaki, A. et al., "Fluorescent indicators for Ca2+ based on green fluorescent proteins and calmodulin." (1997) Nature 388:882-887.

Kd-values of the calcium-binding polypeptides for $Ca^{2+}$ ions can be determined as follows. Fusion polypeptides of CFP with the calcium-binding polypeptide followed by citrine are expressed by methods well known in the art, e.g. following the procedure of Example 2. The fusion polypeptides are purified following the procedure of Example 2 and stored in 300 mM NaCl, 20 mM $NaPO_4$-buffer pH 7.4. Kd-values are then determined by titration assays, in which the proteins are exposed to defined calcium concentrations in an aqueous buffer. To produce such defined calcium concentrations, a buffer system containing $Ca^{2+}$ and its chelator $K_2$ EGTA is used. Aliquots of the protein are mixed with various ratios of two buffer solutions containing either 10 mM $K_2$ EGTA, 100 mM KCl and 30 mM MOPS pH 7.2 or 10 mM Ca EGTA, 100 mM KCl and 30 mM MOPS pH 7.2. The fluorescence emission intensities of the FRET-donor and the FRET-acceptor are then recorded at various concentrations of free calcium. Calcium Kd-values can be calculated by plotting the ratio of the donor and acceptor proteins' emission maximum wavelength against the concentration of free calcium on a double logarithmic scale. Thus, plotting $\log[Ca^{2+}]_{free}$ on the x-axis versus $\log\left(\dfrac{R - R\min}{R\max - R} \bullet \dfrac{F_{527}\min}{F_{527}\max}\right)$ on the y-axis gives an x-intercept that is the log of the proteins Kd in moles/liter.

In the formula above, R is the fluorescence intensity of the emission maximum at lower wavelength (527 nm for YFP/citrine) divided by the fluorescence intensity of the emission maximum at shorter wavelength (432 nm for CFP) at the various calcium concentrations tested. Rmin is the ratio R in a calcium-free sample, i.e. in buffer 1 only. Rmax is the ratio R in the presence of the highest chosen calcium concentration, for example at 1 mM $Ca^{2+}$ if the ratio to buffer 1 to buffer 2 is 1:1. $F_{527}$min is the fluorescence intensity of the emission maximum at lower wavelength (527 nm for citrine) in a calcium-free sample. $F_{527}$max is the fluorescence intensity of the emission maximum at longer wavelength (527 nm for citrine) in the presence of the highest chosen calcium concentration. Further details on the measuring method are disclosed in POZZAN T and TSIEN RY (1989) Methods Enzymol., 172:230-244.

The "local $Ca^{2+}$ concentration" as used herein is a change in calcium concentration, particularly a rise, which is restricted either to membrane-combined cellular organelles or to cellular structures that can handle calcium relatively independently of the remained of the cytosol, such as dendritic spines or shafts or presynaptic boutons. By "local" we also mean changes in the free calcium concentration confined to submicroscopic microenvironments in the cytosol close to a cellular membrane. By "submicroscopic" we mean areas with an extension smaller than 350 nm.

As used herein, the term "inducing a change in the calcium concentration" is any experimental regime which leads to a temporal or spatial change of the calcium distribution within a cell. In the case of studies in cell lines, cell surface receptors which are coupled to the production of an intracellular messenger such as IP3 can lead to a rise in cytosolic or mitochondrial calcium in the cell, when they are activated. An example of such surface receptors are members of the family of G-protein-coupled receptors including olfactory and taste receptors, further receptor tyrosin kinases, chemokine receptors, T-cell receptors, metabotropic amino acid receptors such as metabotropic glutamate receptors or $GABA_b$-receptors, GPI-linked receptors of the TGF beta/GDNF-(glial-derived neurotrophic factor) receptor family. Other receptors can also directly gate calcium influx into cells, such as NMDA receptors or calcium-permeable AMPA receptors. In the case of studies with indicator organisms like transgenic C-elegans or drosophila, administration of a suitable stimulus to the organism may lead to such a calcium redistribution in certain cells which can then give an observable readout. This can, for example, be the administration of a drug to the organism, but also a stimulus with a suitable modality such as of visual, acoustic, mechanic, nociceptive or of hormonal nature. The stimulus can be, for example, cold shock, mechanical stress, osmotic shock, oxidative stress, parasites or also changes in nutrient composition in the case of transgenic plants.

A "small chemical compound" as used herein is a molecule with a molecular weight from 30 D - 5 kD, preferably from 100 D - 2 kD. A "small organic chemical molecule" as used herein further comprises at least one carbon atom, one hydrogen atom and one oxygen atom. Such small chemical compounds can, e.g., be provided by using available combinatorial libraries.

## DETAILED DESCRIPTION OF INVENTION

The present invention is based on the discovery that the calcium-binding protein Troponin C can form the basis for particularly powerful calcium sensors. The modified polypeptide of the invention allows the measurement of calcium fluctuations in cellular microenvironments where prior art calcium sensors like the calmodulin-based "Cameleons" have failed or have only shown poor performance. Furthermore, the Troponin C-based calcium sensors of the invention show minimal interference with the intracellular signalling pathways based on calcium and are therefore, contrary to the prior art "Cameleons", even suitable for use in transgenic vertebrates and even mammals.

Thus, the present invention relates to a modified calcium ($Ca^{2+}$)-binding polypeptide comprising (a) a first chromophor of a donor-acceptable pair for FRET, (b) a calcium-binding polypeptide with an identity of at least 80%, preferably 85%, more preferably 90%, even more preferably 95%, and most preferably with 100% identity, to a 30 amino acid long polypeptide sequence of human Troponin C or chicken skeleton muscle Troponin C, and (c) a second chromophor of a donor-acceptable pair for FRET, more preferably the stretch of the calcium-binding polypeptide with this high degree of identity to human Troponin C or chicken skeletal muscle Troponin C is a 35 amino acid, 40, 45, 50, 55, 60, 65, 70, or even 75 amino acid long polypeptide sequence. These polypeptides are capable of binding Ca2+ ions which induces a conformational change. This functionality can readily be determined as described above. Suitable chromophores are both small fluorescent molecules like, for example, the indocyanin dyes Cy3, Cy3.5, Cy5, Cy7, coumarin, fluoresceine or rhodamine, but also fluorescent polypeptides, like certain derivatives of GFP, the "green fluorescent protein", in particular mutants of GFP with increased stability, or changed spectral characteristics, like EGFP, CFP, BFP or YFP. Other suitable fluorescent polypeptides are cFP 484 from Clavularia and zFP 538, the Zoanthus yellow fluorescent protein. As explained above, the donor chromophore and the acceptor chromophore of a donor-acceptor-pair for FRET must be chosen with regard to their spectral characteristics. As a general rule, a donor chromophore has an absorbance-maximum at lower wavelength, i.e. absorbing higher energy radiation, than an acceptor chromophore. For that reason, CFP, EGFP and YFP (citrine), all derived from Aequoria victoria, DsFP 483 from Discosoma striata., cFP 484 from Clavularia sp., AmCyan from Anemonia majano, Azami-Green from Galaxeidae sp. und As499 from Anemonia sulcata (see Tsien R.Y. "The green fluorescent protein". Ann. Rev. Biochem. 67: 509-544 (1998); Matz M.V. et al. "Fluorescent proteins from nonbioluminescent Anthozoa species." Nat. Biotechnol. 17: 969-973 (1999); Wiedenmann J. et al. "Cracks in the β-can: Fluorescent proteins from Anemonia Sulcata (Anthozoa, Actinaria)" Proc. Natl. Acad Sci. 97: 14091-14096 (2000); Labas Y.A. et al. "Diversity and evolution of the green fluorescent protein family". Proc. Natl. Acad Sci. 99: 4256-4261 (2002). Karasawa S. et al. "A green emitting fluorescent protein from Galaxeidae coral and its monomeric version for use in fluorescent labelling. J. Biol. Chem. [epub ahead of print] (2003)) can be commonly used as donor chromophores. Examples of commonly used acceptor chromophores are YFP (citrine), DsRed from Discosoma sp., zFP 538 from Zoanthus sp., HcRed from Heteractis crispa, EqFP 611 from Entacmaea quadricolor and AsFP 595 from Anemonia sulcata (see Matz M.V. et al. "Fluorescent proteins from nonbioluminescent Anthozoa species." Nat. Biotechnol. 17: 969-973 (1999); Wiedenmann J. et al. "Cracks in the β-can: Fluorescent proteins from Anemonia Sulcata (Anthozoa, Actinaria)" Proc.Natl. Acad Sci. 97: 14091-14096 (2000);. Gurskaya N.G. et al. "GFP-like chromoproteins as source of far-red fluorescent proteins" FEBS lett. 507: 16-20 (2001); Wiedenmann J. et al. A far red fluorescent protein with fast maturation and reduced oligomerization tendency from Entacmaea quadricolor (Anthozoa, Actinaria)" Proc. Natl. Acad Sci. 99: 11646-11651 (2002)). The example of YFP makes clear that depending on its partner in a donor-acceptor-pair for FRET, a particular chromophore may serve as either a donor or an acceptor. For example, YFP can serve as an acceptor chromophore, when in combination with BFP, CFP, cFP 484, AmCyan or DsFP483 and it can function as a donor chromophore when in combination with DsRed, EqFP 611 or HcRed. By analysing the spectral characteristics of two chromophores, the skilled person can identify suitable donor-acceptor-pairs for FRET.

The three components of the modified calcium-binding polypeptide of the invention are linked together by covalent linkages. These covalent linkages between the components (a) and (b) and the components (b) and (c) can be effected by chemical crosslinking. That is, the three components can initially be independent of one another and can then be crosslinked chemically, for example by a spacer which can be selected from the group consisting of bifunctional crosslinkers, flexible amino acid linkers, like the hinge region of immunoglobulins, and homo- and heterobifunctional crosslinkers. For the present invention preferred linkers are heterobifunctional crosslinkers, for example SMPH (Pierce), sulfo-MBS, sulfo-EMCS, sulfo-GMBS, sulfo-SIAB, sulfo-SMPB, sulfo-SMCC, SVSB, SIA and other crosslinkers available, for example from the Pierce Chemical Company (Rockford, IL, USA). Such a preferred chemical crosslinker has one functional group reactive towards amino groups and one functional group reactive towards cystine residues. The above-mentioned crosslinkers lead to formation of thioether bonds, but other classes of crosslinkers suitable in the practice of the invention are characterized by the introduction of a disulfide linkage between the polypeptides of (b) and the component (a) and/or between the polypeptide of (b) and the component of (c). It is apparent that activated conjugates of small chemical fluorophores, like FITC or like rodamine succinimidyl esters, can directly react with nucleophiles like the sulfhydryl groups of cysteins or the amino groups of lysines in the calcium-binding polypeptide of component (b) and thereby create a covalent linkage between (a) and (b) and/or (b) and (c). Amine-reactive dyes and thiol-reactive dyes can be obtained, for example from Molecular Probes Europe BV, Leyden, The Netherlands.

In a preferred embodiment, the modified calcium-binding polypeptide comprises a first chromophore (a), which is a fluorescent polypeptide capable of serving as a donor-chromophore in a donor-acceptor-pair for FRET, and a second chromophore (c), which is a fluorescent polypeptide capable of serving as an acceptor-chromophore in a donor-acceptor-pair for FRET. Preferably, the three polypeptides are part of one fusion polypeptide and the order of the three linked polypeptides starting from the N-terminus of the fusion polypeptide may be (a)-(b)-(c) or (c)-(b)-(a). It is to be understood that there may be further amino acids in the fusion polypeptide at the N- or at the C-terminus as well as between the polypeptides (a) and (b) and/or (b) and (c). In a preferred embodiment, the first chromophore (a) is selected from the group consisting of CFP, EGFP and YFP (Citrine), all derived from Aequoria victoria, DsFP 483 from Discosoma striata, AmCyan from Anemonia majano, cFP 484 from Clavularia sp., Azami-Green from Galaxeidae sp. and As499 from Anemonia sulcata

In another preferred embodiment, the second chromophore is selected from the group consisting of small fluorescent molecules like, for example, the indocyanin dyes Cy3, Cy3.5, Cy5, Cy7, fluoresceine or rhodamine, but also fluorescent polypeptides, like certain derivatives of GFP, the "green fluorescent protein", in particular mutants of GFP with increased stability, or changed spectral characteristics, like EGFP, CFP, BFP or YFP. Other suitable fluorescent polypeptides are cFP 484 from Clavularia and zFP 538, the Zoanthus yellow fluorescent protein.

In another preferred embodiment, the calcium-binding polypeptide of component (b) comprises at least one calcium-binding EF-hand, and in particular comprises 2 or even 3 calcium-binding EF-hands. Most preferably, the modified calcium-binding polypeptide of the invention contains 4, 3, 2 or even only 1 EF-hand. The skilled person will appreciate that the ancestral calcium-binding site of human Troponin C or chicken skeletal muscle Troponin C may be genetically engineered such that its calcium-binding properties are restored.

In a preferred embodiment the calcium-binding polypeptide of the invention comprises a polypeptide sequence with at least 60% identity, more preferably at least 70%, even more preferably at least 80%, even more preferably at least 90% or most preferably 100% identity to amino acids 15-163 of chicken skeletal muscle Troponin C or amino acids 1-161 of human cardiac Troponin C. In this case, 100% identity means 100% identity over the complete 148 or 161 amino acid stretch, respectively.

As mentioned previously, there can be linker sequences between component (a) and (b) and component (b) and (c) of the modified calcium-binding polypeptide of the invention. In one preferred embodiment the polypeptide of the invention therefore further comprises glycin-rich linker-peptides N-terminal or C-terminal to polypeptide (b), particularly directly neighbouring polypeptide (b) on its N-terminus or C-terminus.

In another preferred embodiment the modified calcium-binding polypeptide of the invention further comprises a localization signal, in particular a nuclear localization sequence, a nuclear export sequence, an endoplasmic reticulum localization sequence, a peroxisome localization sequence, a mitochondrial input sequence, a mitochondrial localization sequence, a cell membrane targeting sequence, and most preferably a cell membrane targeting sequence mediating localization to pre- or postsynaptic structures. It has been found that a particular advantage of the modified calcium-binding polypeptides of the invention is that they function in the context of subcellular environments where prior art calcium sensor have failed to work or have shown poor performance. The calcium sensors of the present invention are therefore particularly powerful when targeted to specific subcellular structures, like organelles or functionally distinct regions of the cell-like lamellipodia or filophodes or axons and dendrites in the case of neuronal cells. Such subcellular targeting can be accomplished with the help of particular targeting sequences. Localization to the endoplasmic reticulum can be achieved by fusing the signal peptide of calreticulin, MLLSVPLLLGLLGLAAAD to the N-terminus of a fusion polypeptide and the sequence KDEL as an ER retention motive to the C-terminus of a fusion polypeptide (discussed in Kendal et al. "Targeting aequorin to the endoplasmic reticulum of living cells." Biochem. Biophys. Res. Commun. 189: 1008-1016, (1992)). Nuclear localization can be achieved, for example, by incorporating the bipartite NLS from nucleoplasmin in an accessible region of the fusion polypeptide or, alternatively, the NLS from SV 40 large T-antigen. Most conveniently, those sequences are placed either at the N- or the C-terminus of the fusion polypeptide.

Nuclear exclusion and strict cytoplasmic localization can be mediated by incorporating a nuclear export signal into the modified calcium-binding polypeptide of the invention. Such signals are useful when the modified calcium-binding polypeptide of the invention is smaller than 60 kDa. Nuclear exclusion may not be necessary for modified calcium-binding polypeptides of the invention which are larger than 60 kDa because such polypeptides usually do not enter the cell nucleus and are therefore cytosolic at steady state. Suitable nuclear export signals are the NES from HIV Rev, the NES from PKI, AN3, MAPKK or other signal sequences obtainable from the NES base (http://www.cbs.dtu.dk/databases/NESbase/). For review of nuclear localization signals and nuclear export signals see Mattaj & Englmeier, "Nuclear cytoplasmic transport: the soluble phase" (1998), Annu. Rev. Biochem. 67:265-306.

Mitochondrial targeting can be achieved by fusing the N-terminal 12 amino acid pre-sequence of human cytochrome C oxidase subunit 4 to the N-terminus of a fusion polypeptide (for reference see Livgo, T. "Targeting of proteins to mitochondria" (2000) FEBS Letters, 476:22-26; and Hurt, E.C. et al. (1985) Embo J., 4:2061-2068). Targeting to the Golgi apparatus can be achieved by fusing the N-terminal 81 amino acids of human galactosyl transferase to the N-terminus of a fusion polypeptide and leads to targeting to the trans-cisterne of the Golgi apparatus. (For reference see Llopis J. et al. (1999) Proc. Natl. Acad Sci. USA 95(12):6803-8.)

Suitable targeting sequences for peroxisomal targeting are PTS1 and PTS2. (For reference see Gould S.G. et al. (1987) J. Cell Biol. 105:2923-2931; and Ozumi T. et al. (1991) Biochem. Biophys. Res. Commun., 181:947-954.)

For targeting to the inner leaflet of the cell membrane, the first 20 amino terminal amino acids of GAP-43 (growth associated protein) are useful, i.e. the sequence MLCCMRRTKQVEKNDEDQKI. Alternatively, membrane targeting can be achieved by fusing the 20 most C-terminal amino acids of C-Ha-Ras to the C-terminus of a fusion polypeptide. These amino acids are KLNPPDESGTGCMSCKCVLS. (For reference see Moryoshi K. et al. (1996) Neuron, 16:255-260.)

Targeting to postsynaptic sites can be achieved by fusing the C-terminal PDZ-binding domain of the NMDA-receptor 2B subunit to the C-terminus of a fusion polypeptide. The sequence is VYEKLSSIESDV. Alternatively, the PDZ-binding domain of the inwardly rectifying potassium channel KIR 2.3 can be used as a localization when added to the C-terminus. The sequence is MQAATLPLDNISYRRESAI. (For reference see Liedhammer M. et al. (1996) J. Neurosci., 16:2157-63, and Lemaout S. et al. (2001) Proc. Natl. Acad Sci. USA, 98:10475-10480.) Other PDZ-binding domains useful for localizing indicators can be found in Hung and Sheng (2001) J. Biol. Chem., 277:5699-5702.

Presynaptic targeting can be achieved by fusing presynaptic protein such as syntaxin or synaptobrevin (VAMP-2) to the fusion polypeptides of the invention. (For reference see Bennett et al. (1992) Science, 257:255-259, and Elferink et al. (1989) J. Biol. Chem., 264:11061-4.)

A further preferred embodiment of the invention is a modified calcium-binding polypeptide of the invention which exhibits a ratio change upon calcium addition of more 30%, preferably from 50% to 200%, more preferably from 80% to 180%, even more preferably from 90% to 160%, and most preferably from 100% to 150%. Ratio change is as defined above and calcium is added to a final concentration 10 mM $CaCl_2$ (i.e. an appropriate volume of an 1 M aqueous solution of $CaCl_2$ is added to a buffer containing the polypeptide of the invention, 10 mM MOPS, pH 7.5, 100 mM KCl and 20 $\mu$M EGTA so that the final concentration is 10 mM $CaCl_2$. The polypeptides exhibiting such ratio changes are particularly preferred because they facilitate the measurement of calcium concentration changes within a living cell due to their low signal-to-noise ratio.

In another preferred embodiment, the modified calcium binding polypeptide of the invention has a Kd for $Ca^{2+}$ of below 800 μM, preferably of from 50 nM to 400 μM, more preferably of from 100 nM to 100 μM, and most preferably of from 250 nM to 35 μM. As shown in the exemplifying section, the Kd of the modified calcium-binding polypeptide of the invention for $Ca^{2+}$ ions can be manipulated by targeted mutation of the calcium-binding EF-hands of the Troponin C-derived polypeptide. (For reference see Szczesna et al., (1996) J. Biol. Chem. 271:8381-8386, and Sorensen et al., (1995) J. Biol. Chem. 270:9770-9777). In these references the effects of mutations within the 12 amino acid loops of the EF-hand on the Kd of a calcium-binding polypeptide for calcium are explained. Thus, within certain limits, calcium-binding biosensors can be designed which have the desired affinity for calcium ions.

In a further preferred embodiment, the modified calcium-binding polypeptide of the invention is a fusion polypeptide selected from any one of the polypeptides of SEQ ID NO2, 4, 6, 8, 10, 12, 14, 16 or 18, preferably 2 or 4.

In another aspect the invention provides a nucleic acid molecule comprising a nucleic acid sequence which encodes any one of the above-mentioned fusion polypeptides. In particular, a fusion polypeptide wherein the order of the three linked polypeptides starting from the N-terminus of the fusion polypeptide is (a)-(b)-(c) or (c)-(b)-(a). In a preferred embodiment the nucleic acid comprises (i) a nucleic acid sequence as defined in the SEQ IDs NO:1, 3, 5, 7, 9, 11, 13, 15 or 17, preferably 1 or 3, (ii) a nucleic acid sequence which is degenerate as a result of the genetic code to the nucleic acid as defined in (i) and which encodes a polypeptide as defined in SEQ IDs NO:2, 4, 6, 8, 10, 12, 14, 16 or 18, preferably 2 or 4, or a polypeptide with at least 80% identity to said polypeptides within a 30 amino acid stretch, preferably within a stretch of 45, 60 or even 75 amino acids.

A further embodiment of the invention is a recombinant expression cassette, in particular a vector, comprising a nucleic acid of the invention which is operably linked to at least one regulator sequence allowing expression of the modified protein of the invention. For example, a nucleic acid sequence encoding a modified polypeptide of the invention can be isolated and cloned into an expression vector and the vector can then be transformed into a suitable host cell for expression of a modified polypeptide of the invention. Such a vector can be a plasmid, a phagemid or a cosmid. For example, a nucleic acid molecule of the invention can be cloned in a suitable fashion into prokaryotic or eukaryotic expression vectors (Molecular Cloning: A Laboratory Manual, 3rd edition, eds. Sambrook et al., CSHL Press 2001). These expression vectors comprise at least one promoter and can also comprise a signal for translation initiation and - in the case of prokaryotic expression vectors - a signal for translation termination while in the case of eukaryotic expression vectors preferably expression signals for transcriptional termination and polyadenylation are described. Examples for prokaryotic expression vectors are, for expression in Escherichia Coli, e.g. expression vectors based on promoters recognized by T7 RNA polymerase as described in US 4,952,496, for eukaryotic expression vectors, for expression in Saccharomyces cerevisiae, e.g. the vectors G426/MET25 or P526/GAL1 (Mumberg et al. (1994), Nucl. Acids Res., 22: 5767-5768), for the expression in insect cells, e.g. via bacculovirus vectors, those described by Ziccarone et al. ("Generation of recombinant bacculovirus DNA in E. coli using bacculovirus shuttle vector" (1997) Volume 13, U. Reischt et. (Totoba, N. J.: Humana Press Inc.) and for expression in mammalian cells, e.g. SW40-vectors, which are commonly known and commercially available, or the Sindbis virus expression system (Schlesinger (1993) Trans Bio Technol. 11 (1):18-22) or an adenovirus expression system (Heh et al. (1998) Proc. Natl. Acad. Sci. USA 95:2509-2514). The molecular biological methods for the production of these expression vectors as well as the methods for transfecting host cells and culturing such transfecting host cells as well as the conditions for producing and obtaining the polypeptides of the invention from said transformed host cells are well known to the skilled person.

In another aspect the invention relates to a host cell comprising a polypeptide, in particular a fusion polypeptide of the invention and/or a nucleic acid of the invention. Such a host cell can be a non-human cell inside or outside the animal body or a human cell outside the human body. Particularly preferred are mammalian cells like HEK cells, HELA cells, PC12 cells, CHO cells, NG108-15 cells, Jurkat cells, mouse 3T3 fibroblasts, mouse hepatoma (hepa 1C1C7 cells), mouse hepatoma (H1G1 cells), human neuroblastoma cell lines, but also established neuronal and cancer cell lines of human and animal origin available from ATCC (www.atcc.org). But host cells can also be of non-mammalian origin or even of non-vertebrate origin, like Drosophila Schneider cells, yeast cells, other fungal cells or even grampositive or gramnegative bacteria. Particularly preferred are cells within a transgenic indicator organism and also the transgenic indicator organisms comprising the host cell of the invention. The generation of transgenic flies, nematodes, zebrafish, mice and plants, for example Arabidopsis thaliana, are well established. For the generation of transgenic mice with suitable cell- or tissue-specific promoters reference is made to Hogan D. et al. (1994) "Production of transgenic mice" in Manipulating the Mouse Embryo: A Laboratory Manual - Hogan D., Constantini, F., Lacey E. eds., Cold Spring Harbor Laboratory Press, Cold Spring Harbor NY, pp. 217-252. As an alternative, indicators can be expressed as transgenic mice with an inducible system; reference is made to Albanese C. et al., "Recent advances in inducible expression in transgenic mice" (2002) Semin. Cell. Dev. Biol., 13:129-41. Methods for the generation of transgenic flies, nematodes, zebrafish, and transgenic plants are also well established and exemplatory reference is made to the following documents: Rubin & Spreadling (1982) Science 218:348-53, for the generation of transgenic flies; Mellow & Fire (1995) in: Methods in Cell Biology, Volume 48, H.F. Epstein & T.C. Shakes, eds. (San Diego, CA: Academic Press), pp. 451-482, Higashiyima et al. (1997), Dev. Biol. 192:289-99 for the transformation of zebrafish, and Bechtold et al. (1993) C. R. Acad. Sci. [III] 316:1194-1199 for the transformation of Arabidopsis taliana plants.

In another aspect the invention relates to a method for detecting changes of the local calcium concentration which comprises the following steps: (a) providing a cell or a subcellular membrane as faction of a cell, which cell or subcellular membraneous faction comprise a calcium-binding modified polypeptide of the invention, (b) inducing a change in the local calcium concentration, and (c) measuring FRET between the donor and the acceptor chromophore of the donor-acceptor-pair of said modified calcium-binding polypeptide of the invention, wherein a change in FRET as a response to step (b) is indicative of a change in the local calcium concentration. These steps are all performed under suitable conditions. Provision of the cell in step (a) can be achieved by providing a host cell of the invention, for example a host cell transfected with an expression construct coding for a fusion polypeptide of the invention, in which, as an example, a polypeptide of the invention is expressed, e.g. as in the examples 3 or 4. A subcellular membraneous fraction comprising a calcium-binding polypeptide of the invention can be obtained by biochemical fractionation of the cellular constituents of, for example, above-mentioned host cell. A subcellular membraneous fraction can, for example, be Golgi or ER-derived vesicles from said host cell or isolated organelles from said host cell, like pelleted nuclei or mitochondria. The methods to obtain subcellular membraneous fractions from, for example, cells from cell culture, are well known in the art (for reference see for example McNamee, MG (1989) Isolation and characterization of cell membranes, Biotechnique 7: 466-475 and Joost HG and Schurmann, A. (2001), Subcellular fractionation of adipocytes and 3T3 L1 cells, Meth. Mol. Biol. 155:77-82).

In a preferred embodiment, the subcellular membraneous factioned is an organelle, in particular a mitochondrium, peroxisome, or a nucleus, or a membrane faction derived from a membrane-bound organelle. Particularly interesting are membrane factions derived from the cell membrane. However, in a preferred embodiment a cell, for example a cell in the context of a cell culture dish or a cell in the context of a transgenic organism, if the cell is a non-human cell, is provided in step (a). And preferably the calcium-binding polypeptide of the invention is targeted to a specific subcellular localization within said cell, and most preferably is targeted to the inner surface of the cell membrane.

As explained above, a change in the local calcium concentration can be induced by various stimuli, like the administration of extracellular stimuli. In a preferred embodiment step (b) is effected by the administration of an extracellular stimulus, and in particular by the addition of a small chemical compound or a polypeptide to the extracellular side of the host cell. If changes of the local calcium concentration are to be measured in a cell in the context of a cell culture dish, then it is preferred that the fusion polypeptides of the invention are co-expressed together with a receptor protein or ion channel protein of interest whose activation can be read out in the form of a calcium signal. Such receptors can be receptors coupled to the production of an intracellular messenger such as IP3 that leads to a rise in cytosolic or mitochondrial calcium in the cell when the receptor or ion channel is stimulated, for example, members of the family of G-protein coupled receptors including olfactory and taste receptors, receptor tyrosine kinases, chemokine receptors, T-cell receptors, metabotropic amino acid receptors such as metabotropic glutameic receptors or $Gaba_b$-receptors, GPI-linked receptors of the TGFbeta/GDNF-(glial-derived neurotrophic factor) receptor family. Receptors can also be directly gating calcium influx into transfector cells such as NMDA receptors or calcium-permeable AMPA receptors. Also interesting are calcium channels that are gated by membrane potential under physiological conditions, such as L-type, P/Q-type and N-type calcium channels. After co-expression of the calcium sensors of the invention and such an receptor or channel has been achieved, in the next step (b) an agonist or antagonist of said receptor or channel is provided to the co-transfected host cell, and then in step (c) the change in the local calcium concentration can be read out on a microscope stage by exciting the donor chromophor at a suitable wavelength using a suitable light source such as Xenon Arc Lamp, a monochromator or a laser light source, suitable dichroic mirrors and excitation filters and emission filters of suitable bandwith to extract information on the donor and acceptor emission, finally by recording the signals on a CCD (charge-coupled device) camera or a photomultiplier tube.

If the cell is provided in the context of an indicator organism, then the method is performed by expressing the fusion polypeptide of the invention in a transgenic organism in a cell or tissue type of interest with the help of suitable specific, constitutive or inducible promoters. As the next step (b), a suitable stimulus is provided to the organism, for example a drug is administered to the organism or alternatively a stimulus of suitable modality is provided to the organism, such as a visual, acoustic, mechanic, nociceptive or hormonal stimulus. This stimulus elicits a calcium signal which can be detected when the fusion polypeptide of the invention is expressed in tissues of interest within the transgenic animal, such as the nervous system or in intestinal organs. The stimulus can be, for example, cold shock, mechanical stress, osmotic shock or oxidative stress, parasites or changes in nutrient composition. The change of the local calcium concentration can then be read out by microscopy of the cell or tissue of interest, as indicted above.

In another aspect the invention provides a method for the detection of the binding of a small chemical compound or a polypeptide to a calcium-binding polypeptide with an identity of at least 80% to a 30 amino acid long polypeptide sequence of human Troponin C or chicken skeletal muscle Troponin C. This method comprises the steps of (a), providing a calcium-binding polypeptide of the invention, (b) adding a small chemical compound to be tested for binding or a polypeptide to be tested for binding, and (c) determining the degree of binding by measuring FRET between the donor- and the acceptor-chromophor of the donor-acceptor-pair of said polypeptide under suitable conditions. In a preferred embodiment the calcium-binding polypeptide provided in step (a) is human cardiac muscle Troponin C or a polypeptide derived from human cardiac muscle Troponin C and, in particular, is SEQ ID NO:4. This method is useful to identify small chemical compounds or polypeptides of clinical interest, in particular as in certain clinical settings, such as congenital heart failure, cardiomyopathy or other myocardial diseases such as induced by diabetes leading to reduced performance of the human heart. The method is also useful in identifying compounds that strengthen or weaken skeletal muscle contractive force. Such compounds that strengthen skeletal muscle function can be beneficial therapeutics in diseases leading to muscle degeneration, such as muscular dystrophies as for example Duchenne muscular dystrophy, or spinal muscular atrophy.

Compounds that weaken skeletal muscle contraction may find its use in conditions that lead to excessive muscle convulsions, as for example in Tetanus. Small chemical molecules or polypeptides which help to improve the calcium-binding properties of human cardiac muscle Troponin C or human skeletal muscle troponin C have the potential of being suitable pharmaceuticals for the treatment of the above-mentioned diseases. In a preferred embodiment the small chemical compounds or the polypeptides identified by the above-mentioned screening method are formulated into a pharmaceutical composition which can be used for the treatment of the above-mentioned disease.

In another aspect the invention relates to the use of a modified calcium-binding polypeptide of the invention for the detection of changes in the local calcium concentration within a cell, and in particular for the detection of calcium changes occurring close to a cellular membrane. In one aspect this can be for diagnostic purposes in a subject, e.g. a human patient. Also the modified calcium-binding polypeptide of the invention can be used for the detection of changes in the local calcium concentration within a cell of a transgenic animal of the invention, like a transgenic mouse, or preferably a non-mammalian transgenic animal, like transgenic bakers yeast, *C. elegans, D. melanogaster* or zebrafish. In another aspect this use is not contemplated to be practiced on a human or animal body, but relates to an *ex vivo* use, in particular an *in vitro* use, e.g. in cell lines or in primary cells in cell culture.

In a preferred embodiment such modified calcium-binding polypeptides are used which comprise a localization signal, in particular a nuclear localization signal, a nuclear export signal, an endoplasmic reticulum localization signal, a peroxisome localization signal, a mitochondrial input signal, a cell membrane targeting signal, or a cell membrane targeting signal mediating localization to pre- or postsynaptic structures. Most preferably, such modified calcium-binding polypeptides are used which comprise a cell membrane targeting signal, and in particular a cell targeting signal mediating localization to the cell membrane of pre- or postsynaptic structures. It is desirable that the modified calcium-binding polypeptide is a genetically encoded fusion polypeptide of the invention.

DESCRIPTION OF THE FIGURES

**Figure 1:** Schematic representation of FRET occurring in ratiometric indicators based on troponin C variants.

**Figure 2:** Summary of basic constructs and evaluation of their function. csTnC, chicken skeletal muscle troponin C. csTnC-N90, the N-terminal lobe of chicken skeletal troponin C (amino acids 1-90). csTnC-EFn, the individual EF hands 1-4 of chicken skeletal muscle troponin C. csTnI, chicken skeletal muscle troponin I. csTnI 1-48, csTnI 95-133, csTnI 116-135, various short peptides derived from chicken skeletal muscle troponin I consisting of the indicated amino acid residues. csTnC-L15, truncated chicken skeletal muscle troponin C in which the N-terminal amino acid residues 1-14 are deleted, which makes the protein start at leucin 15. The whole indicator construct was named TN-L15 (SEQ ID NO. 1 and 2). csTnC-L 15 D107A, csTnC-L15 carrying the mutation D107A. The whole indicator construct was named TN-L 15 D107A (SEQ ID NO. 5, 6). csTnC-L15-N90, N-terminal lobe of chicken skeletal muscle troponin C consisting of amino acid residues 15-90. hcardTnC, human cardiac muscle troponin C. The whole indicator construct is referred to as TN-humTnC (SEQ ID NO. 3, 4). hcardTnC1-135, human cardiac muscle troponin C lacking the last EF hand domain. hcardTnC-L12, human cardiac muscle troponin C in which the N-terminal amino acid residues 1-11 are deleted, analogous to csTnC-L15. L, linker: either GG, GSG or GGSGG.

**Figure 3**: Effect of calcium binding on the emission spectrum of two indicator constructs; **A:** TN-L15. **B:** TN-humTnC. The emission spectra of the two constructs are depicted at zero (dashed line, - $Ca^{2+}$) and saturating (solid line, + $Ca^{2+}$) calcium levels.

**Figure 4**: Calcium affinities (A) and pH-sensitivities (B) of selected indicator proteins. **A:** TN-humTnC (open diamonds), TN-L15 (filled squares), TN-L15 D107A (open circles), TN-L15 E42Q/E78Q (filled circles). **B:** Emission ratio of TN-15 in the presence (circles) and absence (squares) of calcium at various pH values.

**Figure 5:** Calcium dissociation from selected purified indicator proteins.

**Figure 6:** Function of TN-L15 within live cells. **A:** HEK 293 cells displaying cytosolic localization and different expression levels (cell 1 and 2) of TN-L15. **B:** Ratio traces of the two cells depicted in **A**. Responses to stimulation with 100 μM carbachol and treatment with 1 μM ionomycin at high calcium to obtain Rmax and at 100 μM EGTA to obtain Rmin are shown. C: Corresponding intensity traces of CFP and Citrine emission for the ratios in B, showing individually the traces of the higher expressing cell 1 and the dimmer expressing cell 2.

**Figure 7:** Function of TN-humTnC in live cells. **A:** Cytosolic expression of TN-humTnC in HEK293 cells. **B:** Imaging trace showing the 527/476 nm emission ratio after stimulation with 100 μM carbachol.

**Figure 8:** Function of TN-L15 in live primary hippocampal neurons. **A**. Primary hippocampal neuron transfected with TN-L15: **B:** Imaging trace of the neuron shown in A.

**Figure 9:** Targeting TN-L15 to the plasma membrane of live cells. A scheme of the construct is depicted. **A:** 293 cells expressing TN-L15-Ras. The arrow points at the cell whose trace is shown in **B**. **B:** TN-L15-Ras readily reports agonist-induced calcium oscillations in 293 cells. The indicator has the same dynamic range as when expressed in the cytosol. C: Primary hippocampal neuron expressing TN-L15-Ras and corresponding imaging trace (D).

**Figure 10:** Comparison of fusions of TN-L15 and Yellow Cameleon 2.3 (YC2.3) to the presynaptic protein Synaptobrevin. **A:.** Imaging trace of TN-L15-Synaptobrevin expressed in 293 cells. **B:** Imaging trace of YC2.3-Synaptobrevin.

**Figure 11:** Comparison of membrane-targeting of TnL-15 and Yellow Cameleon 2.1 (YC2.1) using the membrane targeting sequence of GAP43. **A:** Imaging trace with GAP43-TN-L15 in 293 cells. **B:** Imaging trace with GAP43-YC2.1 in 293 cells. Note the poor performance of GAP43-YC2.1.

DESCRIPTION OF THE SEQUENCES

SEQ ID NO: 1 is the DNA-sequence of TN-L15; a fusion construct of CFP, chicken skeletal muscle troponin C amino acids 15-163, and Citrine.

SEQ ID NO: 2 is the protein sequence of the construct of SEQ ID NO: 1

SEQ ID NO: 3 is the DNA-sequence of TN-humTnC; a fusion construct of CFP, human cardiac muscle troponin C, and Citrine.

SEQ ID NO: 4 is the protein sequence of the construct of SEQ ID NO: 3

SEQ ID NO: 5 is the DNA sequence of TN-L15 D107A; a fusion construct of CFP, chicken skeletal muscle troponin C amino acids 15-163, and Citrine. The third EF-hand of the troponin C is inactivated by the single amino acid exchange D107A

SEQ ID NO: 6 is the protein-sequence of the construct of SEQ ID NO: 5

SEQ ID NO: 7 is the DNA sequence of a fusion construct of CFP, chicken skeletal muscle troponin C, and Citrine

SEQ ID NO: 8 is the protein sequence of the construct of SEQ ID NO: 7

SEQ ID NO: 9 is the DNA sequence of a fusion construct of CFP, the second EF-hand of chicken skeletal muscle troponin C (amino acids 51-91), and Citrine

SEQ ID NO: 10 is the protein sequence of the construct of SEQ ID NO: 9

SEQ ID NO: 11 is the DNA sequence of a fusion construct of CFP, chicken skeletal muscle troponin C, a Gly-Gly linker, chicken skeletal muscle troponin I, and Citrine

SEQ ID NO: 12 is the protein sequence of the construct of SEQ ID NO: 11

SEQ ID NO: 13 is the DNA sequence of a fusion construct of CFP, chicken skeletal muscle troponin I amino acids 116-135, a Gly-Gly linker, chicken skeletal muscle troponin C, and Citrine

SEQ ID NO: 14 is the protein sequence of the construct of SEQ ID NO: 13

SEQ ID NO: 15 is the DNA sequence of a fusion construct of CFP, chicken skeletal muscle troponin I amino acids 95-131, a Gly-Ser-Gly linker, chicken skeletal muscle troponin C amino acids 1-91, and Citrine

SEQ ID NO: 16 is the protein sequence of the construct of SEQ ID NO: 15

SEQ ID NO: 17 is the DNA sequence of TN-L12; a fusion construct of CFP, human cardiac muscle troponin C amino acids 12-161, and Citrine

SEQ ID NO: 18 is the protein sequence of the construct of SEQ ID NO: 17

SEQ ID NO: 19 is the DNA sequence of human cardiac muscle troponin C

SEQ ID NO: 20 is the protein sequence of SEQ ID NO: 19

SEQ ID NO: 21 is the DNA sequence of human cardiac muscle troponin I

SEQ ID NO: 22 is the protein sequence of SEQ ID NO: 21

SEQ ID NO: 23 is the DNA sequence of human skeletal muscle troponin C

SEQ ID NO: 24 is the protein sequence of SEQ ID NO: 23

SEQ ID NO: 25 is the DNA sequence of chicken skeletal muscle troponin C

SEQ ID NO: 26 is the protein sequence of SEQ ID NO: 25

SEQ ID NO: 27 is the DNA sequence of chicken fast skeletal muscle troponin I

SEQ ID NO: 28 is the protein sequence of SEQ ID NO: 27

SEQ ID NO: 29 is the DNA sequence of chicken cardiac muscle troponin C

SEQ ID NO: 30 is the protein sequence of SEQ ID NO: 29

EXAMPLIFYING SECTION

The following examples are meant to further illustrate, but not limit, the invention. The examples comprise technical features and it will be appreciated that the invention relates also to combinations of the technical features presented in this exemplifying section.

EXAMPLE 1: GENE CONSTRUCTION:

Full length and truncated troponin C domains were obtained by PCR from the cDNA of chicken skeletal muscle troponin C (csTnC) using a sense primer containing an SphI site at the 5' end and a reverse primer containing a SacI site at the 3'end. Likewise, full length and truncated domains of human cardiac muscle troponin C (hcardTnC) were obtained from a cDNA sequence from which the intrinsic SacI site had to be removed first by oligonucleotid-directed mutagenesis, resulting in a silent mutation of the GluI35 codon (GAG to GAA). All troponin C DNA fragments were inserted between CFP and Citrine in the bacterial expression vector pRSETB (Invitrogen) carrying a CFP with an SphI site at the 3' end and a Citrine with a SacI site at the 5' end. A schematic representation of FRET occurring in ratiometric indicators based on troponin C variants is shown in **Fig. 1**. Calcium binding to the troponin C domain leads to a conformational change in the protein, thereby enhancing the fluorescence resonance energy transfer (FRET) from the donor to the acceptor fluorescent protein (**Fig. 1**). First constructs with simple insertion of the full-length gene yielded an indicator of moderate performance. We then tested a series of mutations and deletions at the linker regions. We went through a series of optimizations in which individual amino acids at the linking sequences close to the GFPs were exchanged or deleted. Overall, more than 70 different constructs were made, the proteins purified and tested individually for their calcium sensitivity. In addition to the full length sequences of hcardTnC and csTnC and versions thereof with truncations and modified linkers, shorter csTnC domains were engineered in which only specific structural elements of the protein were used individually such as the N-terminal regulatory lobe (amino acids 1-90, termed TnC-N90) of csTnC alone or individual EF-hands of csTnC.

A summary of basic constructs and evaluation of their function can be seen in Fig. 2. Only constructs with moderate to good performance are listed. Performance was evaluated as the maximal % change in the 527/476 nm emission ratio from zero calcium levels to calcium saturation. The best performing constructs giving more than 100 % maximal ratio change were selected for further analysis. These constructs were named TN-humTnC for an indicator using the human cardiac skeletal muscle troponin C (hcardTnC) as calcium binding moiety (SEQ. ID 3, 4), and TN-L15 for an indicator using the chicken skeletal muscle troponin C (csTnC-L15), amino acids 15-163, as calcium binding moiety (SEQ. ID 1,2).

Since TnI, like TnI from chicken, for example, the chicken fast skeletal muscle TnI isoform (csTnI) with the Swissprot Accession Number P02644, is known to form a complex with csTnC *in vivo* and some of these interactions are modified by calcium, peptide sequences of csTnI considered to be responsible for binding to the N- and C-terminal csTnC domains were selected according to the literature. csTnI fusions with csTnC were created by amplifying domains of chicken skeletal muscle TnI cDNA with sense and reverse primers containing both either an SphI site or a SacI site. The resulting csTnI DNA fragments carrying either a SphI site or a SacI site at both ends could then be cloned into the existing SphI or SacI sites in the troponin C indicator fusion constructs.

To alter calcium affinitites of single EF-hands of chicken skeletal muscle troponin C, point mutations were introduced into the gene sequence by site-directed mutagenesis using the primer extension method (QuickChange, Stratagene). For protein expression in mammalian cells, an optimized Kozak consensus sequence (GCC GCC ACC ATG G) was introduced by PCR at the 5' end of CFP; the entire indicator fragments obtained by BamHI/EcoRI restriction of the pRSETB constructs were then subcloned into the mammalian expression vector pcDNA3 (Invitrogen). Membrane targeting of indicator proteins was achieved by extending the indicator DNA sequences with a sequence encoding a membrane localization signal by PCR. In particular, the 20 amino acid sequence KLNPPDESGPGCMSCKCVLS of the c-Ha-Ras membrane-anchoring signal was fused at the 3' end of the indicator sequences, and the 20 amino acid sequence MGCCMRRTKQVEKNDEDQKI of the GAP43 membrane-anchoring signal was fused at the 5' end. See Moriyoshi K., et al., "Labeling Neural Cells Using Adenoviral Gene Transfer of Membrane-Targeted GFP." Neuron 16, 255-260 (1996).

Fusions of TN-L15 (SEQ ID No: 1) or YC3.1 to Synaptobrevin were made by amplifying Synaptobrevin by PCR, thus introducing a KpnI-Site within a GGTGGS linker to its 5'-end. Simultanously, a KpnI-site was introduced at the 3' end of csTnL-15 or YC3.1, respectively. The stop codon was thereby deleted. DNA fragments coding for thus modified Synaptobrevin and TN-L15 or YC3.1 were ligated together into an expression plasmid.

EXAMPLE 2: PROTEIN EXPRESSION, IN VITRO SPECTROSCOPY AND TITRATIONS

Proteins were expressed in bacteria using the T7 expression system in combination with the pRSETB plasmid carrying the fusion protein. Since the pRSETB plasmid also furnishes the fusion protein with an N-terminal polyhistidine tag, proteins could be purified from cleared cell lysates on nickel-chelate columns. Purified proteins were then subjected to in vitro fluorescence measurements in a Cary Eclipse fluorometer (Varian) equipped with a stopped flow RX2000 rapid kinetics accessory unit for kinetic measurements (Applied Photophysics). To obtain the percent ratio change of a protein, the fluorescence emission intensities of the FRET donor and the acceptor were measured at their respective emission maxima. Values were determined at zero calcium levels or at calcium saturation for each indicator. The Ca-free buffer contained an aliquot of the protein in 10 mM MOPS pH 7.5, 100 mM KCl, and 20 $\mu$M EGTA. In the second step, a solution of 1M $CaCl_2$ was added to the mix to a final concentration of 10 mM $CaCl_2$. The effect of calcium binding on the emission spectrum of two indicator constructs are shown in **Fig. 3**. **Fig. 3A** shows the emission spectrum of TN-L15, a fusion protein of amino acids 15-163 of chicken skeletal muscle troponin C (csTnC) as calcium binding moiety sandwiched between CFP and Citrine. Likewise, **Fig. 3B** shows the emission spectrum of TN-humTnC, a fusion protein of amino acids 1-161 of human cardiac muscle troponin C (hcardTnC) as calcium binding polypeptide sandwiched between CFP and Citrine. The emission spectra of the two constructs are depicted at zero (dashed line, - $Ca^{2+}$) and saturating (solid line, + $Ca^{2+}$) calcium levels. The change of the emission ratio upon $Ca^{2+}$ binding is 140% for TN-L15 and 120% for TN-humTnC. To obtain the percent ratio change of a protein, the fluorescence emission intensities of the FRET donor and the acceptor were measured at their respective emission maxima. Values were determined at zero calcium levels or at calcium saturation for each indicator. The Ca-free buffer contained an aliquot of the protein in 10 mM MOPS pH 7.5, 100 mM KCl, and 20 $\mu$M EGTA. In the second step, a solution of 1M $CaCl_2$ was added to the mix to a final concentration of 10 mM $CaCl_2$. The C-terminal domain of TnC is known to have two high-affinity calcium binding sites that also bind magnesium. The N-terminal lobe binds calcium specifically with a somewhat lower affinity. In agreement with this, addition of 1 mM magnesium reduced the maximal dynamic range of TN-L15 and TN-humTnC obtainable by addition of calcium from 140 % to 100 % and 120 % to 70 %, respectively.

Calcium titrations were done in a buffer system containing $Ca^{2+}$ and $K_2$EGTA in various ratios such as to obtain defined concentrations of free $Ca^{2+}$. Thus, by mixing aliquots of the indicator protein with various ratios of two buffer solutions containing either 10 mM $K_2$EGTA, 100 mM KCl and 30 mM MOPS pH 7.2, or 10 mM CaEGTA, 100 mM KCl and 30 mM MOPS pH 7.2, the fluorescence emission intensities of the FRET donor and the acceptor could be recorded at various concentrations of free calcium. Magnesium was added to the buffers when necessary. Calcium Kd values were calculated by plotting the ratio of the donor and acceptor protein's emission maximum wavelengths against the concentration of free calcium on a double logarithmic scale. See Grynkiewicz G., et al. "A New Generation of Ca2+ Indicators with Greatly Improved Fluorescence Properties." J. Biol. Chem. 260, 3440-3450 (1985). Magnesium titrations were done in 10mM MOPS pH 7.0, 100mM KCl and varying amounts of $MgCl_2$. Calcium affinities and pH-sensitivities of selected indicator proteins are depicted in **Fig. 4**. **Fig. 4A** shows the determination of calcium $K_d$ values of selected constructs by $Ca^{2+}$ titrations in the presence of 1 mM free $Mg^{2+}$. Emission ratio changes were normalized to the values at full calcium saturation, and curve fits correspond to the apparent calcium $K_d$ values given in the text. Calcium titrations resulted in response curves with apparent dissociation constants of 470 nM for TN-humcTnC (open diamonds) and 1.2 μM for TN-L15 (filled squares). $K_d$s for magnesium binding were 2.2 mM and 0.5 mM for TN-L15 and TN-humTnC, respectively. Site-directed mutagenesis has been used extensively to study ligand binding properties and conformational change within troponin C. We therefore inactivated individual EF-hands systematically by exchanging crucial aspartate or glutamate residues within the binding loops with either alanine or glutamine. The mutation D107A, by which the third, C-terminal EF-hand was inactivated within TN-L15, resulted in an indicator with reduced calcium affinity. The apparent calcium $K_d$ of this construct was determined to be 29 μM (open circles). As a consequence, the response curve in calcium titrations was significantly shifted to the right, as seen in **Fig. 4A**. Therefore, this mutant appears to be more suitable for measuring larger changes in calcium that can be encountered for example when targeting indicators to synaptic sites or in close vicinity to channels. For comparison, however, inactivating both N-terminal sites by the double mutation E42Q/E78Q yielded a protein that left only the C-terminal high-affinity components intact, resulting in a $K_d$ for calcium of 300 nM (**Fig. 4A,** filled circles). In **Fig. 4B,** we investigated to what extent pH changes affected the ratios of TN-L15 obtained at zero calcium (50 μM BAPTA, filled square, - $Ca^{2+}$) or calcium saturation (10 mM $Ca^{2+}$, filled circle, + $C^{2+}$). As expected, ratios were dependent on pH. Ratios started to drop beginning below pH 6.8 reflecting the pH-properties of Citrine and CFP. In the physiological range of cytosolic pH fluctuations between pH 6.8-7.3 the ratios were, however, remarkably stable. pH-resistance of our probes is a clear advantage over recent non-ratiometric probes based on calmodulin and a single GFP as fluorophore, as these probes are intrinsically sensitive to pH changes and therefore artefact-prone even when expressed in the cytosol.

For measurements of dissociation kinetics, 6 μM purified protein in 10 mM MOPS pH 7, 200 mM KCl, 1mM BAPTA, 1mM free $Mg^{2+}$ and 1 or 50 μM free $Ca^{2+}$ (TN-L 15 D107A: 50 μM or 300 μM free $Ca^{2+}$) were mixed with 20 mM BAPTA (TN-L15 D107A: 35 mM BAPTA) in 10 mM MOPS pH 7, 200 mM KCl and 1mM free $Mg^{2+}$; mixing dead time was 8 ms. In our experience on-rates of genetically encoded calcium probes never appeared to be a problem in experiments. However, slow dissociation rates are the main obstacle to follow fast changing signals. We therefore focused on measuring the dissociation rates of calcium bound to our indicator proteins. Samples were excited at 432 nm and emission monitored at 528 nm. Data sets from at least five experiments were averaged and rate constants derived from monoexponential curve fittings. Traces of individual dissociation experiments are shown in **Fig. 5** As expected for first order reaction kinetics, these rates were independent of the chosen calcium concentration (data not shown). The $\tau$ values obtained from the three selected constructs were 860 ms for TN-L15 (**Fig. 5**, top), 580 ms for TN-L15 D107A and 560 ms for TN-humTnC. In comparison with our proteins yellow cameleon 2.3 (YC2.3) displayed a calcium dissociation rate of 870 ms (**Fig. 5**, bottom).

EXAMPLE 3: FUNCTIONALITY OF TNC-BASED INDICATORS IN LIVE CELLS

HEK-293 cells were transfected with lipofectin reagent (Invitrogen) and imaged two to four days later on a Zeiss Axiovert 35M microscope with a CCD camera (CoolSnap, Roper Scientific). Hippocampal neurons were prepared from 17 day old rat embryos, transfected by calcium phosphate precipitation 1 week after preparation, and imaged 2 days after transfection. The imaging setup was controlled by Metafluor 4.6 software (Universal Imaging). For ratio imaging, a 440/20 excitation filter, a 455 DCLP dichroic mirror and two emission filters (485/35 for CFP, 535/25 for Citrine) operated in a filter wheel (Sutter Instruments) were used. Constructs of the indicators with optimized Kozak consensus sequences for initiation of translation were expressed. Troponin C is a part of the troponin complex and usually not expressed as an isolated protein within the cytosol. It was therefore interesting and satisfying to see that our indicators showed good cytosolic expression. Fluorescence was distributed evenly and homogenously within the cytosol with no signs of aggregation (**Fig. 6A, 7A**). The nucleus was excluded as expected for proteins with molecular weights of 69.7 and 72.5 kD, respectively for TN-L15 and TN-humTnC. In order to examine the function of the indicators inside cells we used the carbachol reponse of 293 cells that can be stimulated via muscarinic receptors. Responses of 293 cells expressing TN-L15 after stimulations with 100 μM carbachol can be seen in Fig. 6. Ratios (**Fig. 6B**) and intensity changes of the individual wavelengths (**Fig. 6C**) are depicted for two cells expressing different levels of the probe. In good agreement with the *in vitro* properties of the indicator, carbachol-induced oscillations of cellular free calcium were readily imaged, with repeated cycles of reciprocal intensity changes of CFP and Citrine. Imaging turned out to be dynamic and reproducible, and it was no problem to obtain Rmax and Rmin. TN-L15 was also functional in primary cultures of rat hippocampal neurons (**Fig. 8**). Responses to glutamate stimulation and depolarization with 100 mM KCl are seen in **Fig. 8b**. A response of HEK293 cells expressing TN-humTnC is shown in **Fig. 7B**. Maximal ratio changes within cells were 100 % for TN-L15 and 70 % for TN-humTnC, in accordance with the indicators' *in vitro* values. For comparison, the maximal ratio change obtainable with yellow cameleon 2.1 on our set-up was 70 % (data not shown).

EXAMPLE 4: SUBCELLULAR TARGETING OF TNC-BASED INDICATORS AND FUNCTIONALITY OF SUCH CONSTRUCTS

We next set out to evaluated the targeting properties of our new indicators within cells. In principle, one great potential of genetic probes is that they can be targeted to cellular organelles and microenvironments with the precision of molecular biology. Although most attractive, no functional labelings of membranes, pre- or postsynaptic structures or calcium channels have been reported previously. In our experience, these types of targetings were not functional when performed with calmodulin-based indicators (O. Griesbeck, unpublished observations and **Fig. 10, 11**). We therefore used the membrane anchor sequence of c-Ha-Ras to target TN-L15 to the membrane (**Fig. 9**). Targeting was achieved by adding the membrane anchor sequence of c-Ha-Ras to the C-terminus of TN-L15. A scheme of the construct is depicted in **Fig. 9**. When expressed in 293 cells ring-shaped labeling of the plasma membrane was evident (**Fig. 9A**). For imaging we defined small regions following the contours of the membrane. Membrane-tagged TN-L15 readily reported agonist-induced increases in cytosolic calcium and had the same dynamic range as in cytosolic expression (Fig. 9B). When expressed in hippocampal neurons, TN-L15-Ras was saturated after stimulation with high potassium, probably due to the close vicinity to calcium channels in the plasma membrane (**Fig. 9C, D**). **Fig 11** shows a comparison of membrane-targeting of TN-L15 and Yellow Cameleon 2.1 (YC2.1) using the membrane targeting sequence of GAP43. The 20 N-terminal amino acid residues of GAP43 were added in the identical manner to the N-terminus of TN-L15 or YC2.1 in order to achieve targeting to the plasma membrane. The functionality of these constructs was tested in 293 cells. A. Imaging trace with GAP43-TN-L15. Long lasting calcium oscillations after stimulation with carbachol are visible. Finally calibration with ionomycin/10 mM CaC12 and ionomycin/20 $\mu$M EGTA to obtain Rmax and Rmin verified that the indicator had its full dynamic range and full functionality when targeted to the plasma membrane. In contrast, GAP43-YC2.1 performed poorly under identical conditions as seen in **Fig. 11B**. No oscillations were detectable, and also calibration with ionomycin indicated a reduced dynamic range, suggesting that the indicator had lost significant features of its calcium binding properties on the pathway to membrane insertion. In another comparison of targeting properties we made use of fusion of TN-L15 and Yellow Cameleon 2.3 (YC2.3) to the presynaptic protein Synaptobrevin (**Fig. 10**). Fusions were done in the indentical manner for both constructs. Fusion constructs were tested for functionality in 293 cells. A. Imaging trace of TN-L15-Synaptobrevin. Good responses to stimulation with carbachol and ionomycin were readily detectable. B. Imaging trace of YC2.3-Synaptobrevin. Within the fusion construct the indicator YC2.3 had largely lost its calcium sensitivity and binding properties. No responses to carbachol stimulation were seen. Ionomycin induced a sluggish rise of the ratio over several minutes that does not reflect the actual cytosolic rise in calcium levels after ionomycin treatment. The trace shown in B is an example chosen from 9 different imaging experiments, none of which elicited a response of this probe. Altogether these results clearly demonstrate the superiority of troponin-based indicators, especially under the experimental conditions of membrane targeting.

## Claims

1. A modified Ca2+-binding polypeptide comprising

   a) a first chromophore of a donor-acceptor-pair for FRET (Fluorescence Resonance Energy Transfer),
   b) a Ca2+-binding polypeptide with an identity of at least 80% to a 30 amino acid long polypeptide sequence of human troponin C or chicken skeletal muscle troponin C, and
   c) a second chromophore of a donor-acceptor-pair for FRET.

2. The polypeptide of claim 1, wherein the first chromophore is a fluorescent polypeptide capable of serving as a donor-chromophore in a donor-acceptor-pair for FRET and the second chromophore is a fluorescent polypeptide capable of serving as an acceptor-chromophore in a donor-acceptor-pair for FRET.

3. The polypeptide of claim 2, wherein the modified polypeptide is a fusion polypeptide wherein the order of the three linked polypeptides starting from the N-terminus of the fusion polypeptide is a)-b)-c) or c)-b)-a).

4. The polypeptide of any one of claims 1 to 3, wherein the first chromophore is selected from the group consisting of CFP, EGFP, YFP, DsFP 483, AmCyan, Azami-Green and As499, particularly wherein the first chromophore is CFP.

5. The polypeptide of any one of claims 1 to 4, wherein the second chromophore is selected from the group consisting of YFP, DsRed, zFP 538, HcRed, EqFP 611 and AsFP 595, particularly wherein the second chromophore is YFP.

6. The polypeptide of any one of claims 1 to 5, wherein the Ca2+-binding polypeptide comprises at least one Ca2+-binding EF-hand.

7. The polypeptide of any one of claims 1 to 6, wherein the Ca2+-binding polypeptide comprises a polypeptide sequence having at least 60% identity to amino acids 15 to 163 of chicken skeletal muscle troponin C or amino acids 1 to 161 of human cardiac troponin C.

8. The polypeptide of any one of claims 1 to 7, further comprising glycine-rich linker peptides N-terminal or C-terminal to polypeptide b).

9. The polypeptide of any one of claims 1 to 8, further comprising a localization signal, in particular a nuclear localization sequence, a nuclear export sequence, an endoplasmic reticulum localization sequence, a peroxisome localization sequence, a mitochondrial import sequence, a mitochondrial localization sequence, a cell membrane targeting sequence, most preferably a cell membrane targeting sequence mediating localization to pre- or postsynaptic structures.

10. The polypeptide of any one of claims 1 to 9 which exhibits a ratio change upon Ca2+-addition of more than 30%, preferably from 50% to 200%, more preferably from 80% to 180% and most preferably from 100% to 150%.

11. The polypeptide of any one of claims 1 to 10 which has a Kd for Ca2+ of from 50 nM to 400 μM, preferably of from

100 nM to 100 μM and most preferably of from 250 nM to 35 μM.

**12.** The polypeptide of claim 3 selected from any one of the polypeptides of SEQ ID NO. 2, 4, 6, 8, 10, 12, 14, 16 or 18, preferably 2 or 4.

**13.** A nucleic acid molecule comprising a nucleic acid sequence encoding a polypeptide according to claims 3 to 12, preferably a nucleic acid sequence of SEQ ID NO 1 or 3.

**14.** An expression vector containing the nucleic acid molecule of claim 13, preferably further comprising expression control sequences operatively linked to a nucleic acid encoding a polypeptide according to claims 3 to 12.

**15.** A host cell, particularly a mammalian, non-human cell, inside or outside of the animal body or a human cell outside of the human body, comprising a polypeptide according to claims 3 to 12 and/or a nucleic acid according to claim 13 and/or an expression vector according to claim 14.

**16.** A transgenic animal comprising a polypeptide according to claims 3 to 12 and/or a nucleic acid according to claim 13 and/or an expression vector according to claim 14 and/or a host cell according to claim 15.

**17.** A method for the detection of changes in the local Ca2+-concentration comprising the following steps:

a) providing a cell or a subcellular membraneous fraction of a cell comprising a Ca2+-binding polypeptide according to any one of claims 1 to 12; and
b) inducing a change in the local Ca2+-concentration; and
c) measuring FRET between the donor and the acceptor chromophore of the donor-acceptor-pair of said polypeptide according to any one of claims 1 to 12, which is indicative of the change in the local Ca2+-concentration.

**18.** The method of claim 17, wherein the cell of step a) is a host cell according to claim 15.

**19.** The method of claim 17, wherein the subcellular membraneous fraction is an organelle, in particular a mitochondrium, a peroxisome or a nucleus, or a membrane fraction derived from a membrane-bound organelle, in particular derived from the cell membrane.

**20.** The method of claim 17, wherein the Ca2+-binding polypeptide is targeted to the inner surface of the cell membrane.

**21.** The method of claim 17, wherein step b) is effected by administering an extracellular stimulus, in particular by adding a small chemical compound or a polypeptide to the extracellular side of the host cell.

**22.** A method for the detection of the binding of a small chemical coumpound or a polypeptide to a Ca2+-binding polypeptide with an identity of at least 80% to a 30 amino acid long polypeptide sequence of human troponin C or chicken skeletal muscle troponin, comprising the following steps:

a) providing a Ca2+-binding polypeptide according to any one of claims 1 to 12; and
b) adding a small chemical compound to be tested for binding or a polypeptide to be tested for binding; and
c) determining the degree of binding by measuring FRET between the donor and the acceptor chromophore of the donor-acceptor-pair of said polypeptide according to any one of claims 1 to 12.

**23.** The method of claim 22, wherein the Ca2+-binding polypeptide is derived from human troponin C, and particularly is SEQ ID NO. 4.

**24.** *Ex vivo* use of a polypeptide according to any one of claims 1 to 12 for the detection of changes in the local Ca2+-concentration close to a cellular membrane.

**25.** The use of claim 24, wherein the polypeptide is a polypeptide according to claim 9 and particularly comprises a cell membrane targeting sequence, most preferably a cell membrane targeting sequence mediating localization to the cell membrane of pre- or postsynaptic structures.

**26.** Use of a polypeptide according to any one of claims 1 to 12 for the preparation of a diagnostic composition for the detection of changes in the local Ca2+-concentration close to a cellular membrane.

**Patentansprüche**

1. Verändertes Ca2+-bindendes Polypeptid umfassend

   a) ein erstes Chromophor eines Donor-Akzeptor-Paares zum FRET (Fluoreszenz-Resonanz-Energie-Transfer),
   b) ein Ca2+-bindendes Polypeptid mit einer Identität von mindestens 80 % zu einer 30 Aminosäuren langen Polypeptidsequenz von menschlichem Troponin C oder Skelettmuskel-Troponin C aus Huhn, und
   c) ein zweites Chromophor eines Donor-Akzeptor-Paares zum FRET.

2. Polypeptid nach Anspruch 1, wobei das erste Chromophor ein fluoreszierendes Polypeptid ist, das die Fähigkeit besitzt, als ein Donor-Chromophor in einem Donor-Akzeptor-Paar zum FRET zu dienen, und das zweite Chromophor ein fluoreszierendes Polypeptid ist, das die Fähigkeit besitzt, als ein Akzeptor-Chromophor in einem Donor-Akzeptor-Paar zum FRET zu dienen.

3. Polypeptid nach Anspruch 2, wobei das veränderte Polypeptid ein Fusions-Polypeptid ist, wobei die Anordnung der drei verbundenen Polypeptide beginnend vom N-Terminus des Fusionspolypeptids a)-b)-c) oder c)-b)-a) ist.

4. Polypeptid nach einem beliebigen der Ansprüche 1 bis 3, wobei das erste Chromophor ausgewählt ist aus der Gruppe bestehend aus CFP, EGFP, YFP, DsFP 483, AmCyan, Azami-Green und As499, insbesondere wobei das erste Chromophor CFP ist.

5. Polypeptid nach einem beliebigen der Ansprüche 1 bis 4, wobei das zweite Chromophor ausgewählt ist aus der Gruppe bestehend aus YFP, DsRed, zFP 538, HcRed, EqFP 611 und AsFP 595, insbesondere wobei das zweite Chromophor YFP ist.

6. Polypeptid nach einem beliebigen der Ansprüche 1 bis 5, wobei das Ca2+-bindende Polypeptid mindestens eine Ca2+-bindende EF-Hand umfasst.

7. Polypeptid nach einem beliebigen der Ansprüche 1 bis 6, wobei das Ca2+-bindende Polypeptid eine Polypeptidsequenz umfasst, die mindestens 60 % Identität zu den Aminosäuren 15 bis 163 von Skelettmuskel Troponin C aus Huhn oder Aminosäuren 1 bis 161 von humanem Herz-Troponin C besitzt.

8. Polypeptid nach einem beliebigen der Ansprüche 1 bis 7, weiterhin umfassend Glycin-reiche Linkerpeptide N-terminal oder C-terminal zum Polypeptid b).

9. Polypeptid nach einem beliebigen der Ansprüche 1 bis 8, weiterhin umfassend ein Lokalisationssignal, insbesondere eine Kern-Lokalisationssequenz, eine Kem-Exportsequenz, eine Lokalisationssequenz für das endoplasmatische Reticulum, eine Peroxisomen-Lokalisationssequenz, eine mitochondriale Importsequenz, eine mitochondriale Lokalisationssequenz, eine Sequenz zum Zielrichten an die Zellmembran, am meisten bevorzugt eine Sequenz zum Zielrichten an die Zellmembran, die eine Lokalisation an prä- oder postsynaptische Strukturen vermittelt.

10. Polypeptid nach einem beliebigen der Ansprüche 1 bis 9, das eine Verhältnisänderung (ratio change) bei Zugabe von Ca2+ von mehr als 30 %, bevorzugt von 50 % bis 200 %, weiter bevorzugt von 80 % bis 180 %, und am meisten bevorzugt von 100 % bis 150 % aufweist.

11. Polypeptid nach einem beliebigen der Ansprüche 1 bis 10, das einen Kd für Ca2+ von 50 nM bis 400 μM, bevorzugt von 100 nM bis 100 μM und am meisten bevorzugt von 250 nM bis 35 μM besitzt.

12. Polypeptid nach Anspruch 3, ausgewählt aus einem beliebigen der Polypeptide von SEQ ID Nr. 2, 4, 6, 8, 10, 12, 14, 16 oder 18, bevorzugt 2 oder 4.

13. Nukleinsäuremolekül umfassend eine Nukleinsäuresequenz, die ein Polypeptid nach Ansprüchen 3 bis 12 kodiert, bevorzugt eine Nukleinsäuresequenz gemäß SEQ ID Nr. 1 oder 3.

14. Expressionsvektor enthaltend das Nukleinsäuremolekül nach Anspruch 13, bevorzugt weiterhin umfassend Expressions-Kontrollsequenzen, die mit einer Nukleinsäure funktionell verbunden sind, die ein Polypeptid nach Ansprüchen 3 bis 12 kodiert.

**15.** Wirtszelle, insbesondere eine Säuger-, nicht-humane Zelle, innerhalb oder außerhalb des tierischen Körpers oder eine humane Zelle außerhalb des menschlichen Körpers, umfassend ein Polypeptid nach Ansprüchen 3 bis 12 und/oder eine Nukleinsäure nach Anspruch 13 und/oder einen Expressionsvektor nach Anspruch 14.

**16.** Transgenes Tier umfassend ein Polypeptid nach Ansprüchen 3 bis 12 und/oder eine Nukleinsäure nach Anspruch 13 und/oder einen Expressionsvektor nach Anspruch 14 und/oder eine Wirtszelle nach Anspruch 15.

**17.** Verfahren zum Nachweis von Veränderungen der lokalen Ca2+-Konzentration, umfassend die folgenden Schritte:

a) Bereitstellen einer Zelle oder einer subzellulären membranhaltigen Fraktion einer Zelle, umfassend ein Ca2+-bindendes Polypeptid nach einem beliebigen der Ansprüche 1 bis 12; und
b) Hervorrufen einer Veränderung der lokalen Ca2+-Konzentration; und
c) Messen von FRET zwischen dem Donor- und dem Akzeptor-Chromophor des Donor-Akzeptor-Paares des Polypeptids nach einem beliebigen der Ansprüche 1 bis 12, was eine Veränderung der lokalen Ca2+-Konzentration anzeigt.

**18.** Verfahren nach Anspruch 17, wobei die Zelle von Schritt a) eine Wirtszelle nach Anspruch 15 ist.

**19.** Verfahren nach Anspruch 17, wobei die subzelluläre membranhaltige Fraktion eine Organelle, insbesondere ein Mitochondrium, ein Peroxisom oder ein Kern, oder eine Membranfraktion abgeleitet aus einer membrangebundenen Organelle, insbesondere abgeleitet aus der Zellmembran, ist.

**20.** Verfahren nach Anspruch 17, wobei das Ca2+-bindende Polypeptid an die innere Oberfläche der Zellmembran zielgerichtet wird.

**21.** Verfahren nach Anspruch 17, wobei Schritt b) durch Verabreichen eines extrazellulären Stimulus ausgeführt wird, insbesondere durch Zufügen einer kleinen chemischen Substanz oder eines Polypeptids an die extrazelluläre Seite der Wirtszelle.

**22.** Verfahren zum Nachweis des Bindens einer kleinen chemischen Substanz oder eines Polypeptids an ein Ca2+-bindendes Polypeptid mit einer Identität von mindestens 80 % zu einer 30 Aminosäuren langen Polypeptidsequenz von humanem Troponin C oder Skelettmuskel-Troponin aus Huhn, umfassend die folgenden Schritte:

a) Bereitstellen eines Ca2+-bindenden Polypeptids nach einem beliebigen der Ansprüche 1 bis 12; und
b) Zufügen einer kleinen chemischen Substanz, die auf Binden getestet werden soll, oder eines Polypeptids, das auf Binden getestet werden soll; und
c) Bestimmen des Grades des Bindens durch Messen von FRET zwischen dem Donor- und dem Akzeptor-Chromophor des Donor-Akzeptor-Paares des Polypeptids nach einem beliebigen der Ansprüche 1 bis 12.

**23.** Verfahren nach Anspruch 22, wobei das Ca2+-bindende Polypeptid von humanem Troponin C abgeleitet ist, und insbesondere SEQ ID Nr. 4 ist.

**24.** Ex vivo-Verwendung eines Polypeptids nach einem beliebigen der Ansprüche 1 bis 12 zum Nachweis von Veränderungen der lokalen Ca2+-Konzentration nahe einer Zellmembran.

**25.** Verwendung nach Anspruch 24, wobei das Polypeptid ein Polypeptid gemäß Anspruch 9 ist und insbesondere eine Sequenz zum Zielrichten an die Zellmembran umfasst, am meisten bevorzugt eine Sequenz zum Zielrichten an die Zellmembran, die eine Lokalisation an die Zellmembran von prä- oder postsynaptischen Strukturen vermittelt.

**26.** Verwendung eines Polypeptids nach einem beliebigen der Ansprüche 1 bis 12 zur Zubereitung einer diagnostischen Zusammensetzung zum Nachweis von Veränderungen der lokalen Ca2+-Konzentration nahe einer Zellmembran.

## Revendications

**1.** Polypeptide fixant le $Ca^{2+}$ modifié comprenant

a) un premier chromophore d'une paire donneur-accepteur pour FRET (transfert d'énergie par résonance de fluorescence),
b) un polypeptide fixant le $Ca^{2+}$ avec une identité d'au moins 80 % à une séquence polypeptidique d'une longueur de 30 acides aminés de troponine C humaine ou de troponine C de muscle squelettique de poulet, et
c) un second chromophore d'une paire donneur-accepteur pour FRET.

**2.** Polypeptide selon la revendication 1, dans lequel le premier chromophore est un polypeptide fluorescent capable de servir de chromophore donneur dans une paire donneur-accepteur pour FRET et le second chromophore est un polypeptide fluorescent capable de servir de chromophore accepteur dans une paire donneur-accepteur pour FRET.

**3.** Polypeptide selon la revendication 2, le polypeptide modifié étant un polypeptide de fusion dans lequel l'ordre des trois polypeptides liés en partant de l'extrémité N-terminale du polypeptide de fusion est a)-b)-c) ou c)-b)-a).

**4.** Polypeptide selon l'une quelconque des revendications 1 à 3, dans lequel le premier chromophore est choisi dans le groupe constitué par CFP, EGFP, YFP, DsFP 483, AmCyan, Azami-Green et As499, en particulier dans lequel le premier chromophore est CFP.

**5.** Polypeptide selon l'une quelconque des revendications 1 à 4, dans lequel le second chromophore est choisi dans le groupe constitué par YFP, DsRed, zFP 538, HcRed, EqFP 611 et AsFP 595, en particulier dans lequel le second chromophore est YFP.

**6.** Polypeptide selon l'une quelconque des revendications 1 à 5, dans lequel le polypeptide fixant le $Ca^{2+}$ comprend au moins une main EF fixant le $Ca^{2+}$.

**7.** Polypeptide selon l'une quelconque des revendications 1 à 6, le polypeptide fixant le $Ca^{2+}$ comprenant une séquence polypeptidique ayant une identité d'au moins 60 % aux acides aminés 15 à 163 de troponine C de muscle squelettique de poulet ou aux acides aminés 1 à 161 de troponine C cardiaque humaine.

**8.** Polypeptide selon l'une quelconque des revendications 1 à 7, comprenant en outre des peptides de liaison riches en glycine N-terminaux ou C-terminaux par rapport au polypeptide b).

**9.** Polypeptide selon l'une quelconque des revendications 1 à 8, comprenant en outre un signal de localisation, en particulier un signal de localisation nucléaire, une séquence d'exportation nucléaire, une séquence de localisation dans le réticulum endoplasmique, une séquence de localisation dans le peroxysome, une séquence d'importation mitochondriale, une séquence de localisation mitochondriale, une séquence de ciblage de la membrane cellulaire, plus préférablement une séquence de ciblage de la membrane cellulaire médiant une localisation dans des structures pré- ou post-synaptiques.

**10.** Polypeptide selon l'une quelconque des revendications 1 à 9 qui présente un changement de rapport lors de l'addition de $Ca^{2+}$ de plus de 30 %, de préférence de 50 % à 200 %, plus préférablement de 80 % à 180 %, et encore plus préférablement de 100 % à 150 %.

**11.** Polypeptide selon l'une quelconque des revendications 1 à 10 qui a un Kd pour le $Ca^{2+}$ allant de 50 nM à 400 $\mu$M, de préférence de 100 nM à 100 $\mu$M et plus préférablement de 250 nM à 35 $\mu$M.

**12.** Polypeptide selon la revendication 3 choisi parmi tous les polypeptides de SEQ ID NO. : 2, 4, 6, 8, 10, 12, 14, 16 et 18, de préférence 2 ou 4.

**13.** Molécule d'acide nucléique comprenant une séquence d'acide nucléique codant pour un polypeptide selon les revendications 3 à 12, de préférence une séquence d'acide nucléique de SEQ ID NO. : 1 ou 3.

**14.** Vecteur d'expression contenant la molécule d'acide nucléique selon la revendication 13, de préférence comprenant en outre des séquences de contrôle de l'expression fonctionnellement liées à un acide nucléique codant pour un polypeptide selon les revendications 3 à 12.

**15.** Cellule hôte, en particulier cellule de mammifère non humaine à l'intérieur ou l'extérieur du corps de l'animal, ou cellule humaine à l'extérieur du corps humain, comprenant un polypeptide selon les revendications 3 à 12 et/ou un acide nucléique selon la revendication 13 et/ou un vecteur d'expression selon la revendication 14.

**16.** Animal transgénique comprenant un polypeptide selon les revendications 3 à 12 et/ou un acide nucléique selon la revendication 13 et/ou un vecteur d'expression selon la revendication 14 et/ou une cellule hôte selon la revendication 15.

**17.** Procédé de détection de changements de la concentration locale de $Ca^{2+}$ comprenant les étapes suivantes :

a) fournir une cellule ou une fraction membranaire subcellulaire d'une cellule comprenant un polypeptide fixant le $Ca^{2+}$ selon l'une quelconque des revendications 1 à 12 ;
b) induire un changement de la concentration locale de $Ca^{2+}$ ; et
c) mesurer le FRET entre les chromophores donneur et accepteur de la paire donneur-accepteur dudit polypeptide selon l'une quelconque des revendications 1 à 12, qui est indicateur du changement de la concentration locale de $Ca^{2+}$.

**18.** Procédé selon la revendication 17, dans lequel la cellule de l'étape a) est une cellule hôte selon la revendication 15.

**19.** Procédé selon la revendication 17, dans lequel la fraction membranaire subcellulaire est un organite, en particulier une mitochondrie, un peroxysome ou un noyau, ou une fraction membranaire dérivée d'un organite lié à la membrane, en particulier dérivé de la membrane cellulaire.

**20.** Procédé selon la revendication 17, dans lequel le polypeptide fixant le $Ca^{2+}$ est ciblé vers la surface interne de la membrane cellulaire.

**21.** Procédé selon la revendication 17, dans lequel l'étape b) est effectuée en administrant un stimulus extracellulaire, en particulier en ajoutant un petit composé chimique ou un polypeptide à la face extracellulaire de la cellule hôte.

**22.** Procédé de détection de la liaison d'un petit composé chimique ou d'un polypeptide à un polypeptide fixant le $Ca^{2+}$ avec une identité d'au moins 80 % à une séquence polypeptidique d'une longueur de 30 acides aminés de troponine C humaine ou de troponine de muscle squelettique de poulet, comprenant les étapes suivantes :

a) fournir un polypeptide fixant le $Ca^{2+}$ selon l'une quelconque des revendications 1 à 12 ;
b) ajouter un petit composé chimique pour lequel on veut tester la liaison ou un polypeptide pour lequel on veut tester la liaison; et
c) déterminer le degré de liaison en mesurant le FRET entre les chromophores donneur et accepteur de la paire donneur-accepteur dudit polypeptide selon l'une quelconque des revendications 1 à 12.

**23.** Procédé selon la revendication 22, dans lequel le polypeptide fixant le $Ca^{2+}$ est dérivé de troponine C humaine, et en particulier est SEQ ID N° : 4.

**24.** Utilisation *ex vivo* d'un polypeptide selon l'une quelconque des revendications 1 à 12 pour la détection de changements de la concentration locale de $Ca^{2+}$ près d'une membrane cellulaire.

**25.** Utilisation selon la revendication 24, dans laquelle le polypeptide est un polypeptide selon la revendication 9 et comprend en particulier une séquence de ciblage de la membrane cellulaire, plus préférablement une séquence de ciblage de la membrane cellulaire médiant une localisation au niveau de la membrane cellulaire de structures pré- ou post-synaptiques.

**26.** Utilisation d'un polypeptide selon l'une quelconque des revendications 1 à 12 pour la préparation d'une composition diagnostique destinée à la détection de changements de la concentration locale de $Ca^{2+}$ près d'une membrane cellulaire.

Fig. 1

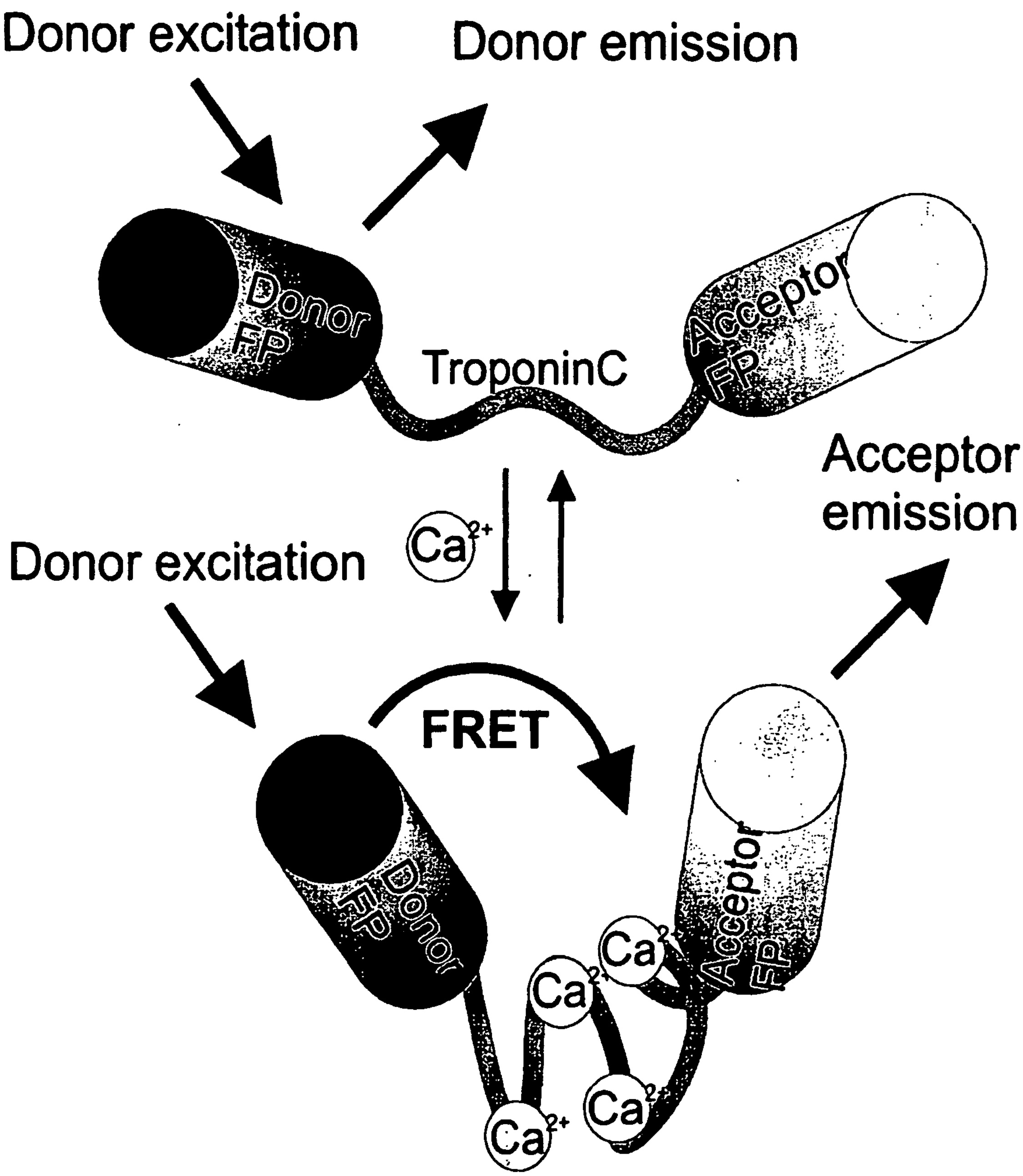
Donor excitation
Donor emission
Donor FP
TroponinC
Acceptor FP
Ca2+
Acceptor emission
Donor excitation
FRET
Donor FP
Acceptor FP
Ca2+
Ca2+
Ca2+
Ca2+

Fig. 2

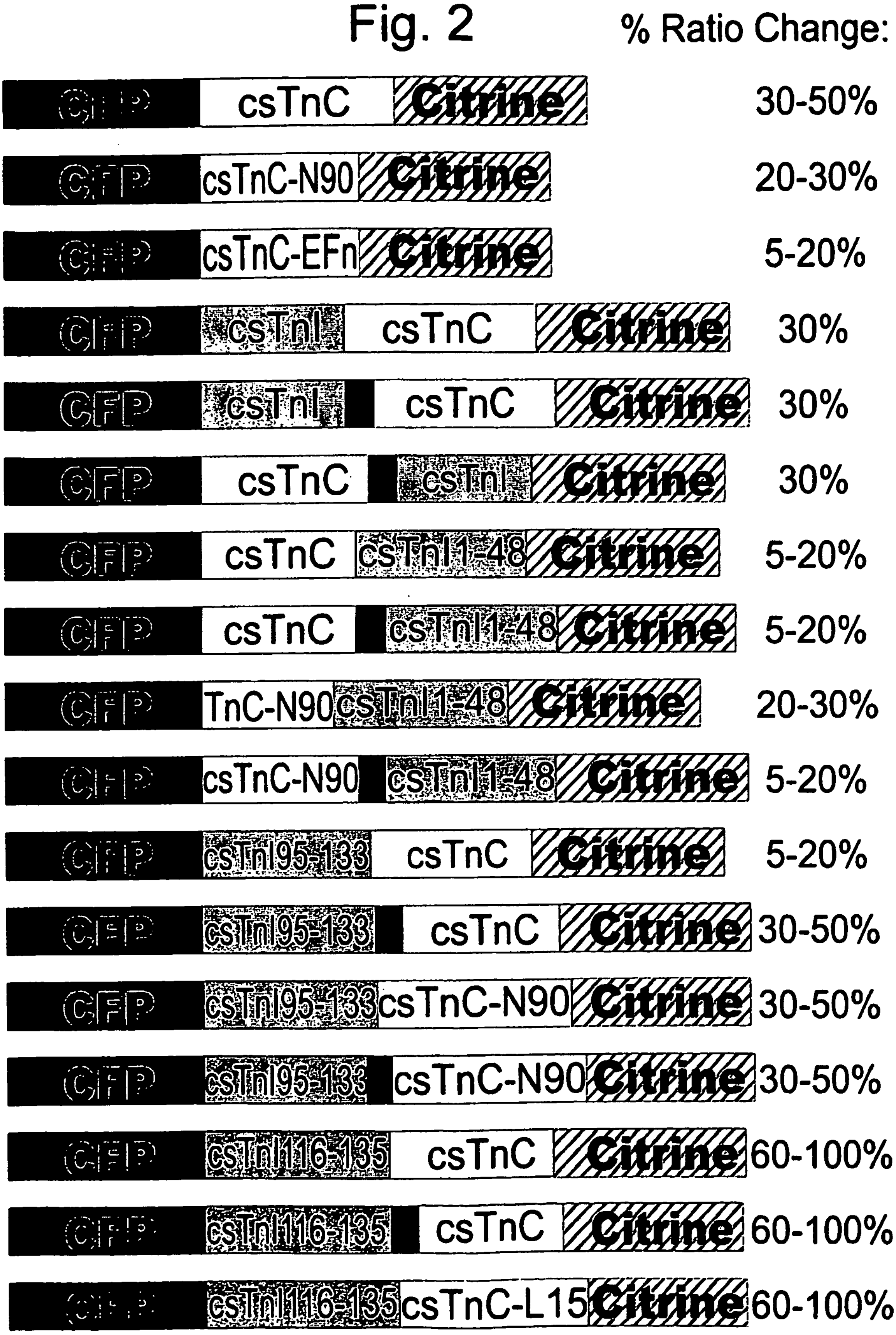
% Ratio Change:
CFP csTnC Citrine 30-50%
CFP csTnC-N90 Citrine 20-30%
CFP csTnC-EFn Citrine 5-20%
CFP csTnI csTnC Citrine 30%
CFP csTnI csTnC Citrine 30%
CFP csTnC csTnI Citrine 30%
CFP csTnC csTnI1-48 Citrine 5-20%
CFP csTnC csTnI1-48 Citrine 5-20%
CFP TnC-N90 csTnI1-48 Citrine 20-30%
CFP csTnC-N90 csTnI1-48 Citrine 5-20%
CFP csTnI95-133 csTnC Citrine 5-20%
CFP csTnI95-133 csTnC Citrine 30-50%
CFP csTnI95-133 csTnC-N90 Citrine 30-50%
CFP csTnI95-133 csTnC-N90 Citrine 30-50%
CFP csTnI116-135 csTnC Citrine 60-100%
CFP csTnI116-135 csTnC Citrine 60-100%
CFP csTnI116-135 csTnC-L15 Citrine 60-100%

Fig. 2 (continued)

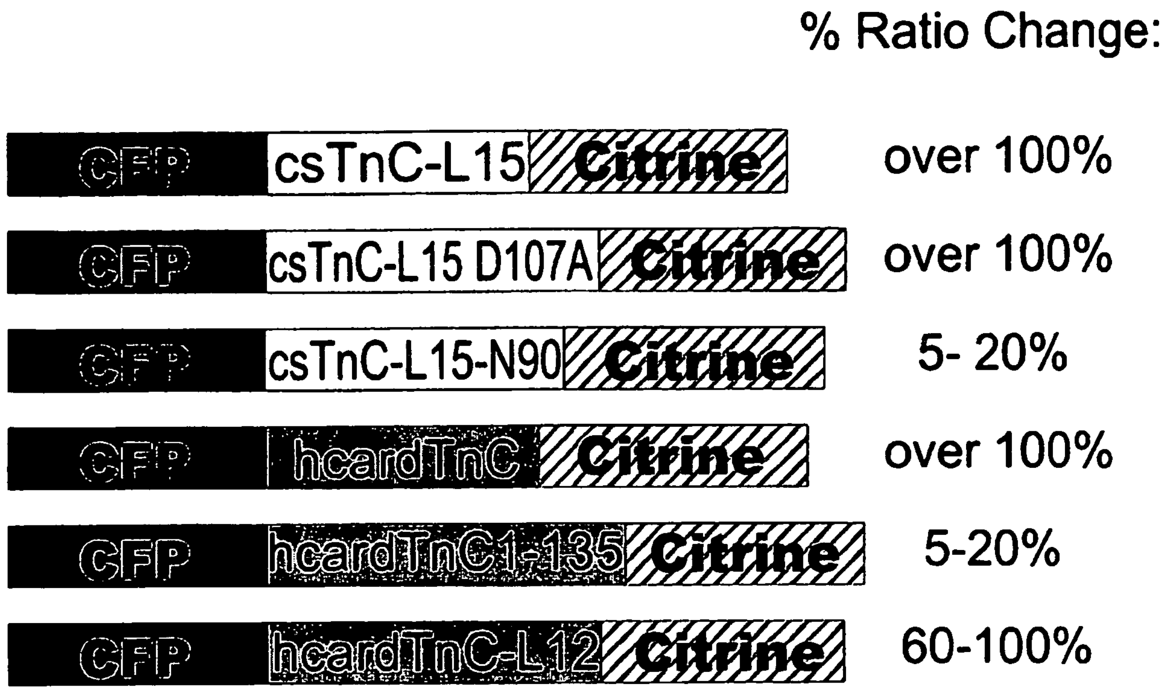

Fig. 3

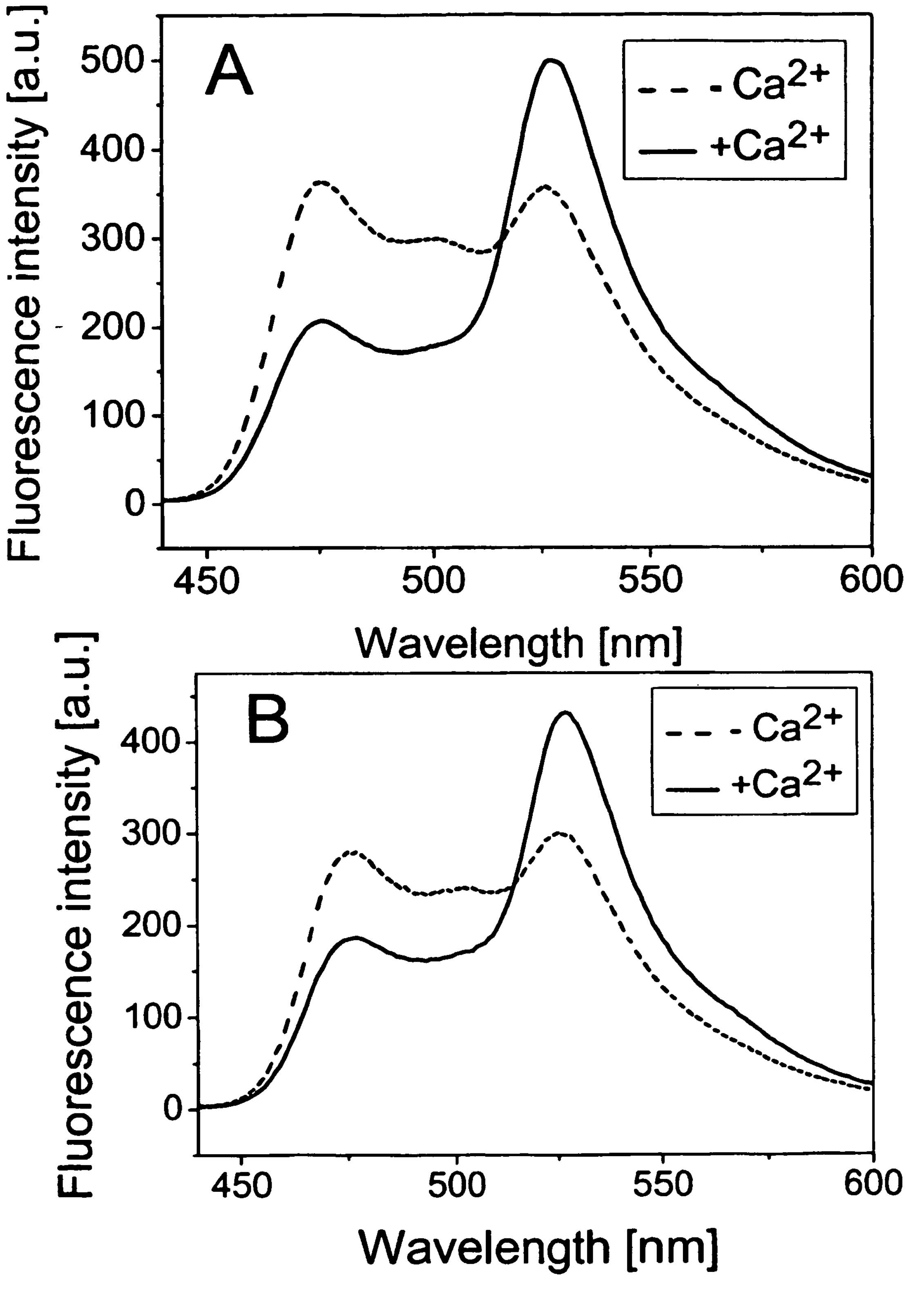
A
- - - - Ca2+
—— +Ca2+
Fluorescence intensity [a.u.]
0
100
200
300
400
500
450
500
550
600
Wavelength [nm]
B
- - - - Ca2+
—— +Ca2+
Fluorescence intensity [a.u.]
0
100
200
300
400
450
500
550
600
Wavelength [nm]

Fig. 4

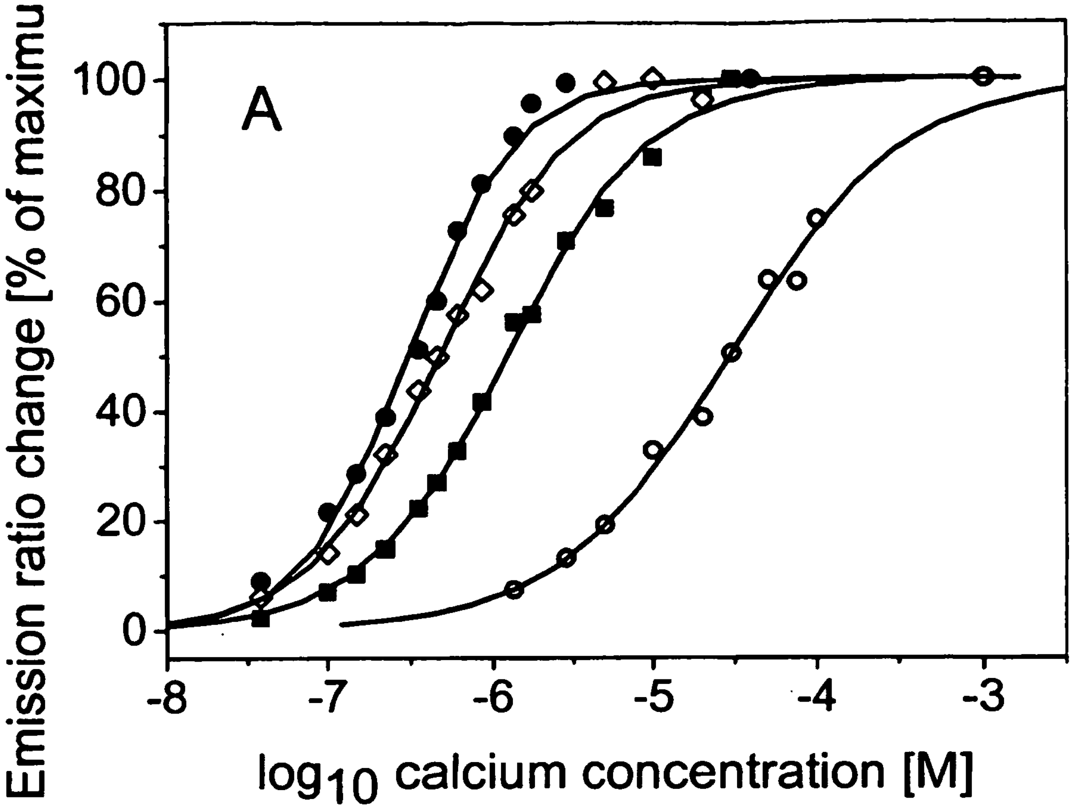
A
Emission ratio change [% of maximum]
100
80
60
40
20
0
-8
-7
-6
-5
-4
-3
log10 calcium concentration [M]

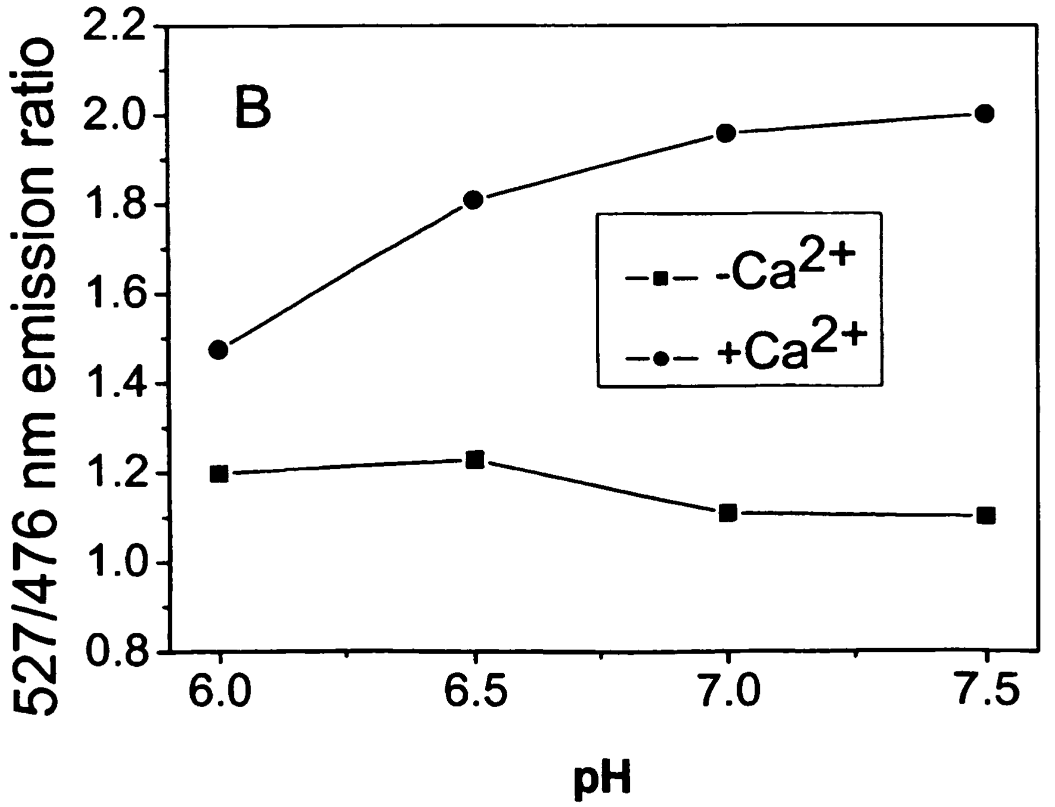
B
527/476 nm emission ratio
2.2
2.0
1.8
1.6
1.4
1.2
1.0
0.8
6.0
6.5
7.0
7.5
pH
-Ca2+
+Ca2+

Fig. 5

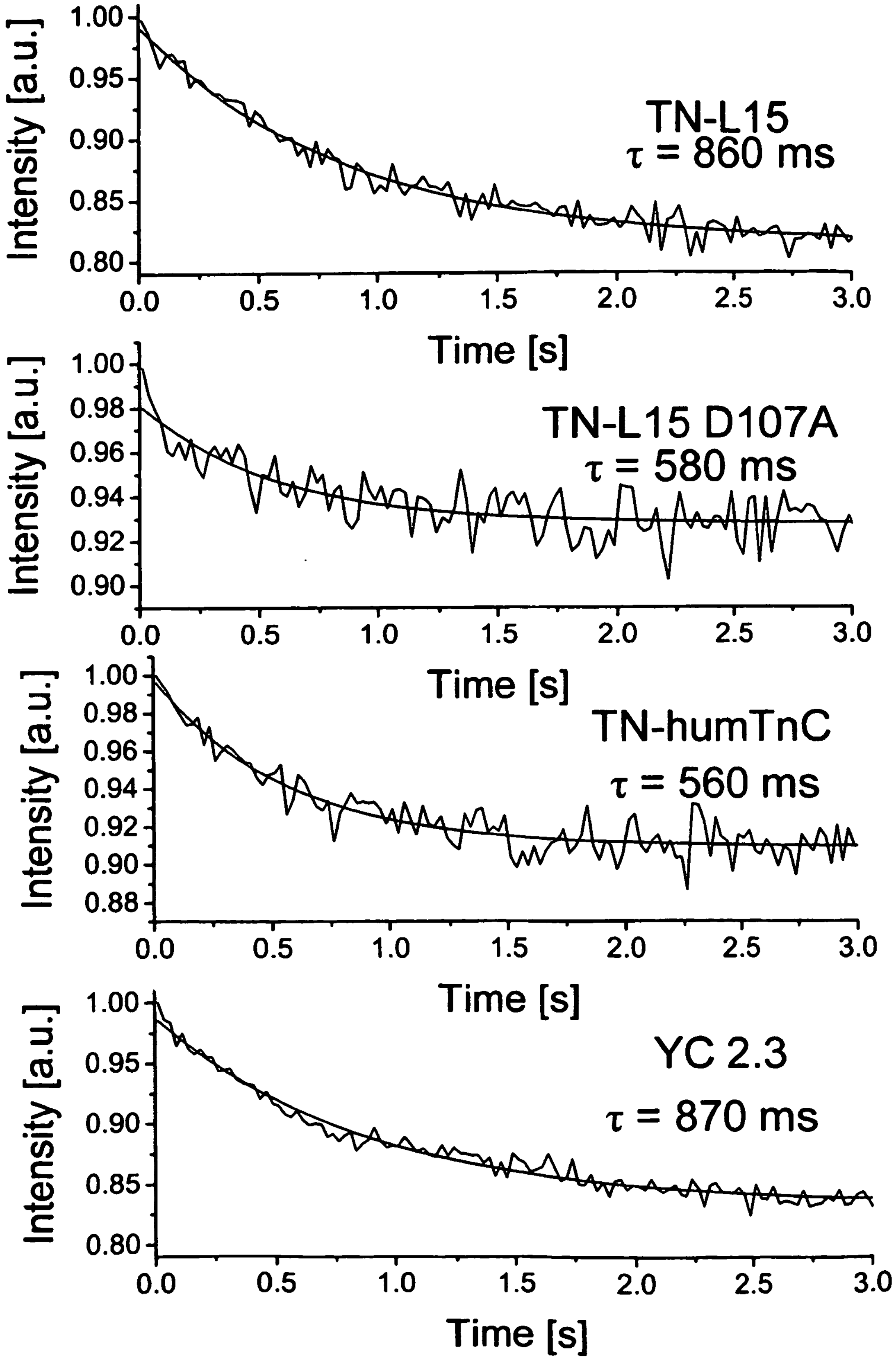
Intensity [a.u.]
Time [s]
TN-L15
τ = 860 ms
TN-L15 D107A
τ = 580 ms
TN-humTnC
τ = 560 ms
YC 2.3
τ = 870 ms

Fig. 6

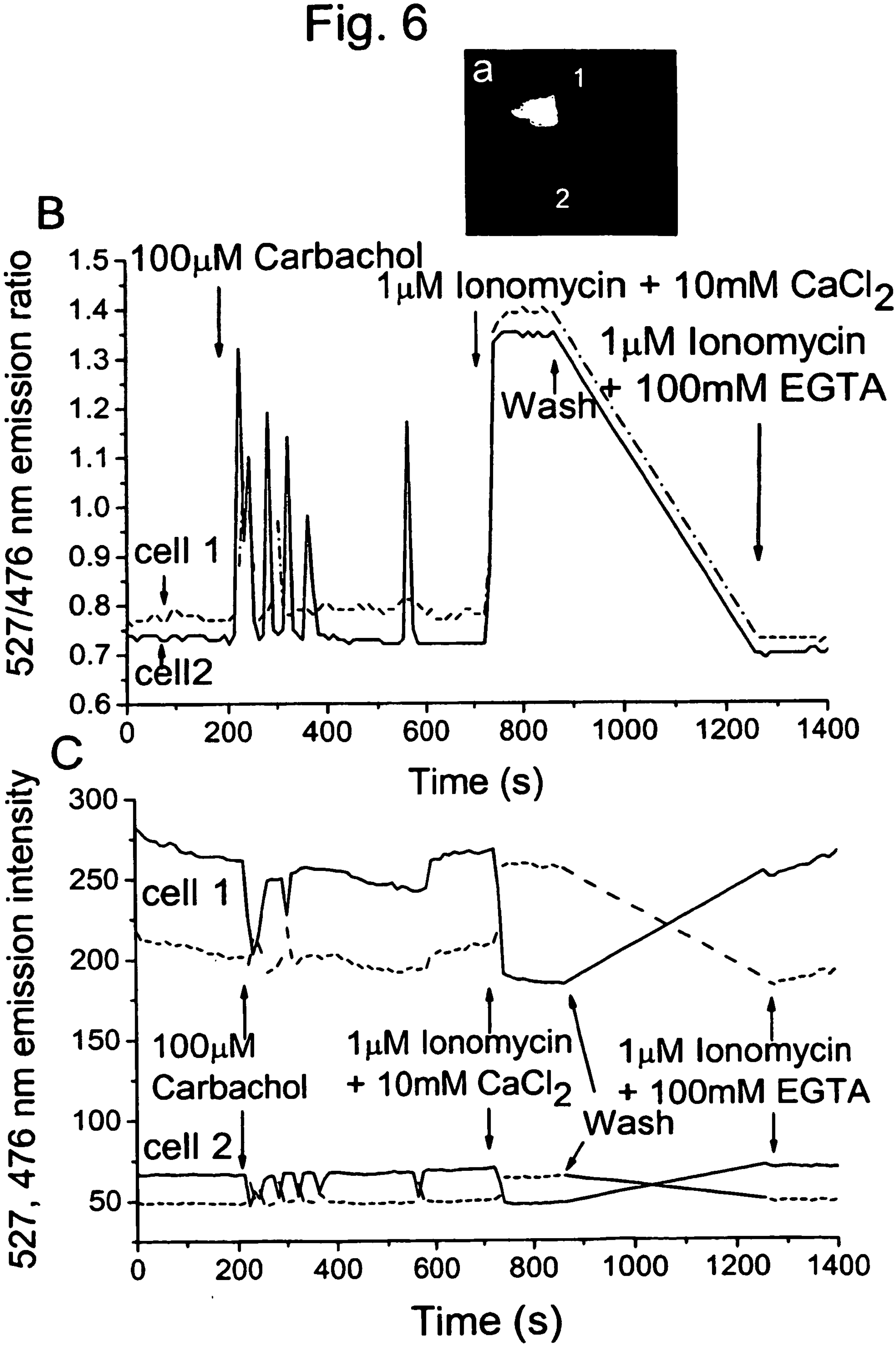
a
1
2
B
527/476 nm emission ratio
100μM Carbachol
1μM Ionomycin + 10mM CaCl2
1μM Ionomycin
+ 100mM EGTA
Wash
cell 1
cell2
Time (s)
C
527, 476 nm emission intensity
cell 1
cell 2
100μM
Carbachol
1μM Ionomycin
+ 10mM CaCl2
Wash
1μM Ionomycin
+ 100mM EGTA
Time (s)

Fig. 7

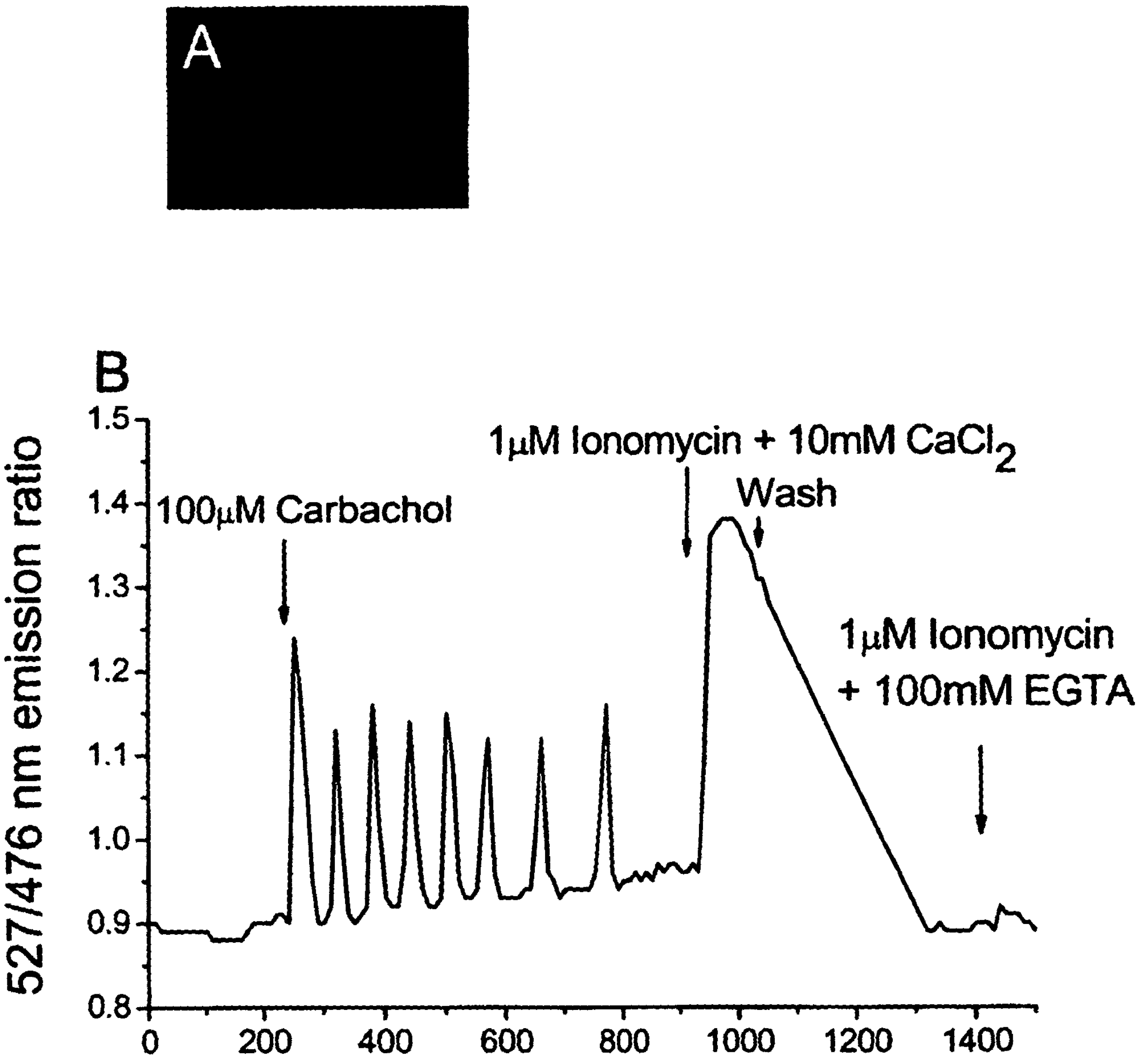
A
B
527/476 nm emission ratio
1.5
1.4
1.3
1.2
1.1
1.0
0.9
0.8
100μM Carbachol
1μM Ionomycin + 10mM CaCl2
Wash
1μM Ionomycin
+ 100mM EGTA
0
200
400
600
800
1000
1200
1400
Time (s)

Fig. 8

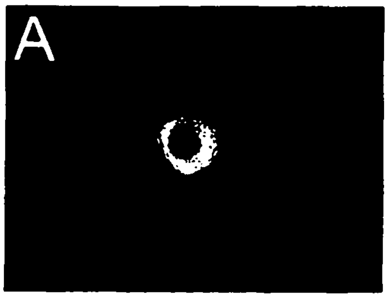
A

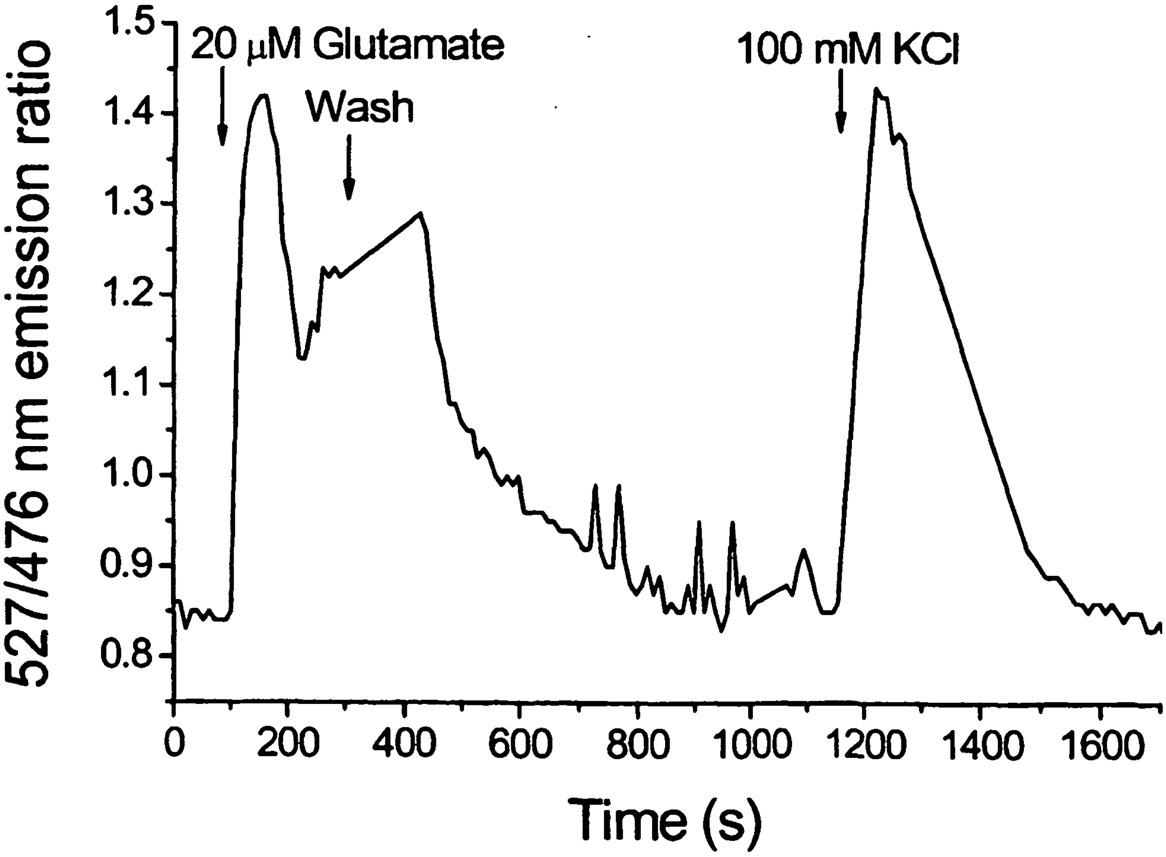
20 μM Glutamate
Wash
100 mM KCl
527/476 nm emission ratio
1.5
1.4
1.3
1.2
1.1
1.0
0.9
0.8
0
200
400
600
800
1000
1200
1400
1600
Time (s)

Fig. 9

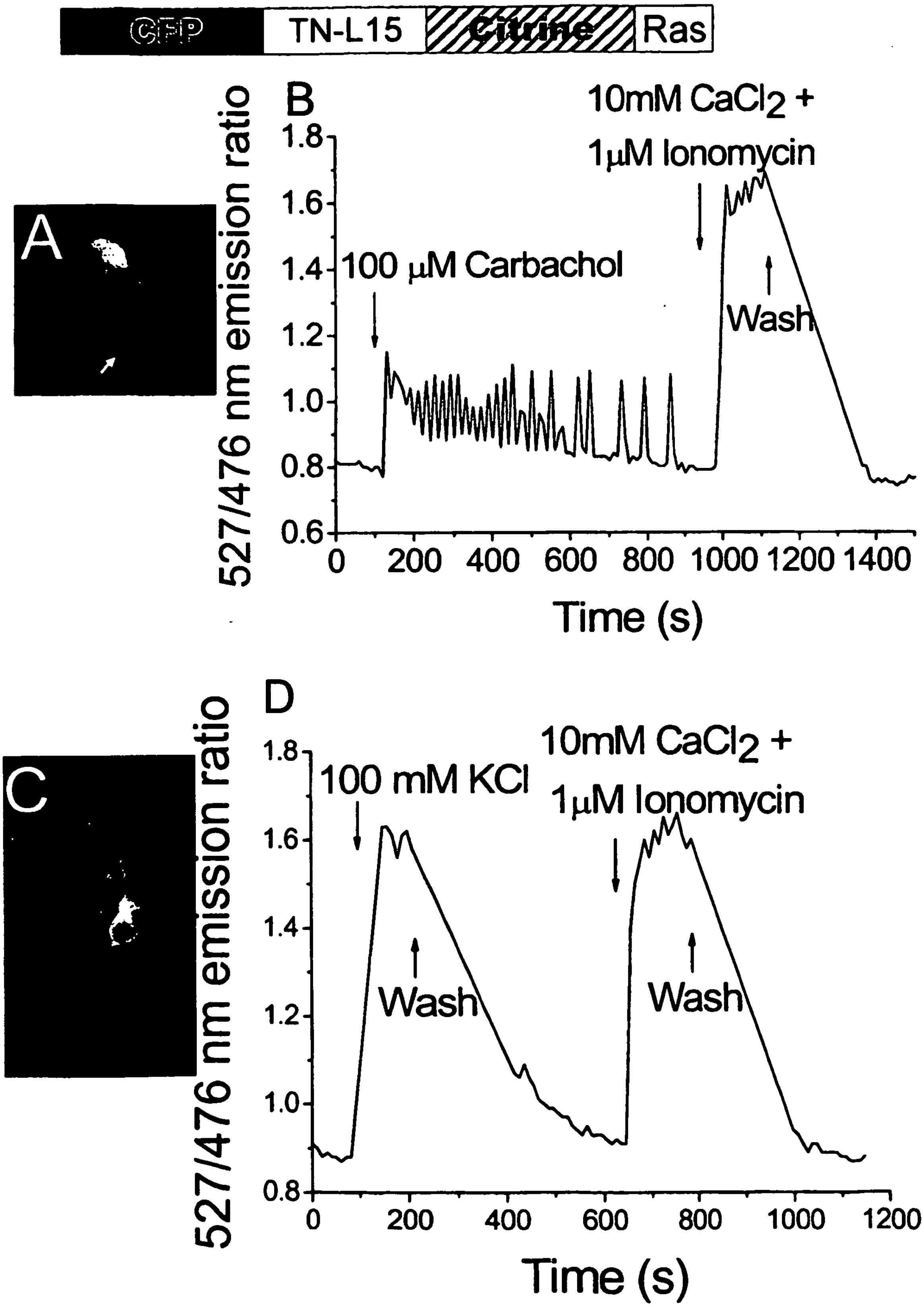
CFP
TN-L15
Citrine
Ras
A
B
10mM $CaCl_2$ +
1μM Ionomycin
100 μM Carbachol
Wash
527/476 nm emission ratio
1.8
1.6
1.4
1.2
1.0
0.8
0.6
0
200
400
600
800
1000
1200
1400
Time (s)
C
D
100 mM KCl
10mM $CaCl_2$ +
1μM Ionomycin
Wash
Wash
527/476 nm emission ratio
1.8
1.6
1.4
1.2
1.0
0.8
0
200
400
600
800
1000
1200
Time (s)

Fig. 10

A

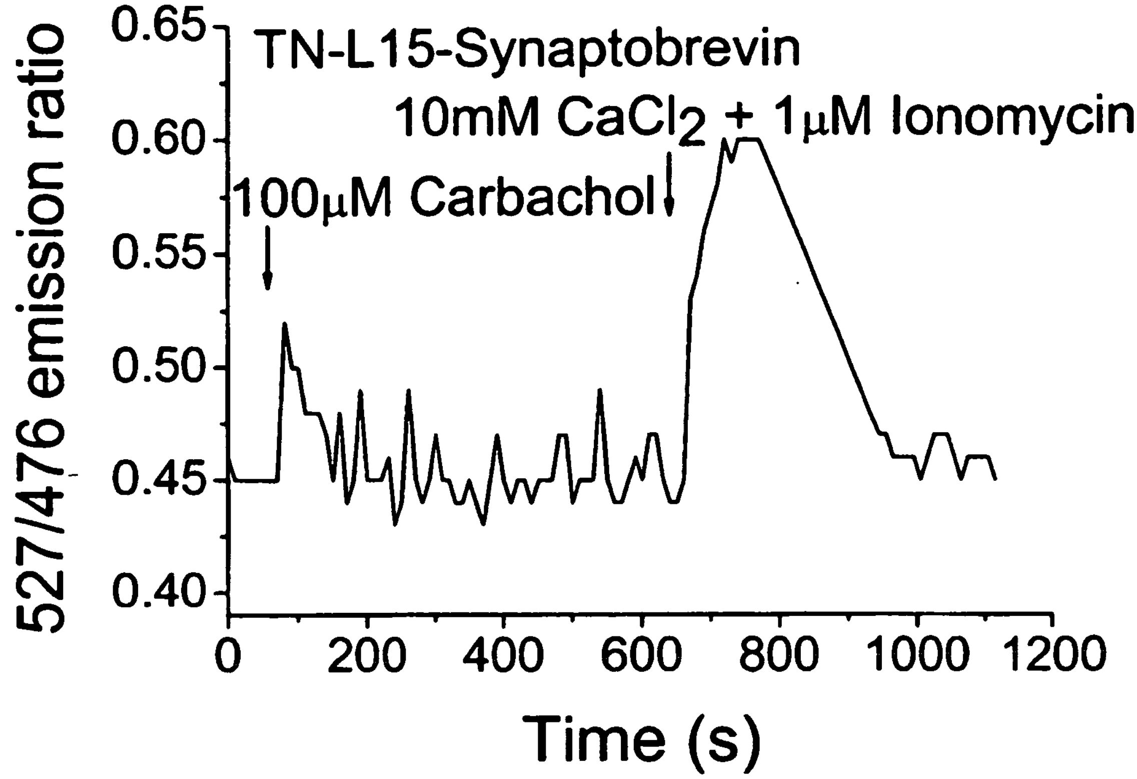
TN-L15-Synaptobrevin
10mM CaCl2 + 1μM Ionomycin
100μM Carbachol
527/476 emission ratio
0.65
0.60
0.55
0.50
0.45
0.40
0
200
400
600
800
1000
1200
Time (s)

B

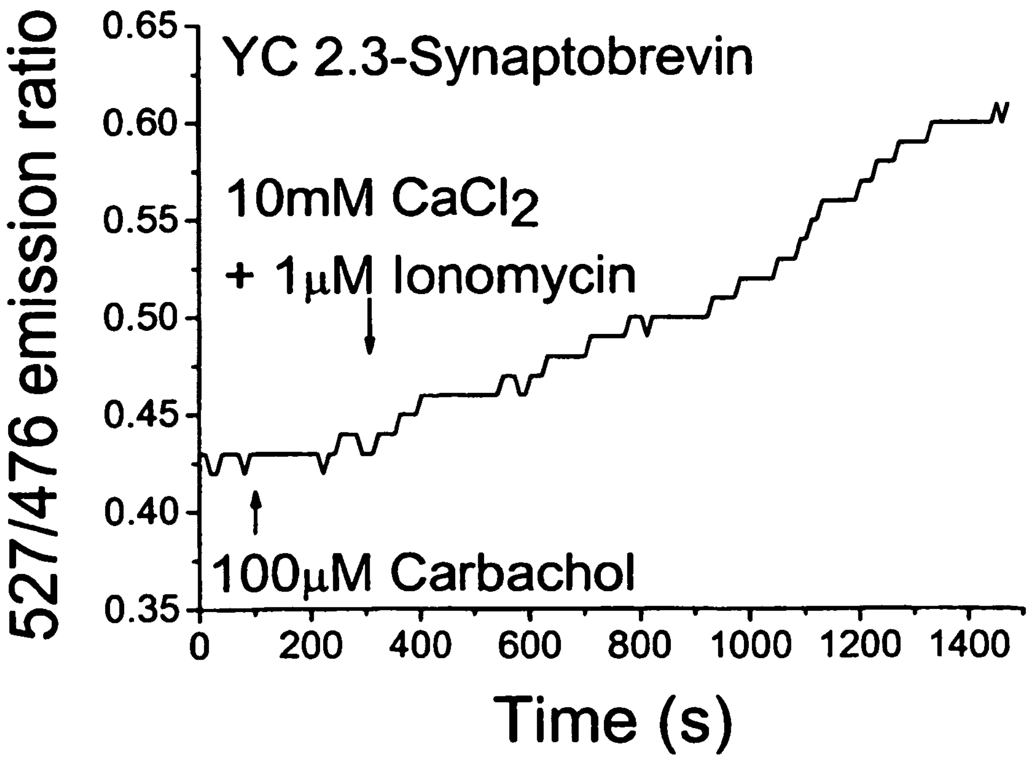
YC 2.3-Synaptobrevin
10mM CaCl2
+ 1μM Ionomycin
100μM Carbachol
527/476 emission ratio
0.65
0.60
0.55
0.50
0.45
0.40
0.35
0
200
400
600
800
1000
1200
1400
Time (s)

Fig. 11

A

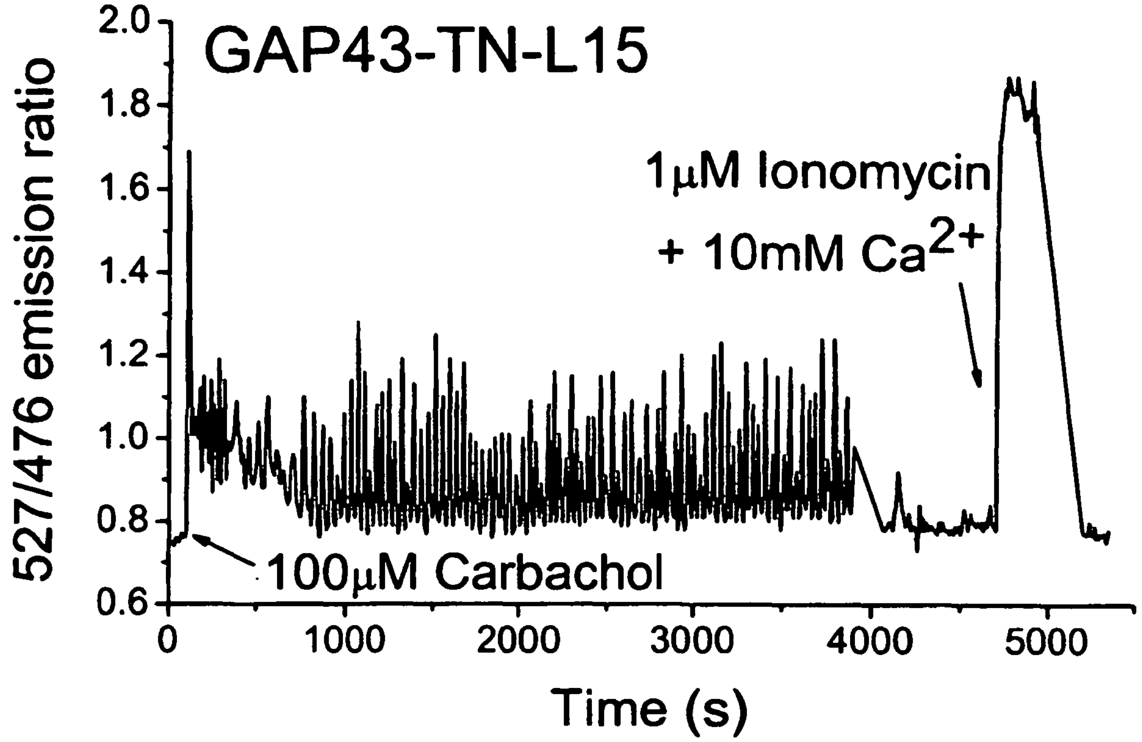
GAP43-TN-L15
527/476 emission ratio
2.0
1.8
1.6
1.4
1.2
1.0
0.8
0.6
1μM Ionomycin
+ 10mM Ca2+
100μM Carbachol
0
1000
2000
3000
4000
5000
Time (s)

B

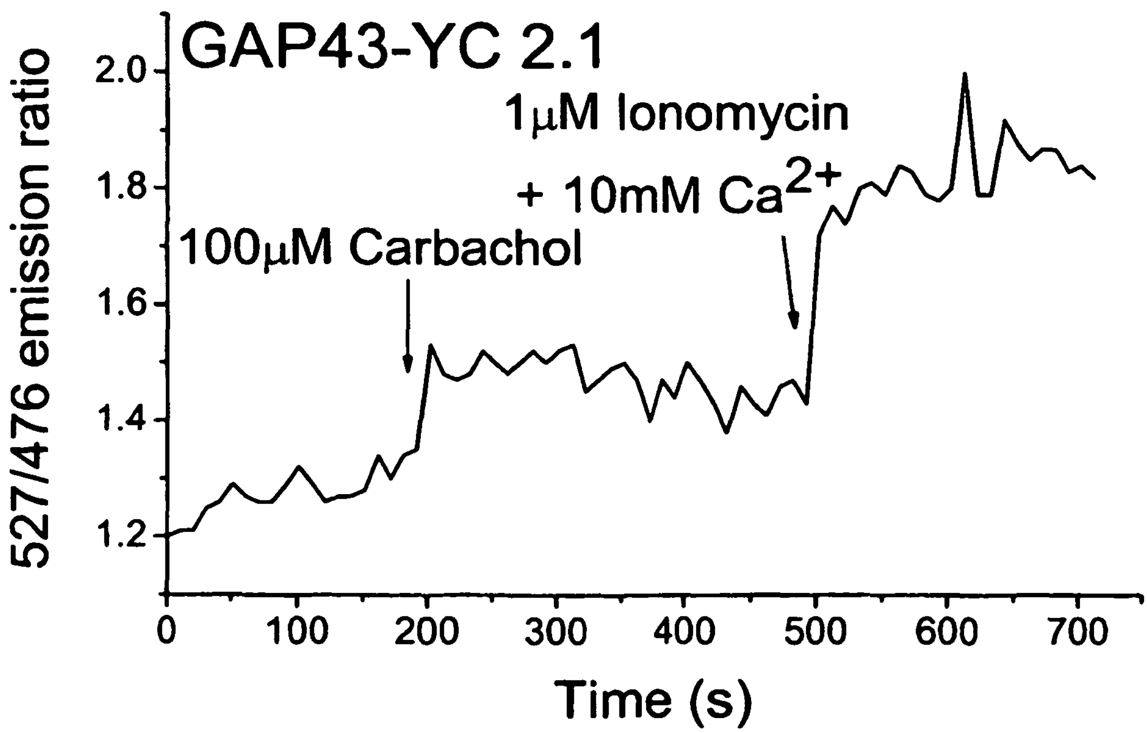
GAP43-YC 2.1
527/476 emission ratio
2.0
1.8
1.6
1.4
1.2
1μM Ionomycin
+ 10mM Ca2+
100μM Carbachol
0
100
200
300
400
500
600
700
Time (s)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4952496 A


### Non-patent literature cited in the description


- **ZHANG J. et al.** Creating new fluorescent probes for cell biology. *Nat. Rev. Mol. Biol.,* 2002, vol. 3, 906-918
- **MIYAWAKI, A. et al.** Fluorescent indicators for Ca2+ based on green fluorescent proteins and calmodulin. *Nature,* 1997, vol. 388, 882-887
- **MIYAWAKI, A. et al.** Dynamic and quantitative calcium measurements using improved cameleons. *Proc. Natl. Acad. Sci. USA,* 1999, vol. 96, 2135-2140
- **TRUONG et al.** FRET-based in vivo Ca2+ imaging by a new calmodulin-GFP fusion molecule. *Nat. Struct. Biol.,* 2001, vol. 8, 1069-1073
- **BAIRD, G.S. et al.** Circular permutation and receptor insertion within green fluorescent proteins. *Proc. Natl. Acad. USA,* 1999, vol. 96, 11241-11246
- **NAGAI, T. et al.** Circularly permuted green fluorescent proteins engineered to sense Ca2+. *Proc. Natl. Acad Sci. USA,* 2001, vol. 98, 3197-3202
- **NAKAI, J. et al.** A high signal-to-noise Ca2+ probe composed of a single green fluorescent protein. *Nat. Biotechnol.,* 2001, vol. 19, 137-141
- **GRIESBECK, O. et al.** Reducing the environmental sensitivity of yellow fluorescent protein: mechanism and applications. *J. Biol. Chem.,* 2001, vol. 276, 29188-29194
- **REIFF, D.F. et al.** Differential regulation of active zone density during long-term strengthening of Drosophila neuromuscular junctions. *J. Neurosci.,* vol. 22, 9399-9409
- **KERR R et al.** Optical imaging of calcium transients in neurons and pharyngeal muscle of C. elegans. *Neuron,* vol. 26, 583-594
- **FIALA et al.** Genetically expressed cameleon in Drosophila melanogaster is used to visualize olfactory information in projection neurons. *Curr. Biol.,* 2002, vol. 12, 1877-1884
- **JURADO, L.A. et al.** Apocalmodulin. *Physiol. Rev.,* 1999, vol. 79, 661-682
- **SAIMI, Y. ; KUNG, C.** Calmodulin as an ion channel subunit. *Ann. Rev. Physiol.,* 2002, vol. 64, 289-311
- **BENAIM, G. ; VILLALOBO, A.** Phosphorylation of calmodulin. *Eur. J Biochem.,* 2002, vol. 269, 3619-3725
- **WITCHLOW M. et al.** An improved linker for single-chain Fv with reduced aggregation and enhanced proteolytic stability. *Prot. Engineering,* 1993, vol. 6, 989-995
- **MIYAWAKI, A. et al.** Fluorescent indicators for Ca2+ based on green fluorescent proteins and calmodulin. *Nature,* 1997, vol. 388, 882-887
- **POZZAN T ; TSIEN RY.** *Methods Enzymol.,* 1989, vol. 172, 230-244
- **TSIEN R.Y.** The green fluorescent protein. *Ann. Rev. Biochem.,* 1998, vol. 67, 509-544
- **MATZ M.V. et al.** Fluorescent proteins from nonbioluminescent Anthozoa species. *Nat. Biotechnol.,* 1999, vol. 17, 969-973
- **WIEDENMANN J. et al.** Cracks in the β-can: Fluorescent proteins from Anemonia Sulcata (Anthozoa, Actinaria. *Proc. Natl. Acad Sci.,* 2000, vol. 97, 14091-14096
- **LABAS Y.A. et al.** Diversity and evolution of the green fluorescent protein family. *Proc. Natl. Acad Sci.,* 2002, vol. 99, 4256-4261
- **KARASAWA S. et al.** A green emitting fluorescent protein from Galaxeidae coral and its monomeric version for use in fluorescent labelling. *J. Biol. Chem.,* 2003
- **WIEDENMANN J. et al.** Cracks in the β-can: Fluorescent proteins from Anemonia Sulcata (Anthozoa, Actinaria. *Proc.Natl. Acad Sci.,* 2000, vol. 97, 14091-14096
- **GURSKAYA N.G. et al.** GFP-like chromoproteins as source of far-red fluorescent proteins. *FEBS lett.,* 2001, vol. 507, 16-20
- **WIEDENMANN J. et al.** A far red fluorescent protein with fast maturation and reduced oligomerization tendency from Entacmaea quadricolor (Anthozoa, Actinaria. *Proc. Natl. Acad Sci.,* 2002, vol. 99, 11646-11651
- **KENDAL et al.** Targeting aequorin to the endoplasmic reticulum of living cells. *Biochem. Biophys. Res. Commun.,* 1992, vol. 189, 1008-1016

- **MATTAJ ; ENGLMEIER.** Nuclear cytoplasmic transport: the soluble phase. *Annu. Rev. Biochem.,* 1998, vol. 67, 265-306
- **LIVGO, T.** Targeting of proteins to mitochondria. *FEBS Letters,* 2000, vol. 476, 22-26
- **HURT, E.C. et al.** *Embo J.,* 1985, vol. 4, 2061-2068
- **LLOPIS J. et al.** *Proc. Natl. Acad Sci. USA,* 1999, vol. 95 (12), 6803-8
- **GOULD S.G. et al.** *J. Cell Biol.,* 1987, vol. 105, 2923-2931
- **OZUMI T. et al.** *Biochem. Biophys. Res. Commun.,* 1991, vol. 181, 947-954
- **MORYOSHI K. et al.** *Neuron,* 1996, vol. 16, 255-260
- **LIEDHAMMER M. et al.** *J. Neurosci.,* 1996, vol. 16, 2157-63
- **LEMAOUT S. et al.** *Proc. Natl. Acad Sci. USA,* 2001, vol. 98, 10475-10480
- **HUNG ; SHENG.** *J. Biol. Chem.,* 2001, vol. 277, 5699-5702
- **BENNETT et al.** *Science,* 1992, vol. 257, 255-259
- **ELFERINK et al.** *J. Biol. Chem.,* 1989, vol. 264, 11061-4
- **SZCZESNA et al.** *J. Biol. Chem.,* 1996, vol. 271, 8381-8386
- **SORENSEN et al.** *J. Biol. Chem.,* 1995, vol. 270, 9770-9777
- Molecular Cloning: A Laboratory Manual. CSHL Press, 2001
- **MUMBERG et al.** *Nucl. Acids Res.,* 1994, vol. 22, 5767-5768
- Generation of recombinant bacculovirus DNA in E. coli using bacculovirus shuttle vector. **ZICCARONE ; U. REISCHT et al.** Generation of recombinant bacculovirus DNA in E. coli using bacculovirus shuttle vector. Humana Press Inc, 1997, vol. 13
- **SCHLESINGER.** *Trans Bio Technol.,* 1993, vol. 11 (1), 18-22
- **HEH et al.** *Proc. Natl. Acad. Sci. USA,* 1998, vol. 95, 2509-2514
- Production of transgenic mice. **HOGAN D. et al.** Manipulating the Mouse Embryo: A Laboratory Manual. Cold Spring Harbor Laboratory Press, 1994, 217-252
- **ALBANESE C. et al.** Recent advances in inducible expression in transgenic mice. *Semin. Cell. Dev. Biol.,* 2002, vol. 13, 129-41
- **RUBIN ; SPREADLING.** *Science,* 1982, vol. 218, 348-53
- **MELLOW ; FIRE.** Methods in Cell Biology. Academic Press, 1995, vol. 48, 451-482
- **HIGASHIYIMA et al.** *Dev. Biol.,* 1997, vol. 192, 289-99
- **BECHTOLD et al.** *C. R. Acad. Sci.,* 1993, vol. 316, 1194-1199
- **MCNAMEE, MG.** Isolation and characterization of cell membranes. *Biotechnique,* 1989, vol. 7, 466-475
- **JOOST HG ; SCHURMANN, A.** Subcellular fractionation of adipocytes and 3T3 L1 cells. *Meth. Mol. Biol.,* 2001, vol. 155, 77-82
- **MORIYOSHI K. et al.** Labeling Neural Cells Using Adenoviral Gene Transfer of Membrane-Targeted GFP. *Neuron,* 1996, vol. 16, 255-260
- **GRYNKIEWICZ G. et al.** A New Generation of Ca2+ Indicators with Greatly Improved Fluorescence Properties. *J. Biol. Chem.,* 1985, vol. 260, 3440-3450